# EUROPEAN PATENT SPECIFICATION

(11) **EP 4 249 470 B1**
(45) Date of publication and mention of the grant of the patent: **13.11.2024**
(21) Application number: 22163544.4
(22) Date of filing: 22.03.2022
(51) Int. Cl.: C07C 269/06, C07C 271/22, C07C 319/20, C07C 323/63, C07D 209/20, C07D 209/28, C07D 209/88, C07D 215/14, C07D 313/12, C07D 317/60, C07D 333/24, C07J 41/00, C07B 43/04, C07B 59/00, B01J 31/18, B01J 31/22

(54) **METHOD FOR PREPARING ALPHA-AMINO ACIDS**
VERFAHREN ZUR HERSTELLUNG VON ALPHA-AMINOSÄUREN
PROCÉDÉ DE PRÉPARATION D'ACIDES AMINÉS ALPHA

(43) Date of publication of application: 27.09.2023
(73) Proprietor: PHILIPPS-UNIVERSITÄT MARBURG, 35037 Marburg (DE)
(72) Inventor: Meggers, Eric, 35039 Marburg (DE); Ye, Chen-Xi, 35039 Marburg (DE); Shen, Xiang, 35039 Marburg (DE); Zhou, Bing, 35039 Marburg (DE)
(74) Representative: Durm Patentanwälte PartG mbB

(56) References cited:
- J.M. JANEY: "Recent advances in catalytic, enantioselective alpha-aminations and alpha-oxygenations of carbonyl compounds", ANGEWANDTE CHEMIE, INTERNATIONAL EDITION, vol. 44, no. 28, 11 July 2005 (2005-07-11), Wiley-VCH Verlag, Weinhem, DE, pages 4292 - 4300, XP055958676, ISSN: 1433-7851, DOI: 10.1002/anie.200462314
- LI-MEI JIN, ET AL.: "Enantioselective intermolecular radical C-H amination", JOURNAL OF THE AMERICAN CHEMICAL SOCIETY, vol. 142, no. 49, 25 November 2020 (2020-11-25), American Chemical Society, Washington, DC, US, pages 20828 - 20836, XP055958682, ISSN: 0002-7863, DOI: 10.1021/jacs.0c10415
- HUI FU, ET AL.: "Synthesis of protected alpha-amino acids via decarboxylation amination from malonate derivatives", ORGANIC AND BIOMOLECULAR CHEMISTRY, vol. 18, no. 23, 13 May 2020 (2020-05-13), Royal Society of Chemistry, Cambridge, GB, pages 4439 - 4446, XP055958684, ISSN: 1477-0520, DOI: 10.1039/D0OB00677G

## Description

The present invention relates to a method for preparing non-racemic α-amino acids and the use of a chiral non-racemic catalyst for carrying out this method. In an extension, this invention can be used for the preparation of racemic α-amino acids by using achiral or racemic catalysts.

α-Amino acids are essential building blocks of life. It is therefore not surprising that there is a high demand for all kinds of unnatural and non-proteinogenic α-amino acids to modulate the chemical, physical, and pharmaceutical properties of peptides, proteins and other bioactive molecules. Beyond biological applications, chiral α-amino acids are also used as chiral building blocks for chiral catalysts, chiral auxiliaries and diverse (macro)molecules. Synthetic chemistry have therefore developed many powerful methods to satisfy the increasing demand for optically active α-amino acids. However, an efficient and economical catalytic enantioselective method is still thought after.

A direct and straightforward strategy for the synthesis of optically active α-amino acids is the catalytic enantioselective introduction of an amino group in α-position of readily available carboxylic acids.^{1,2} A number of methods for direct asymmetric C(sp³)-H aminations have been reported and typically exploit the acidity of the C-H group next to a carbonyl functionality for electrophilic aminations via enolate intermediates.³⁻⁷ However, most existing methods use aldehydes, ketones or dicarbonyl compounds as starting materials instead of more desirable but less acidic carboxylic acid derivatives. To further complicate matters, the electrophilic amination reagents employed are usually diazo compounds, which lead to amination products that cannot be easily converted to the target α-amino acids.

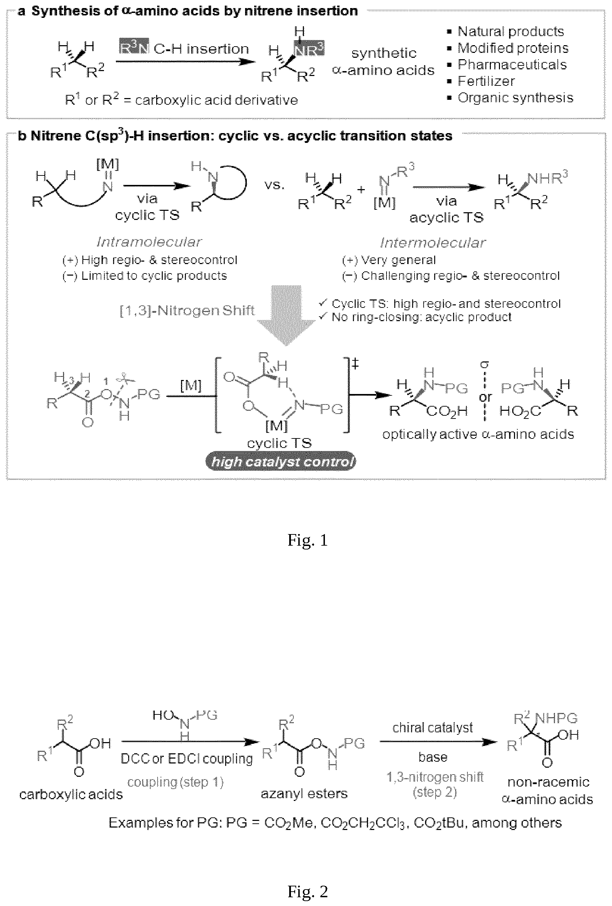

The insertion of nitrenes into C-H bonds provides a more tunable alternative platform for C(sp³)-H aminations as the reactivity of nitrenes can be controlled by transition metal coordination. In addition, milder reaction conditions can often be used (Figure 1a).⁸ Much progress has been made employing chiral transition metal catalysts for the enantioselective conversion of prochiral C(sp³)-H bonds into C-N bonds by nitrene insertion.^{9,10} However, intermolecular nitrene insertion reactions suffer from problems with regioselectivity and enantioselectivity (Figure 1b).¹¹⁻¹⁹ Although this is not the case for intramolecular C(sp³)-H amination reactions, in which well-defined intramolecular cyclic transition states allow exquisite regio- and stereocontrol, such intramolecular C-H nitrene insertions are always ring-closing reactions and therefore lack general applicability.²⁰⁻²⁶ Thus, the catalytic enantioselective synthesis of acyclic amines by catalytic enantioselective C(sp³)-H nitrene insertion remains a challenge, and its application to the synthesis of chiral α-amino acids would be highly desirable.

The technical problem underlying the present invention is to provide a method for preparing in particular non-racemic amino acids in an efficient way without producing undesired side products at low prices in a fast process.

This object has been solved by the method of claim 1 and the use of claim 14. Preferred embodiments are defined in dependent claims 2 to 13.

According to the present invention, there is provided a method for preparing in particular non-racemic α-amino acids and non-racemic α-deuterated α-amino acids, comprising the following steps:
providing carboxylic acid azanyl esters, in which the N-atom of the azanyl ester group is protected by a protection group, and
carrying out a stereocontrolled 1,3-nitrogen migration in the presence of a chiral non-racemic catalyst to provide chiral non-racemic α-amino acids.

In this catalyzed stereocontrolled 1,3-nitrogen migration, the protected N-atom of the azanyl ester is migrated from the O-atom to the α-C-atom of the carbonyl group.

Azanyl ester of carboxylic acids are known in the prior art. Therefore, the skilled person knows methods and materials for preparing the carboxylic acid azanyl esters employed in the method according to the present invention.

In one embodiment, the carboxylic acid azanyl ester is prepared by reacting the carboxylic acid with the compound OH-NH-PG, wherein PG is the protecting group. This reaction can be carried out, for example, as a coupling reaction by employing DCC (N,N'-dicyclohexylcarbodiimide) or EDCI (1-ethyl-3-(3-dimethylaminopropyl)carbodiimide) as coupling reagent. The protecting group PG can remain in the obtained α-amino acid or it can be later removed depending on the particular further use of the α-amino acid. That is, if for the subsequent reactions and use of the α-amino acid the protection of the N-atom is required, the protecting group can be remained. If the protective group is cleaved, this can be carried out in a manner known in the prior art.

In a further embodiment, the protecting group is an electron withdrawing protecting group, preferably a carbamate moiety. For example, the electron withdrawing protecting group is selected from the group consisting of the CO₂Me group, the CO₂CH₂CCl₃ (Troc) group, the tert-butyloxycarbonyl (Boc) group, the benzyloxycarbonyl (Cbz) group, the allyloxycarbonyl (Alloc) group, among other amine protecting group that form a carbamate.

The method according to the present invention is applicable to a wide range of carboxylic acids having at least one hydrogen at the α-C-atom and one or two groups, also called substituents, bound to the α-C-atom. Such carboxylic acids can be formally represented by the general formula R-CH₂-C(O)OH (a single group R bound to the α-C-atom) or R¹R²CH-C(O)OH (two groups R¹ and R² bound to the α-C-atom, also called a α-branched carboxylic acid). In one embodiment, the carboxylic acid has a side chain R at the α-C-atom containing a residue selected from the group consisting of aryl, allyl, vinyl, propargyl, alkyl moieties, or other typical side chains. In another embodiment, the carboxylic acid is branched in α-position and has two side chains R¹ and R² consisting of aryl, allyl, vinyl, propargyl, alkyl moieties, or other typical side chains. This means that the residue R, R¹ and R² in this formula has a group derivable from aryl, allyl, vinyl, propargyl, alkyl moieties, or other typical side chains, wherein further groups, atoms and substituent can be present depending on the intended further use of the obtained α-amino acids.

As pointed out above, the method according to the present invention is carried out in the presence of a catalyst. Every catalyst can be employed in the method according to the present invention, which can provide the above mentioned 1,3-nitrogen migration to generate α-amino acids. Furthermore, every chiral non-racemic catalyst can be employed in the method according to the present invention, which can provide the above mentioned 1,3-nitrogen migration in a stereocontrolled fashion to generate non-racemic α-amino acids. For example, the catalyst is a chiral non-racemic ruthenium catalyst or a chiral non-racemic iron catalyst. In one embodiment, the chiral non-racemic ruthenium catalyst is a chiral non-racemic ruthenium(II) catalyst containing organic ligands or the chiral non-racemic iron catalyst is a chiral non-racemic iron(II) catalyst containing organic ligands. In the catalyst, for example in the chiral ruthenium(II) catalyst and in the chiral iron(II) catalyst, the central metal has six coordination sites to which ligands can bind. In one embodiment the catalyst contains two mondodentate ligands, in particular wherein the monodentate ligands are selected from the group consisting of acetonitrile, triflate or other sulfonates, acetate or other carboxylates, chloride, bromide, iodide, other pseudohalides, and other potentially labile monodentate ligands. The two monodentate ligands can be identical or different from each other. These two monodentate ligands, which can be removable from the catalyst, provide two binding sites at the catalyst so that after the cleavage of the azanyl ester both obtained fragments can bind to the catalyst for providing the 1,3-nitrogen migration.

Furthermore, the catalyst can contain either two bidentate ligands or one tetradentate ligand, wherein the bidentate ligand contains one C-atom and one N-atom to bind to the central metal, in particular Ru, while the tetradentate ligand can contain four N-atoms to bind to the central atom, in particular Fe. Further, the catalyst can contain cyclic organic residue in which these N-atoms are contained as heteroatoms.

Other chiral non-racemic transition metal catalysts which are known in the prior art are also suitable for catalyzing the stereocontrolled 1,3-nitrogen migration, such as catalysts with the metals manganese, cobalt, nickel, copper, rhodium and iridium in various oxidation states with monodentate, bidentate, tridentate, and/or tetradentate ligands or a combination thereof.

Other transition metal catalysts which are known in the prior art are suitable for catalyzing the 1,3-nitrogen migration in a non-stereocontrolled fashion, thereby providing racemic α-amino acids.

In one embodiment the catalyst selected from the group consisting of RuDMP, RuTMS, RuCF₃, RuH, Δ-Fe1, *(R,R)*-Fe2, (R,R)-Fe3 and *(R,R)*-FeBIP.
A-RuH: A-bis(acetonitrile)bis[1-mesityl-3-(pyridin-2-yl)-1,3-dihydro-2H-imidazol-2-ylidene-κ²*C*,*N*]ruthenium(II) bis(hexafluorophosphate)
A-RuDMP: Λ-bis(acetonitrile)bis{1-[5-(3,5-dimethylphenyl)pyridin-2-yl]-3-mesityl-1,3-dihydro-2H-imidazol-2-ylidene-κ²*C*,*N*}ruthenium(II) bis(hexafluorophosphate)
A-RuCF₃: Λ-bis(acetonitrile)bis{1-mesityl-3-[5-(trifluoromethyl)pyridin-2-yl]-1,3-dihydro-2H-imidazol-2-ylidene-κ²*C*,*N*}ruthenium(II) bis(hexafluorophosphate)
A-RuTMS: Λ-bis(acetonitrile)bis{1-mesityl-3-[5-(trimethylsilyl)pyridin-2-yl]-1,3-dihydro-2*H-*imidazol-2-ylidene-κ²*C*,*N*}ruthenium(II) bis(hexafluorophosphate)
Δ-Fe1: Δ-bis(acetonitrile)bis{1-mesityl-3-[5-(trifluoromethyl)pyridin-2-yl]-1,3-dihydro-2H-imidazol-2-ylidene-κ²*C*,*N*}iron(II) bis(hexafluorophosphate)
(R,R)-Fe2: bis(acetonitrile)[(2*R*,2'*R*)-1,1'-bis(pyridin-2-ylmethyl)-2,2'-bipyrrolidine-κ⁴*N*]iron(II) bis(hexafluoroantimonate)
(R,R)-Fe3: bis(acetonitrile)[(2*R*,2'*R*)-1,1'-bis({5-[2,6-bis(trifluoromethyl)phenyl]pyridin-2-yl}methyl)-2,2'-bipyrrolidine-κ⁴*N*]iron(II) bis(hexafluoroantimonate)
(*R*,*R*)-FeBIP: dichlorido{(2*R*,2'*R*)-1,1'-bis[(1-methyl-1*H*-benzo[*d*]imidazol-2-yl)methyl]-2,2'-bipyrrolidine-κ⁴*N*}iron(II)

The reaction conditions for carrying out the method according to the present invention are chosen so that the stereocontrolled 1,3-migration can be provided. For example, the amount of catalyst can be below 10 mol%, referred to the amount of the carboxylic acid azanyl ester), for example in the range of 1 mol% to 8 mol%. Lower amounts of catalyst are also feasible. The reaction temperature is chosen so that the reaction is sufficiently completed, the amount of side products is not too high and the enatioselectivity is sufficient. The reaction temperature can for example be in the range of 0 °C to 40 °C. The method according to the present invention can be carried out in a solvent. Examples of suitable solvents are halogenated hydrocarbons such as methylene chloride, 1,2-dichloroethane, chloroform, chlorobenzene, 1,2-dichlorobenzene, and 1,2-difluorobenzene. Other solvents are tetrahydrofuran, toluene, ethyl acetate, acetone, acetonitrile, dimethylformamide, among others. Furthermore, the method according to the present invention can be carried out in the presence of a base, for example K₂CO₃, KHCO₃, piperidine, 2,2,6,6-tetramethyl piperidine. The amount of the base employed in the method according to the present invention is 0,5 to 3 equivalents of the base to 1 equivalent of the carboxylic acid azanyl ester. Other amounts of base are also feasible.

As pointed out above, the α-amino acid obtained by the method according to the present invention contains the protecting group, which is introduced into it by using a protected hydroxylamine compound in the reaction forming the carboxylic acid azanyl ester. If required depending on the further use of the α-amino acid the protecting group can be cleaved off.

Furthermore the α-amino acid, which may contain the protecting group, can be recrystallized so that the enantiopurity can be increased. An example of a solvent to be used in the recrystallization can be a mixture of ethanol and n-hexane.

Subject-matter of the present invention is further the use of a catalyst, in particular the catalyst as defined above, for carrying out a 1,3-nitrogen migration of carboxylic acid azanyl esters, wherein the N-atom of the azanyl ester group is protected by a protecting group, to provide chiral non-racemic α-amino acids, wherein the 1,3-nitrogen migration is carried out in the presence of the catalyst. If the catalyst is chiral non-racemic, this 1,3-nitrogen migration is stereocontrolled and affords chiral non-racemic α-amino acids. If the catalyst is achiral or racemic, the 1,3-nitrogen migration affords racemic α-amino acids, which are also desirable reaction products. For the details of the use it is referred to the above disclosure.

The present invention will be further illustrated in detail by the following examples and figures, wherein it is emphasized that neither the examples nor the figures shall be understood to limit the invention thereto. In the figures,

Fig. 1 shows the stereocontrolled nitrene C(sp³)-H insertions for the synthesis of α-amino acids. (TS = transition state, PG = protecting group).

Fig. 2 provides an overview of the invented method for the synthesis of non-racemic α-amino acids. Briefly, carboxylic acids which possess at least one C-H group in α-position are first converted to azanyl esters. Then, a chiral catalyst catalyzes a stereocontrolled 1,3-nitrogen migration to obtain nitrogen-protected α-amino acids in a non-racemic fashion. TS = transition state, PG = protecting group. R¹ = Alkyl, aryl, alkenyl, vinyl, and other side chains. R² = H or alkyl, aryl, alkenyl, vinyl, and other side chains.

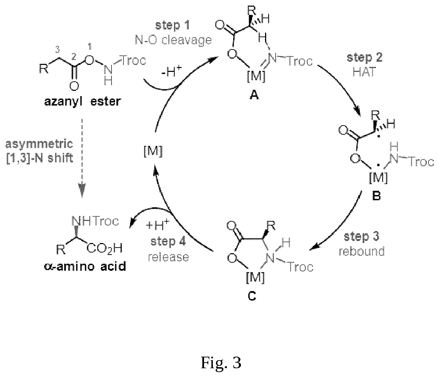

Fig. 3 shows the proposed simplified mechanism for a non-α-branched carboxylic acid as the starting material. The azanyl ester substrate is first activated by N-O cleavage to provide intermediate A after deprotonation. A subsequent hydrogen atom transfer then affords the diradical B, followed by a radical rebound to yield the chelate complex C. Protonation of the carboxylate induces product dissociation and concludes the catalytic cycle. The net result of the N-O bond fragmentation and subsequent stepwise C-H insertion is a novel asymmetric 1,3-nitrogen shift to provide non-racemic chiral α-amino acids.

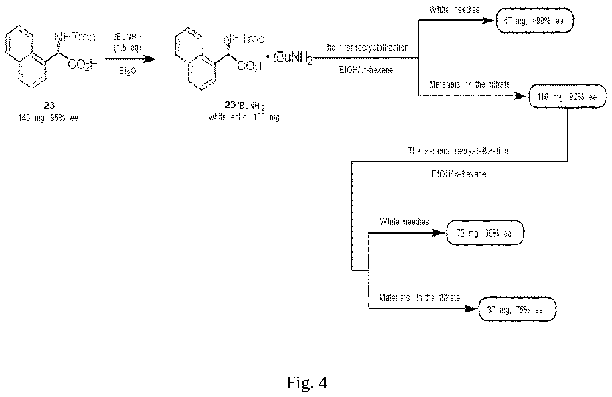

Fig. 4 shows the recrystallization of a N-Troc protected α-amino acid which was synthesized by a stereocontrolled 1,3-nitrogen migration. Enantiopure N-Troc protected α-amino acid 23 (up to >99% e.e., e.e. = enantiomeric excess) was obtained by recrystallization of the corresponding ammonium salt from ethanol/n-hexane.

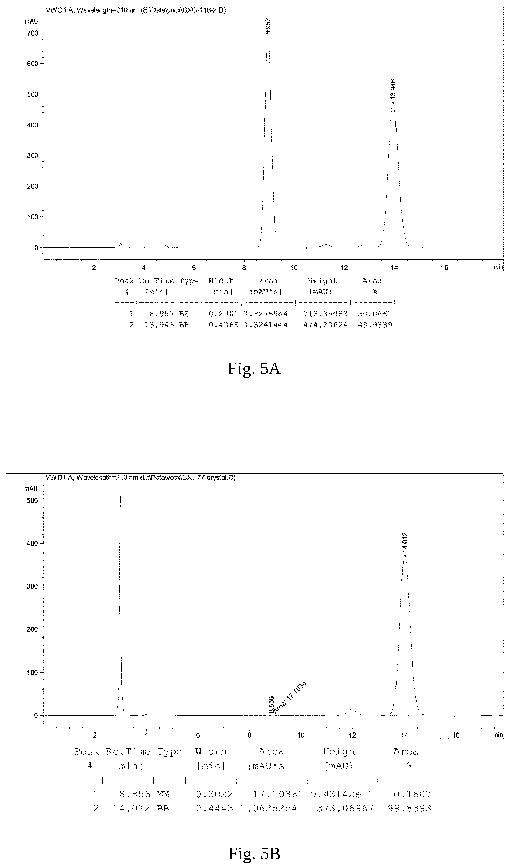

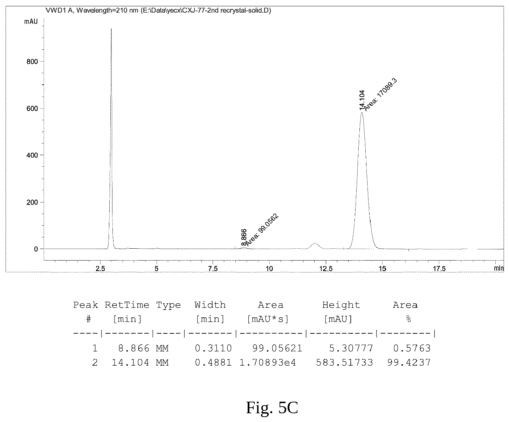

Fig. 5A-C show the HPLC chromatograms of **23** in the recrystallization method. Fig. 5A: HPLC of racemic **23** (CHIRALPAK IG, 25 °C, iPrOH/n-hexane = 20/80 + 0.1% TFA, 1.0 mL/min, 210 nm); Fig. 5B: HPLC of **23** derived from the first recrystallization (>99% e.e.); Fig. 5C: HPLC of **23** derived from the second recrystallization (99% e.e.).

The present invention provides a method for the economical and practical synthesis of non-racemic (optically active) α-amino acids based on an unprecedented stereocontrolled 1,3-nitrogen shift. Likewise, the presented invention provides a method for the economical and practical synthesis of racemic α-amino acids by performing the 1,3-nitrogen shift in a non-stereocontrolled fashion. The method according to the present invention employs abundant and easily accessible carboxylic acids as starting materials, which are first connected to a nitrogenation reagent, followed by a highly regio- and enantioselective for example ruthenium- or iron-catalyzed C(sp³)-H amination. This straightforward method displays a very broad scope, providing rapid access to optically active α-amino acids with aryl, allyl, vinyl, propargyl, and alkyl side chains, and also permits stereocontrolled late-stage amination of carboxylic acid-containing drugs and natural products. The method according to the present invention combines the advantages of intramolecular (regio- and stereocontrol via intramolecular cyclic transition state) and intermolecular C-H nitrene insertion chemistry (more general, acyclic products) by covalently connecting a nitrene precursor to a carboxylic acid functionality. After O-N bond cleavage and binding of both fragments to the catalyst, a cyclic transition state facilitates a C(sp³)-H amination. If the catalyst is chiral non-racemic, the 1,3-nitrogen shift is stereocontrolled and provides non-racemic α-amino acids. This reaction constitutes an unprecedented stereocontrolled 1,3-nitrogen shift and is applied to the catalytic asymmetric synthesis of α-amino acids (Figure 1b). If the catalyst is achiral or racemic, the 1,3-nitrogen shift is non-stereocontrolled and provides racemic α-amino acids, which are also of interest.

The carboxylic acid azanyl esters **1a,b** are provided in which the nitrogen bears different electron-withdrawing protecting groups, which is established to be beneficial for generating electrophilic nitrene intermediates. The catalysts used were "chiral-at-metal" ruthenium complexes in which two bidentate N-(2-pyridyl)-substituted N-heterocyclic carbenes and two acetonitrile ligands are coordinated to a central ruthenium in a *C*₂-symmetric fashion (Table 1).²⁷ Despite all ligands being achiral, the overall chirality required for asymmetric catalysis originates from the stereogenic ruthenium center. It has been previously demonstrated such complexes to be capable of catalyzing enantioselective ring-closing C-H aminations.^{28,29} Importantly, this class of catalysts feature two vacant coordination sites adjacent to each other (coordination sites of the two labile acetonitrile), which is suitable for the method according to the present invention.

By using the methoxycarbonyl protecting group a it was found that the substrate 1a was completely consumed under the reaction conditions of 1 mol%, referred to the educt, of the catalyst in the presence of K₂CO₃ (3 equivalents, referred to the educts) at room temperature (18 °C to 25 °C) for 16 hours, and the chiral amino acid (*R*)-**2a** formed in 86% yield with 89% enantiomeric excess (ee) (entry 1). Finally, suitable results were obtained with the 2,2,2-trichloroethoxycarbonyl (Troc) protecting group **(b),** which provided the amino acid (*R*)-**2b** in an excellent 93% yield as determined by ¹H-NMR and with 95% ee (entry 2). Other catalysts with different substituents on the pyridine moieties were also used, namely the 3,5-dimethylphenyl groups with trimethylsilyl (TMS) groups (**RuTMS**²⁸), CF₃ groups (**RuCF₃**²⁹), or just hydrogen (**RuH**²⁷)(entries 3-5). Table 1 also reveals that CH₂Cl₂ is the preferable solvent and K₂CO₃ the preferable base for this stereocontrolled 1,3-nitrogen shift (entries 6-9).

**Table 1. Initial experiments and optimization of enantioselective 1,3-nitrogen shift**

| 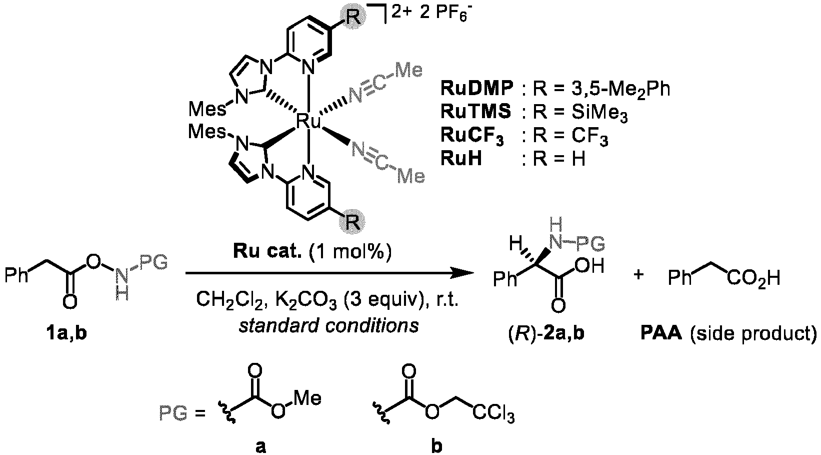 | | | | | | | |
|---|---|---|---|---|---|---|---|
| Entry | Catalyst | PG*^{a}* | Conditions*^{b}* | Conv. (%)*^{c}* | **2** (%)*^{c}* | **PAA** (%)*^{c}* | ee of **2** (%)*^{d}* |
| 1 | Λ**-RuDMP** | CO₂Me **(a)** | standard | 100 | 86 | 12 | 89 |
| 2 | Λ**-RuDMP** | Troc **(b)** | standard | 100 | 93 (91)*^{e}* | 4 | 95 |
| 3 | Λ**-RuTMS** | Troc **(b)** | standard | 98 | 80 | 9 | 92 |
| 4 | Λ**-RuCF₃** | Troc **(b)** | standard | 19 | 6 | 5 | 90 |
| 5 | Λ**-RuH** | Troc **(b)** | standard | 100 | 85 | 9 | 92 |
| 6 | Λ**-RuDMP** | Troc **(b)** | Et₃N as base | 100 | 25 | 73 | 95 |
| 7 | Λ**-RuDMP** | Troc **(b)** | Na₂CO₃ as base | 99 | 87 | 5 | 95 |
| 8 | Λ**-RuDMP** | Troc **(b)** | THF as solvent | 94 | 15 | 75 | 82 |
| 9 | Λ**-RuDMP** | Troc **(b)** | MeOH as solvent | 96 | 0 | 64 | - |

| | | | | | | | |
|---|---|---|---|---|---|---|---|
| *^{a}* Troc = CO₂CH₂CCl₃. *^{b}* Shown are the deviations from the standard reaction conditions. Standard conditions: substrate 1 (0.1 mmol), Ru catalyst (1 mol%) and the indicated base (3 equiv) in the indicated solvent (2 mL) were stirred at room temperature for 16 hours. *^{c}* Conversion and yield were determined by ¹H NMR analysis using hexamethylbenzene as an internal standard. *^{d}* The ee was determined by HPLC on chiral stationary phases. *^{e}* Yield of the isolated α-amino acid. n.d. = not determined. | | | | | | | |

A substrate scope for ruthenium catalysis was also investigated. With the preferred reaction conditions in hand, the substrates of this new stereocontrolled 1,3-nitrogen shift were investigated. *N*,*N'*-Dicyclohexylcarbodiimide (DCC)-induced coupling of readily available carboxylic acids with *N-*Troc-protected hydroxylamine provided rapid access to a variety of azanyl esters, which were then subjected to the rearrangement under the above reaction conditions (Table 2). The phenyl moiety of phenylacetic acid azanyl ester **1e** was functionalized and it was found that the method according to the present invention tolerates a large variety of different substituents in the phenyl ring, affording the corresponding chiral α-amino acids **3-21** in up to 96% yield and with up to 98% ee (Table 2a). Electron-donating functional groups in the phenyl moiety such as methyl (α-amino acid **3),** methoxy (α-amino acids **5),** 1,3-dioxole (α-amino acid **8),** a Fmoc-protected alcohol (α-amino acid **9),** hydroxymethyl (α-amino acid **10),** and *N*-pivaloylamide (α-amino acid **19)** are tolerated, as are electron-withdrawing substituents such as halogens (α-amino acid **11-14),** trifluoromethyl (α-amino acid **15),** nitro (α-amino acid **16),** acetyl (α-amino acid **17),** and methoxycarbonyl (α-amino acid **18).** The method according to the present invention also enables the synthesis of the azido α-amino acid **20** (95% yield and 97% ee) and the alkynyl α-amino acid **21** (88% yield, 94% ee), both of which are building blocks of interest for click chemistry.³⁰ The stereocontrolled 1,3-nitrogen shift was also applied to the synthesis of α-aryl amino acids with benzannulated aromatic and heteroaromatic systems (α-amino acids **22-25)** in 77-92% yield and with 90-96% ee. Notably, the reaction could be readily scaled up to afford the naphthyl α-amino acid **23** in gram quantities with high yield (91%) and enantiopurity (95% ee, increased to 99% ee after crystallization protocol, see Figure 3). It is noteworthy that the stereocontrolled 1,3-nitrogen shift can also be applied to the late-stage functionalization of bioactive compounds. For example, isoxepac,³¹ an arylacetic acid derivative with anti-inflammatory and analgesic activity, was converted to the corresponding α-amino acid **26** in 87% yield and with 98% ee. Diclofenac,³² a pain medication for the treatment of inflammatory diseases, was converted into its azanyl ester and then rearranged to afford α-amino acid **27** in 54% yield and with 96% ee, which is remarkable considering the presence of an unprotected and sterically hindered aniline moiety in the ortho-position.

Also investigated was the C-H aminations at non-benzylic positions. Table 2b shows that the stereocontrolled 1,3-nitrogen shift can be applied to the asymmetric synthesis of chiral α-amino acids with alkenyl **(28-32)** and alkynyl **(34** and **35)** side chains, with enantioselectivities of62-84% ee. Even the unusual Troc-protected 2-amino-(*E*)-3,5-hexadienoic acid **(33)** could be synthesized in 72% yield and with 81% ee. These results show that the stereocontrolled 1,3-nitrogen shift is applicable to benzylic, allylic, and propargylic C-H bonds.

The asymmetric synthesis of α-disubstituted α-amino acids by amination of tertiary C-H bonds at a stereogenic carbon center is shown in Table 2c. When the racemic azanyl ester **37** was reacted under the above reaction conditions with a catalyst loading of 2 mol%, the corresponding 2-amino-2-phenylpropanoic acid **38** was obtained in 71% yield with a 48% ee. Since the azanyl ester is chiral, it was expected high stereodiscrimination between enantiotopic C-H bonds. Indeed, the *S*-enantiomer (*S*)-**37** (99% ee) afforded the α-amino acid (*R*)-**38** smoothly in 91% yield and with 98% ee (matched substrate/catalyst combination) while the mirror-imaged substrate (*R*)-**37** reacted sluggishly, providing the α-amino acid in 34% yield with 28% ee (mismatched substrate/catalyst combination). The stereospecific C-H amination of chiral non-racemic substrates is general and was applied to the synthesis of the α-disubstituted α-amino acids **39-42** in 25-86% yield and with 86-99% ee. For example, the azanyl ester of the nonsteroidal anti-inflammatory drug naproxen, which contains an (*S*)-configured stereocenter, was converted to the corresponding α-amino acid **39** in 86% yield and 99% ee, with virtually complete stereoretention. The C-H amination is also applicable to completely non-activated tertiary C(sp³)-H bonds, as demonstrated by α-amino acids **41** and **42.**

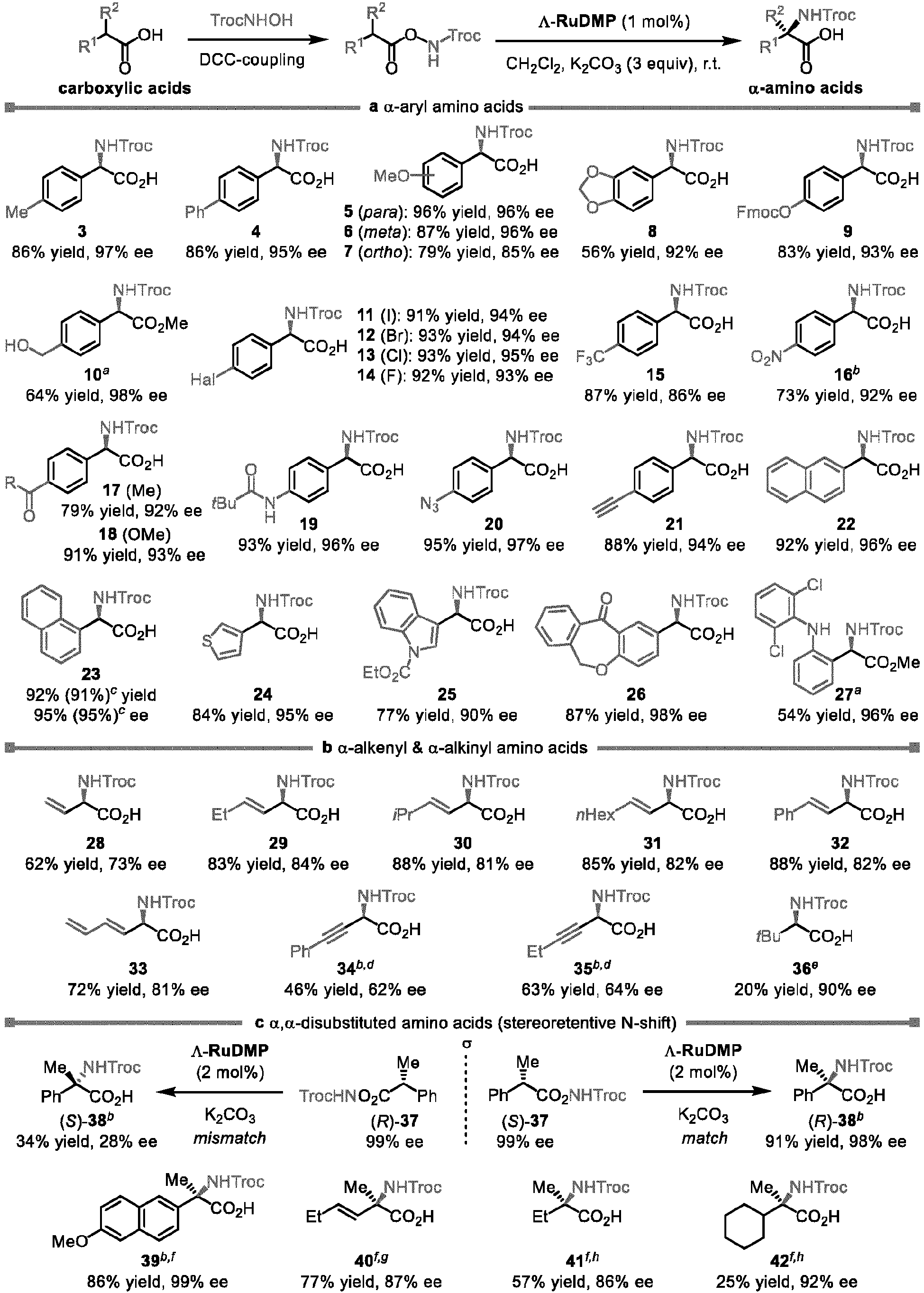

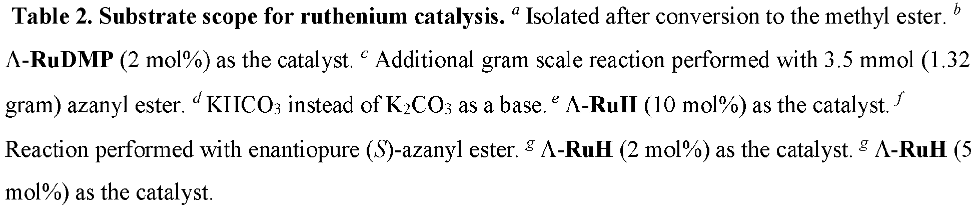

A substrate scope for iron catalysis was also investigated. Iron catalysis³³ was investigated which has additional economic and environmental benefits. Iron-catalyzed C-H bond aminations through nitrenoid intermediates have been reported, including the (racemic) amination of non-activated aliphatic C(sp³)-H bonds.³⁴ The conversion of azanyl ester **1e** to the A-Troc-protected phenylglycine **2e** as the initial model reaction to identify an iron catalyst (Table 3). The chiral-at-iron complex Δ-**Fe1**,³⁵ a lighter homolog of the chiral-at-ruthenium complexes used above (Table 1) and non-heme iron complexes with linear tetradentate N4-donor ligands coordinated in a *cis*-α-topological configuration. The overall helical topology is similar to the ruthenium catalysts, including the presence of two adjacent labile ligands. Using bis-pyridine complexes (*R,R*)-**Fe2** and (*R,R*)-**Fe3** featuring a rigid chiral 2,2'-bipyrrolidine backbone,^{36,37} the desired amino acid (*S*)-**2e** were obtained in 91% and 75% yield, respectively, albeit with <20% ee. Replacing the pyridyl moieties with *N*-methylbenzimidazole³⁸ **(BIP** ligand) and using labile chloride ligands instead of acetonitrile resulted in the air- and moisture-stable neutral complex (*R*,*R*)-[FeCl₂(**BIP**)] ((*R,R*)-**FeBIP**), which provided phenylglycine (*S*)-**2e** in 95% yield and with a 91% ee. It was found that (*R,R*)-**FeBIP** is a preferable catalyst for the generation of non-racemic α-amino acids with non-activating aliphatic side chains. Substrates with primary **(43-50),** secondary **(51),** and tertiary **(36)** aliphatic side chains underwent amination in 48-75% yield and 85-92% ee. The method is also suitable for the late-stage amination of more complex molecules. Accordingly, azanylester formation followed by iron-catalyzed stereocontrolled 1,3-nitrogen shift converted lithocholic acid to amino acid **52** (77% yield, 23:1 dr).

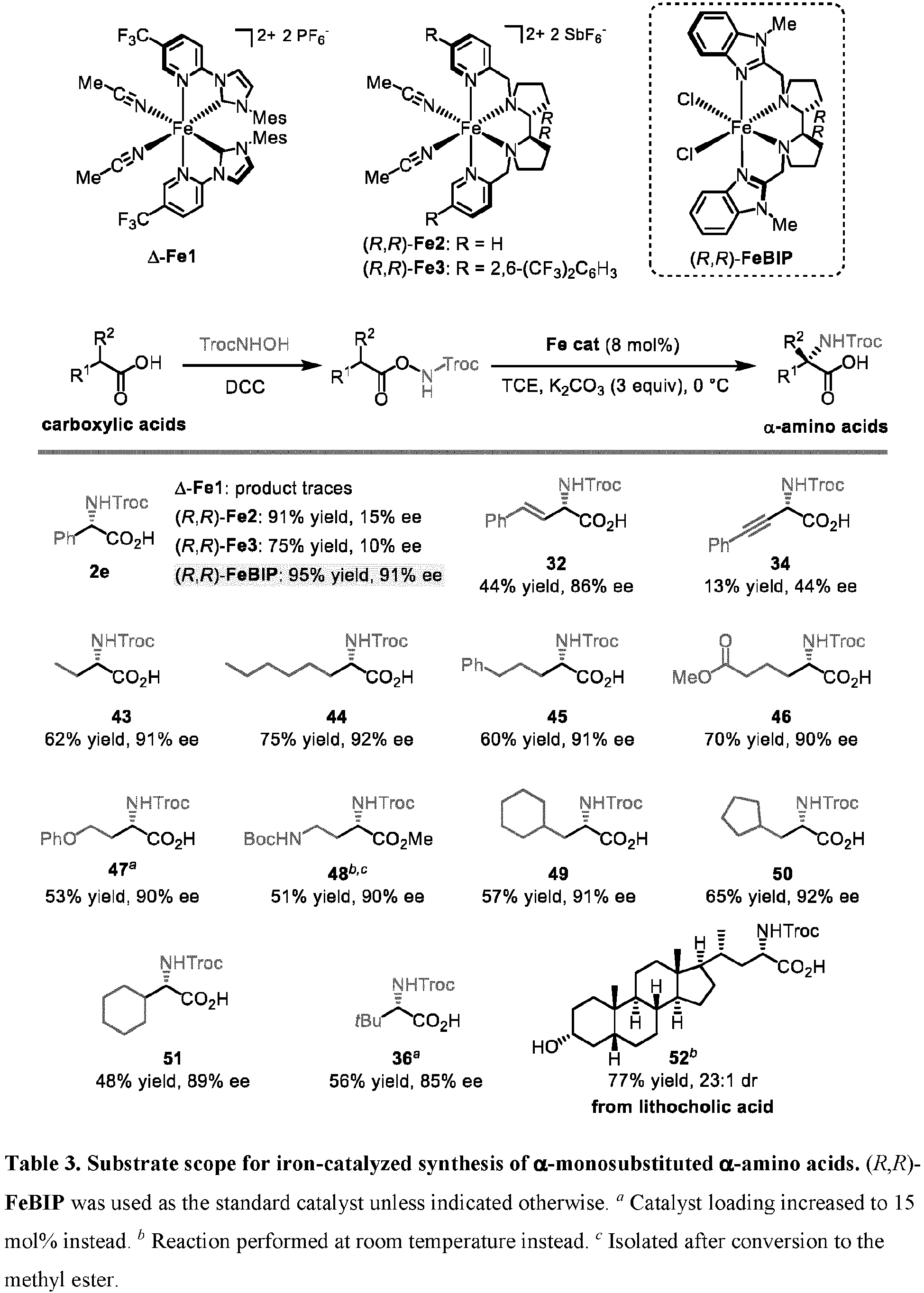

α,α-Disubstituted α-amino acids are interesting due to their restricted conformational flexibility, increase in hydrophobicity, increased metabolic stability, and increased stability towards racemization and this has been used to develop peptides and proteins with enhanced properties as well as natural product antibiotics.^{39,40} The iron-catalyzed protocol can also be used for the amination of tertiary C(sp³)-H bonds, which provides non-racemic α,α-disubstituted α-amino acids as shown in Table 4 for the stereocontrolled synthesis of the α,α-disubstituted α-amino acids **53-83** in up to 88% ee. The enantiomeric excess could be increased to 99% ee with a single recrystallization as investigated for selected examples. Importantly, the method starts from racemic α-branched carboxylic acids. After conversion to the respective racemic azanyl esters via a DCC/EDCI-coupling, the iron-catalyzed rearrangement provides in an enantioconvergent 1,3-nitrogen shift the respective enantioenriched α,α-disubstituted α-amino acids.

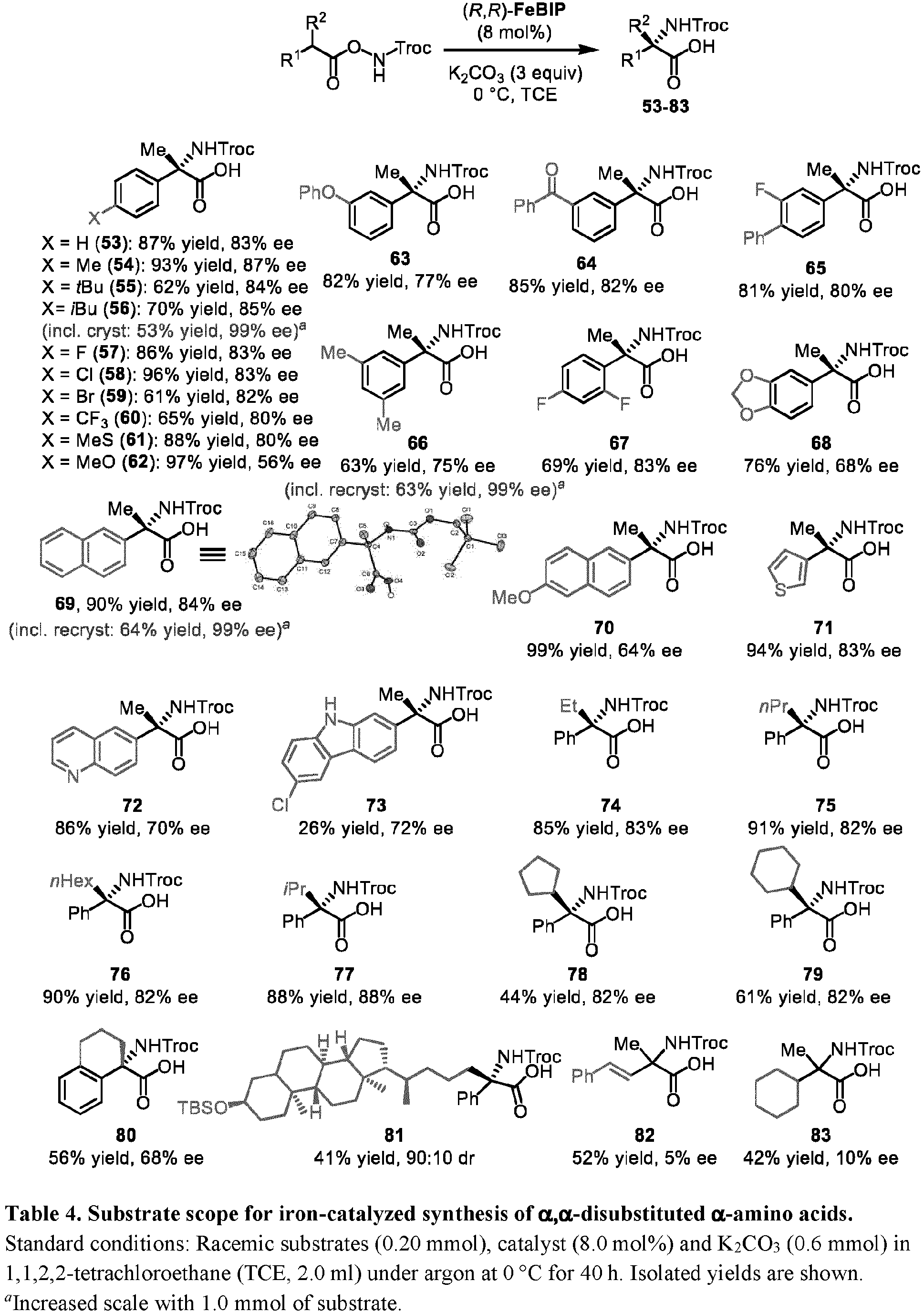

The method according to the present invention is not limited to the Troc-protection group. Table 5 demonstrates that the method according to the present invention can be applied to the known Boc-protection group.⁴¹ In particular, the base 2,2,6,6-tetramethylpiperidine (0.5 equiv.) is used instead of K₂CO₃.

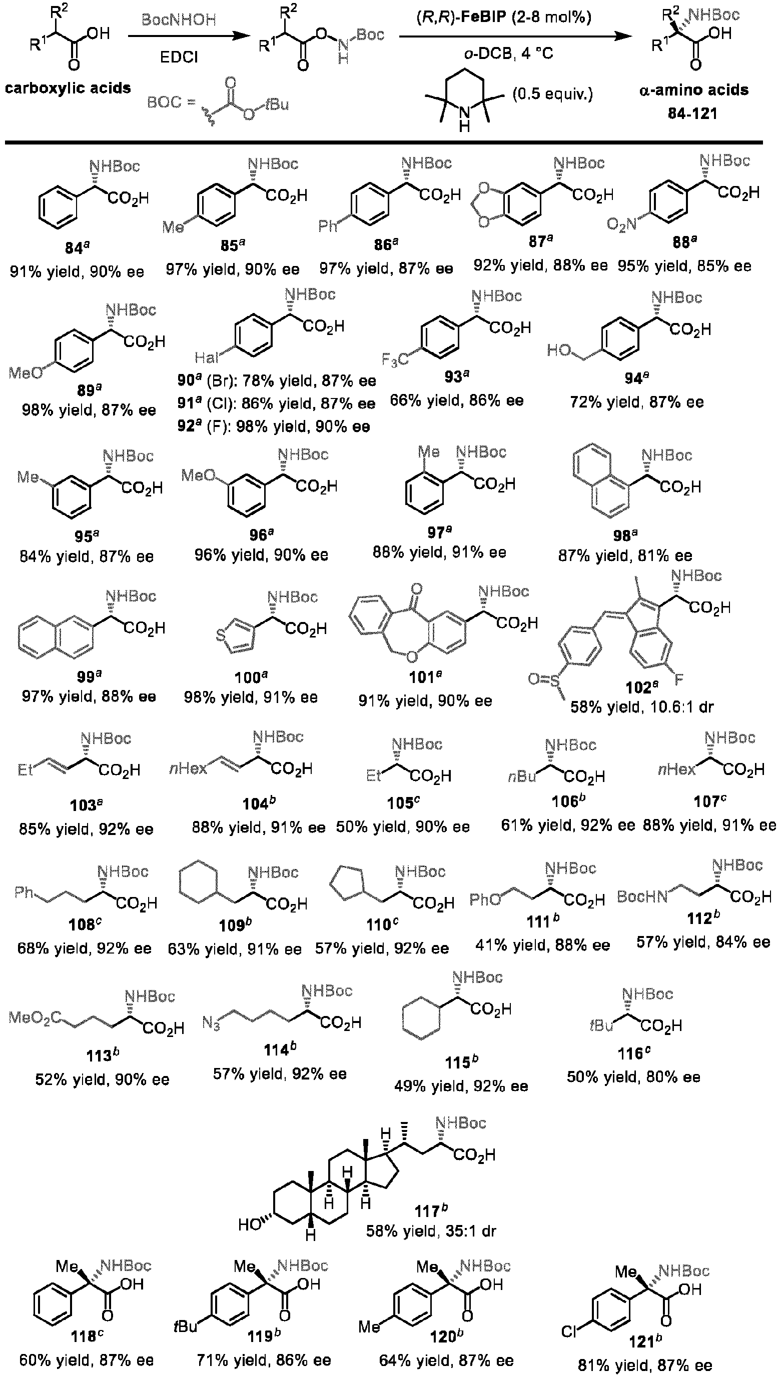

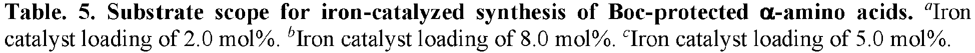

It is also worth noting, that the method can be applied to the synthesis of α-deuterated α-amino acids as shown for one example in the following equation showing the iron-catalyzed synthesis of a Boc-protected α-deuterated α-amino acid (**122→123**).

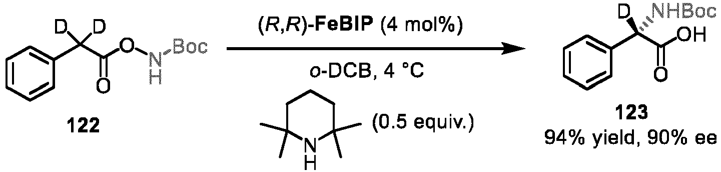

In a further embodiment, the α-amino acid which may contain the protecting group, can be recrystallized so that the enantiopurity can bee increased. An example of a solvent to be used in the recrystallization can be a mixture of ethanol and n-hexane. An example of a recrystallization protocol is represented in Fig. 5.

In conclusion, the method according to the present invention provides the catalytic enantioselective synthesis of chiral α-amino acids by nitrene C(sp³)-H insertion. The method is based on a unique stereocontrolled 1,3-nitrogen shift from one carboxylic acid oxygen to the α-carbon. The method according to the present invention employs abundant and easily accessible carboxylic acids as starting materials. Ligation to a nitrene precursor, followed by intramolecular C-H cleavage through a cyclic transition state, ensures high regio- and stereocontrol in the synthesis of non-racemic chiral α-amino acids. Chiral ruthenium and iron catalysis jointly provide a very broad scope, enabling rapid access to optically active α-amino acids with aryl, allyl, propargyl (ruthenium catalysis), and non-activated alkyl (iron catalysis) side chains, including α,α-disubstituted amino acids by stereoretentive (ruthenium catalysis) or enantioconvergent (iron catalysis) C-H amination. The high functional group tolerance of this method also permits the enantioselective late-stage amination of carboxylic acid-containing drugs and natural products. Also worth emphasizing, this new 1,3-nitrogen migration method is also suitable for synthesizing racemic α-amino acids in a practical and economical fashion by using inexpensive achiral or racemic catalysts. This method according to the present invention will expedite the synthesis of unnatural α-amino acids, which are important building blocks of peptidomimetic drugs, as well as engineered proteins and enzymes with modulated properties.⁴²⁻⁴⁴

### References

1. Saghyan, A. S. & Langer, P. Asymmetric Synthesis of Non-Proteinogenic Amino Acids (Wiley-VCH, Weinheim, 2016).
2. Nájera, C. & Sansano, J. M. Catalytic asymmetric synthesis of α-amino acids. Chem. Rev. 107, 4584-4671 (2007).
3. Janey, J. M. Recent advances in catalytic, enantioselective α aminations and α oxygenations of carbonyl compounds. Angew. Chem. Int. Ed. 44, 4292-4300 (2005).
4. Bøgevig, A., Juhl, K., Kumaragurubaran, N., Zhuang, W. & Jorgensen, K. A. Direct organo-catalytic asymmetric α-amination of aldehydes-a simple approach to optically active α-amino aldehydes, α-amino alcohols, and α-amino acids. Angew. Chem. Int. Ed. 41, 1790-1793 (2002).
5. List, B. Direct catalytic asymmetric α-amination of aldehydes. J. Am. Chem. Soc. 124, 5656-5657 (2002).
6. Kumaragurubaran, N., Juhl, K., Zhuang, W., Bøgevig, A. & Jørgensen, K. A. Direct L-proline-catalyzed asymmetric α-amination of ketones. J. Am. Chem. Soc. 124, 6254-6255 (2002).
7. Morrill, L. C., Lebl, T., Slawin, A. M. Z. & Smith, A. D. Catalytic asymmetric α-amination of carboxylic acids using isothioureas. Chem. Sci. 3, 2088-2093 (2012).
8. Dequirez, G., Pons, V. & Dauban, P. Nitrene chemistry in organic synthesis: still in its infancy? Angew. Chem. Int. Ed. 51, 7384-7395 (2012).
9. Park, Y., Kim, Y. & Chang, S. Transition metal-catalyzed C-H amination: scope, mechanism, and applications. Chem. Rev. 117, 9247-9301 (2017).
10. Collet, F., Lescot, C. & Dauban, P. Catalytic C-H amination: the stereoselectivity issue. Chem. Soc. Rev. 40, 1926-1936 (2011).
11. Nägeli, I., Baud, C., Bemardinelli, G., Jacquier, Y., Moraon, M. & Müllet, P. Rhodium(II)-catalyzed CH insertions with {[(4-nitrophenyl)sulfonyl]imino}phenyl-λ3-iodane. Helv. Chim. Acta 80, 1087-1105 (1997).
12. Zhou, X.-G., Yu, X.-Q., Huang, J.-S. & Che, C.-M. Asymmetric amidation of saturated C-H bonds catalysed by chiral ruthenium and manganese porphyrins. Chem. Commun. 2377-2378 (1999).
13. Kohmura, Y. & Katsuki, T. Mn(salen)-catalyzed enantioselective C-H amination. Tetrahedron Lett. 42, 3339-3342 (2001).
14. Yamawaki, M., Tsutsui, H., Kitagaki, S., Anada, M. & Hashimoto, S. Dirhodium(II) tetrakis[N-tetrachlorophthaloyl-(S)-tert-leucinate]: a new chiral Rh(II) catalyst for enantioselective amidation of C-H bonds. Tetrahedron Lett. 43, 9561-9564 (2002).
15. Liang, C., Robert-Peillard, F., Fruit, C., Müller, P., Dodd, R. H. & Dauban, P. Efficient diastereoselective intermolecular rhodium-catalyzed C-H amination. Angew. Chem. Int. Ed. 45, 4641-4644 (2006).
16. Nishioka, Y., Uchida, T. & Katsuki, T. Enantio- and regioselective intermolecular benzylic and allylic C-H bond amination. Angew. Chem. Int. Ed. 52, 1739-1742 (2013).
17. Höke, T., Herdtweck, E. & Bach, T. Hydrogen-bond mediated regio- and enantioselectivity in a C-H amination reaction catalysed by a supramolecular Rh(II) complex. Chem. Commun. 49, 8009-8011 (2013).
18. Nasrallah, A., Boquet, V., Hecker, A., Retailleau, P., Darses, B. & Dauban, P. Catalytic enantioselective intermolecular benzylic C(sp3)-H Amination. Angew. Chem. Int. Ed. 58, 8192-8196 (2019).
19. Jin, L.-M., Xu, P., Xie, J. & Zhang, X. P. Enantioselective intermolecular radical C-H amination. J. Am. Chem. Soc. 142, 20828-20836 (2020).
20. Liang, J.-L., Yuan, S.-X., Huang, J.-S., Yu, W.-Y. & Che, C.-M. Highly diastereo- and enantioselective intramolecular amidation of saturated C-H bonds catalyzed by ruthenium porphyrins. Angew. Chem. Int. Ed. 41, 3465-3468 (2002).
21. Milczek, E., Boudet, N. & Blakey, S. Enantioselective C-H amination using cationic ruthenium(II)-pybox catalysts. Angew. Chem. Int. Ed. 47, 6825-6828 (2008).
22. Ichinose, M., Suematsu, H., Yasutomi, Y., Nishioka, Y., Uchida, T. & Katsuki, T. Enantioselective intramolecular benzylic C-H bond amination: efficient synthesis of optically active benzosultams. Angew. Chem. Int. Ed. 50, 9884-9887 (2011).
23. Zalatan, D. N. & Du Bois, J. A chiral rhodium carboxamidate catalyst for enantioselective C-H amination. J. Am. Chem. Soc. 130, 9220-9221 (2008).
24. Lang, K., Torker, S., Wojtas, L. & Zhang, X. P. Asymmetric induction and enantiodivergence in catalytic radical C-H amination via enantiodifferentiative H-atom abstraction and stereoretentive radical substitution. J. Am. Chem. Soc. 141, 12388-12396 (2019).
25. Park, Y. & Chang, S. Asymmetric formation of γ-lactams via C-H amidation enabled by chiral hydrogen-bond-donor catalysts. Nat. Catal. 2, 219-227 (2019).
26. Van Vliet, K. M. & de Bruin, B. Dioxazolones: stable substrates for the catalytic transfer of acyl nitrenes. ACS Catal. 2020, 10, 4751-4769.
27. Zheng, Y., Tan, Y., Harms, K., Marsch, M., Riedel, R., Zhang, L. & Meggers, E. Octahedral ruthenium complex with exclusive metal-centered chirality for highly effective asymmetric catalysis. J. Am. Chem. Soc. 139, 4322-4325 (2017).
28. Zhou, Z., Chen, S., Qin, J., Nie, X., Zheng, X., Harms, K., Riedel, R., Houk, K. N. & Meggers, E. Catalytic enantioselective intramolecular C(sp3)-H amination of 2-azidoacetamides. Angew. Chem. Int. Ed. 58, 1088-1093 (2019).
29. Zhou, Z., Tan, Y., Yamahira, T., Ivlev, S., Xie, X., Riedel, R., Hemming, M., Kimura, M. & Meggers, E. Enantioselective ring-closing C-H amination of urea derivatives. Chem 6, 2024-2034 (2020).
30. Thirumurugan, P., Matosiuk, D. & Jozwiak, K. Click chemistry for drug development and diverse chemical-biology applications. Chem. Rev. 113, 4905-4979 (2013).
31. Ueno, K., Kubo, S., Tagawa, H., Yoshioka, T., Tsukada, W., Tsubokawa, M., Kojima, H. & Kasahara, A. 6,11-Dihydro-11-oxodibenz[b,e]oxepinacetic acids with potent antiinflammatory activity. J. Med. Chem. 19, 941-946 (1976).
32. Krupp, P. J., Menassé-Gdynia, R., Sallmann, A., Wilhelmi, G., Ziel R. & Jaques, R. Sodium [o-[(2,6-dichlorophenyl)-amino]-phenyl]-acetate (GP 45 840), a new non-steroidal anti-inflammatory agent. Experientia 29, 450-452 (1973).
33. Bauer, I. & Knölker, H.-J. Iron Catalysis in Organic Synthesis. Chem. Rev. 115, 3170-3387 (2015).
34. Liu, Y., You, T., Wang, H.-X., Tang, Z., Zhou, C.-Y. & Che, C.-M. Iron- and cobalt-catalyzed C(sp3)-H bond functionalization reactions and their application in organic synthesis. Chem. Soc. Rev. 49, 5310-5358 (2020).
35. Hong, Y., Jarrige, L., Harms, K., Meggers, E. Chiral-at-Iron Catalyst: Expanding the Chemical Space for Asymmetric Earth-Abundant Metal Catalysis. J. Am. Chem. Soc. 141, 4569-4572 (2019).
36. Chen, M. S. & White, M. C. A Predictably Selective Aliphatic C-H Oxidation Reaction for Complex Molecule Synthesis. Science 318, 783-787 (2007).
37. White, M. C. & Zhao, J. Aliphatic C-H Oxidations for Late-Stage Functionalization. J. Am. Chem. Soc. 140, 13988-14009 (2018).
38. Mitra, M., Cusso, O., Bhat, S. S., Sun, M., Cianfanelli, M., Costas, M. & Nordlander, E. Highly enantioselective epoxidation of olefins by H2O2 catalyzed by a non-heme Fe(II) catalyst of a chiral tetradentate ligand. Dalton Trans. 48, 6123-6131 (2019).
39. Toniolo, C., Formaggio, F., Kaptein, B. & Broxterman, Q. B. You Are Sitting on a Gold Mine! Synlett 1295-1310 (2006).
40. Tanaka, M. Design and Synthesis of Chiral α,α-Disubstituted Amino Acids and Conformational Study of Their Oligopeptides. Chem. Pharm. Bull. 55, 349-358 (2007).
41. Isidro-Llobet, A., Alvarez, M. & Albericio, F. Amino acid-protecting groups. Chem. Rev. 109, 2455-2504 (2009).
42. Blaskovich, M. A. T. Unusual Amino Acids in Medicinal Chemistry. J. Med. Chem. 59, 10807-10836 (2016).
43. Agostini, F., Völler, J.-S., Koksch, B., Acevedo-Rocha, C. G., Kubyshkin, V. & Budisa, N. Biocatalysis with Unnatural Amino Acids: Enzymology Meets Xenobiology. Angew. Chem. Int. Ed. 56, 9680-9703 (2017).
44. Drienovská, I. & Roelfes, G. Expanding the enzyme universe with genetically encoded unnatural amino acids. Nat. Catal. 3, 193-202 (2020).

### Examples

### 1. General Information

Catalytic reactions were performed in Schlenk tubes (10 mL) under nitrogen atmosphere with magnetic stirring. Chemicals were used as received from commercial suppliers unless stated otherwise. Anhydrous CH₃CN and CH₂Cl₂ were distilled under nitrogen from calcium hydride. Anhydrous THF was distilled under nitrogen from sodium/benzophenone. Anhydrous 1,1,2,2-tetrachloroethane (TCE) and 1,2-dichlorobenzene (o-DCB) was used as received from commercial suppliers. Flash column chromatography was performed with silica gel 60 M from Macherey-Nagel (230-400 mesh). Infrared (IR) spectra were measured on a Bruker Alpha FT-IR spectrometer. ¹H, ¹³C and ¹⁹F NMR spectra were recorded on a Bruker Avance 300 MHz spectrometer at ambient temperature. Chemical shifts are expressed in parts per million (δ) referenced to chloroform (7.26 ppm or 77.16 ppm), dichloromethane (5.32 ppm or 53.84 ppm), methanol (3.31 ppm or 49.00 ppm), and trichlorofluoromethane (0.00 ppm, for ¹⁹F NMR). The NMR data are presented as follows: chemical shift, multiplicity (s = singlet, d = doublet, t = triplet, q = quartet, dd = doublet of doublet, dt = doublet of triplet, td = triplet of doublet, ddd = doublet of doublet of doublet, etc., m = multiplet, br = broad), coupling constant and integration. High resolution mass spectra (HRMS) were recorded on a Bruker En Apex Ultra 7.0 T FT-MS mass spectrometer. Optical rotations were measured using a Perkin-Elmer 241 polarimeter with [α]_{D}²⁵ values reported in degrees with concentrations reported in g/100 mL. Enantiomeric excesses (e.e.) were determined by HPLC analysis using an Agilent HPLC 1260, or by GC analysis using an Agilent GC 7820A, on chiral stationary phases.

### 2. Catalyst Synthesis

### Synthesis of ruthenium catalysts A-RuDMP and A-RuH:

Ruthenium catalysts **A-RuDMP** and **A-RuH** were prepared following the reported procedures.¹

Characterization data of **A-RuDMP:** ¹H NMR (300 MHz, CD₂Cl₂) δ 1.51 (s, 6H), 1.98 (s, 6H), 2.02 (s, 6H), 2.31 (s, 6H), 2.47 (s, 12H), 6.58 (s, 2H), 6.60 (s, 2H), 6.92 (d, *J* = 2.3 Hz, 2H), 7.19 (s, 2H), 7.20 (s, 4H), 7.58 (d, *J* = 8.6 Hz, 2H), 8.00 (d, *J* = 2.3 Hz, 2H), 8.04 (dd, *J* = 8.6, 2.2 Hz, 2H), 8.50 (d, *J* = 2.2 Hz, 2H).

Characterization data of **A-RuH:** ¹H NMR (300 MHz, CD₂Cl₂) δ 1.45 (s, 6H), 1.98 (s, 6H), 2.19 (s, 6H), 2.29 (s, 6H), 6.52 (s, 2H), 6.75 (s, 2H), 6.92 (d, *J* = 2.2 Hz, 2H), 7.06 (t, *J* = 6.6 Hz, 2H), 7.46 (d, *J* = 8.3 Hz, 2H), 7.82 (t, *J* = 7.9 Hz, 2H), 7.92 (d, *J* = 2.2 Hz, 2H), 8.36 (d, *J* = 5.7 Hz, 2H).

The ¹H NMR data of **A-RuDMP** and **A-RuH** matched those reported in the literature.¹

### Synthesis of iron catalyst (R,R)-[FeCl₂(BIP)]:

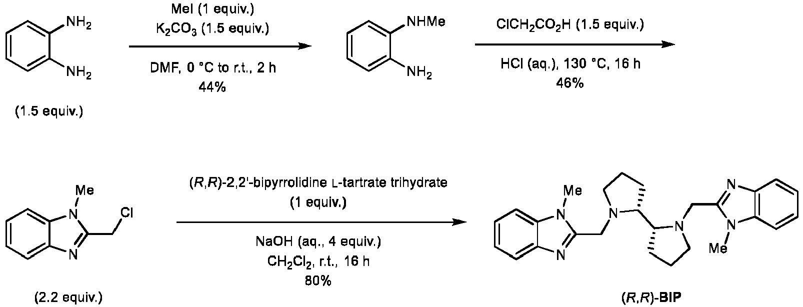

Preparation of the ligand: to a solution of benzene-1,2-diamine (2.43 g, 22.5 mmol, 1.5 equiv.) and potassium carbonate (3.10 g, 22.5 mmol, 1.5 equiv.) in N,N-dimethylformamide (30 mL) at 0 °C was added iodomethane (0.93 mL, 15 mmol, 1 equiv.) dropwise. The reaction mixture was warmed to room temperature and was stirred for 2 hours. After completion, the reaction mixture was diluted with water (100 mL) and was extracted with EtOAc for two times. The combined organic layer was washed with brine and was dried over anhydrous sodium sulfate. After filtration, the solvent was evaporated under reduced pressure, and the residue was purified by column chromatography (silica gel, eluent: EtOAc/n-hexane = 1/3) to afford *N*₁-methylbenzene-1,2-diamine as a yellow oil (815 mg, 44% yield).

To a solution of *N*₁-methylbenzene-1,2-diamine (815 mg, 6.7 mmol, 1 equiv.) in hydrochloric acid (aqueous solution, 2 M, 13 mL) was added 2-chloroacetic acid (945 mg, 10 mmol, 1.5 equiv.). The reaction mixture was refluxed at 130 °C for 16 hours. After completion, the reaction mixture was cooled to 0 °C and was neutralized with sodium hydroxide (aqueous solution, 1 M) until the pH reached 7-8. The reaction mixture was extracted with EtOAc for three times. The combined organic layer was washed with brine and was dried over anhydrous sodium sulfate. After filtration, the solvent was evaporated under reduced pressure, and the residue was purified by column chromatography (silica gel, eluent: EtOAc/*n*-hexane = 1/2) to afford 2-(chloromethyl)-1-methyl-1*H*-benzo[*d*]imidazole as a yellow solid (552 mg, 46% yield).

Following a literature procedure,² to a solution of (R,R)-2,2'-bipyrrolidine L-tartrate trihydrate (465 mg, 1.35 mmol, 1 equiv.) and 2-(chloromethyl)-1-methyl-1*H*-benzo[*d*]imidazole (537 mg, 3.0 mmol, 2.2 equiv.) in dichloromethane (14 mL) at room temperature was added NaOH (aqueous solution, 1 M, 5.4 mL, 5.4 mmol, 4 equiv.). The reaction mixture was stirred at room temperature for 16 hours. After completion, the reaction mixture was diluted with sodium hydroxide (aqueous solution, 1 M, 30 mL) and was extracted with CH₂Cl₂ for three times. The combined organic layer was dried over anhydrous sodium sulfate. After filtration, the solvent was evaporated under reduced pressure, and the residue was purified by column chromatography (silica gel, eluent: MeOH/CH₂Cl₂ = 1/20 with 0.1% NH₃•H₂O) to afford (*R,R*)-**BIP** as a yellow solid (465 mg, 80% yield): ¹H NMR (300 MHz, CDCl₃) δ 1.50-1.93 (m, 8H), 2.15-2.51 (m, 2H), 2.62-2.80 (m, 2H), 2.80-3.00 (m, 2H), 3.53-3.75 (m, 2H), 3.80 (s, 6H), 4.24 (d, *J* = 13.4 Hz, 2H), 7.17-7.34 (m, 6H), 7.65-7.78 (m, 2H); ¹³C NMR (75 MHz, CDCl₃) δ 24.1, 26.5, 30.0, 52.5, 55.6, 65.7, 109.1, 119.6, 122.0, 122.6, 136.1, 142.1, 152.3.

Synthetic procedure for (*R*,*R*)-[FeCl₂(**BIP**)]: to a stirred solution of the ligand (*R,R*)-**BIP** (990 mg, 2.3 mmol, 1.05 equiv.) in 13 mL CH₃CN under N₂ atmosphere was added FeCl₂•4H₂O (437 mg, 2.2 mmol, 1 equiv.) in one portion. A yellow precipitate was formed within several seconds. The reaction mixture was stirred for 4 hours at room temperature before being diluted with diethyl ether (40 mL). The slurry was transferred into a centrifuge tube, and the solid material was separated by centrifugation and was washed with a mixed solvent of CH₃CN/diethyl ether (v/v = 1/2.5) for three times (35×3 mL in total). The solid was dried under nitrogen flow to afford (*R*,*R*)-[FeCl₂(**BIP**)] **(FeBIP)** as a yellow powder (1.14 g, 93% yield).

Characterization data of (*R*,*R*)-[FeCl₂(**BIP**)]: HRMS calcd for [C₂₆H₃₂ClFeN₆]⁺ [(M - Cl)⁺]: 519.1726; found: 519.1722; elemental analysis calcd for [C₂₆H₃₂Cl₂FeN₆•2H₂O]: C, 52.81; H, 6.14; N, 14.21; found: C, 52.90; H, 5.82; N, 14.17.

### 3. Synthesis of Azanyl Esters

### 3.1. Synthesis of N-Troc-protected azanyl esters

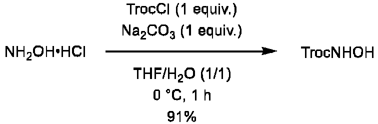

To a solution of hydroxylamine hydrochloride (2.08 g, 30 mmol, 1 equiv.) and Na₂CO₃ (3.18 g, 30 mmol, 1 equiv.) in tetrahydrofuran/water (v/v = 1/1, 60 mL) at 0 °C was added 2,2,2-trichloroethyl carbonochloridate (4.1 mL, 30 mmol, 1 equiv.) dropwise. The reaction mixture was stirred at 0 °C for 1 hour. After completion, the reaction mixture was extracted with EtOAc for three times. The combined organic layer was washed with brine and was dried over anhydrous sodium sulfate. After filtration, the solvent was evaporated under reduced pressure, and the residue was purified by column chromatography (silica gel, eluent: EtOAc/n-hexane = 1/2) to afford 2,2,2-trichloroethyl hydroxycarbamate as a white solid (5.69 g, 91% yield): IR (film) vₘₐₓ: 3360, 3262, 1707, 1502, 1283, 1137, 757, 718 cm⁻¹; ¹H NMR (300 MHz, CDCl₃) δ 4.79 (s, 2H), 6.68-7.12 (br, 1H), 7.51-7.76 (br, 1H); ¹³C NMR (75 MHz, CDCl₃) δ 75.1, 94.9, 157.2; HRMS calcd for [C₃H₄Cl₃NO₃Na]⁺ [(M + Na)⁺]: 229.9149; found: 229.9154.

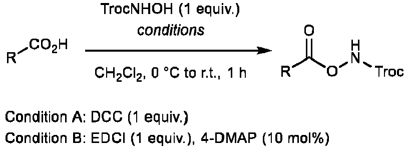

Condition A (DCC coupling): to a solution of carboxylic acid (1 equiv.) and TrocNHOH (1 equiv.) in dichloromethane (0.2 M) at 0 °C was added a solution of DCC (1 equiv.) in dichloromethane (1 mL/mmol DCC) dropwise. The reaction mixture was warmed to room temperature and was stirred for 1 hour. After completion, the reaction mixture was filtered and washed with Et₂O. After being concentrated under reduced pressure, the residue was purified by chromatography on silica gel using indicated solvent as the eluent.

Condition B (EDCI coupling): to a solution of carboxylic acid (1 equiv.), 4-DMAP (10 mol%) and TrocNHOH (1 equiv.) in dichloromethane (0.2 M) at 0 °C was added EDCI (1 equiv.) in one portion. The reaction mixture was warmed to room temperature and was stirred for 1 hour. After completion, water was added, and the mixture was extracted with EtOAc for three times. The combined organic layer was washed with brine and was dried over anhydrous sodium sulfate. After filtration, the solvent was evaporated under reduced pressure, and the residue was purified by chromatography on silica gel using indicated solvent as the eluent.

### 2,2,2-Trichloroethyl (2-phenylacetoxy)carbamate (1e)

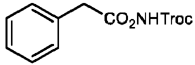

DCC coupling of 2-phenylacetic acid with TrocNHOH gave **1e** as a white solid (chromatography on silica gel, eluent: EtOAc/n-hexane = 1/10): IR (film) vₘₐₓ: 3224, 1761, 1463, 1229, 1119, 712 cm⁻¹ ; ¹H NMR (300 MHz, CDCl₃) δ 3.81 (s, 2H), 4.79 (s, 2H), 7.27-7.40 (m, 5H), 8.24-8.33 (br, 1H); ¹³C NMR (75 MHz, CDCl₃) δ 38.6, 75.3, 94.5, 127.9, 129.0, 129.4, 132.0, 154.6, 170.4; HRMS calcd for [C₁₁H₁₀Cl₃NO₄Na]⁺ [(M + Na)⁺]: 347.9568; found: 347.9571.

### 2,2,2-Trichloroethyl (2-(p-tolyl)acetoxy)carbamate

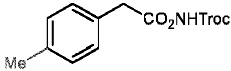

DCC coupling of 2-(p-tolyl)acetic acid with TrocNHOH gave 2,2,2-trichloroethyl (2-(*p*-tolyl)acetoxy)carbamate as a colorless oil (chromatography on silica gel, eluent: EtOAc/n-hexane = 1/10): IR (film) vₘₐₓ: 3239, 1762, 1467, 1227, 1115, 731 cm⁻¹; ¹H NMR (300 MHz, CDCl₃) δ 2.34 (s, 3H), 3.76 (s, 2H), 4.79 (s, 2H), 7.12-7.18 (m, 2H), 7.18-7.24 (m, 2H), 8.25-8.36 (br, 1H); ¹³C NMR (75 MHz, CDCl₃) δ 21.2, 38.2, 75.3, 94.5, 128.9, 129.3, 129.7, 137.6, 154.6, 170.6; HRMS calcd for [C₁₂H₁₂Cl₃NO₄Na]⁺ [(M + Na)⁺]: 361.9724; found: 361.9729.

### 2,2,2-Trichloroethyl (2-([1,1'-biphenyl]-4-yl)acetoxy)carbamate

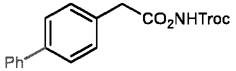

DCC coupling of 2-([1,1'-biphenyl]-4-yl)acetic acid with TrocNHOH gave 2,2,2-trichloroethyl (2-([1,1'-biphenyl]-4-yl)acetoxy)carbamate as a white solid (chromatography on silica gel, eluent: EtOAc/n-hexane = 1/10): IR (film) vₘₐₓ: 3189, 1794, 1727, 1248, 1071, 717 cm⁻¹; ¹H NMR (300 MHz, CDCl₃) δ 3.85 (s, 2H), 4.81 (s, 2H), 7.32-7.50 (m, 5H), 7.53-7.63 (m, 4H), 8.26-8.37 (br, 1H); ¹³C NMR (75 MHz, CDCl₃) δ 38.2, 75.3, 94.5, 127.2, 127.6, 127.7, 129.0, 129.9, 130.9, 140.7, 140.9, 154.6, 170.4; HRMS calcd for [C₁₇H₁₄Cl₃NO₄Na]⁺ [(M + Na)⁺]: 423.9881; found: 423.9884.

### 2,2,2-Trichloroethyl (2-(4-methoxyphenyl)acetoxy)carbamate

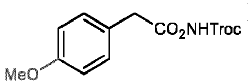

DCC coupling of 2-(4-methoxyphenyl)acetic acid with TrocNHOH gave 2,2,2-trichloroethyl (2-(4-methoxyphenyl)acetoxy)carbamate as a colorless oil (chromatography on silica gel, eluent: EtOAc/n-hexane = 1/6): IR (film) vₘₐₓ: 3264, 1752, 1511, 1244, 1114, 1087, 717 cm⁻¹; ¹H NMR (300 MHz, CDCl₃) δ 3.74 (s, 2H), 3.80 (s, 3H), 4.79 (s, 2H), 6.82-6.93 (m, 2H), 7.19-7.26 (m, 2H), 8.24-8.41 (br, 1H); ¹³C NMR (75 MHz, CDCl₃) δ 37.7, 55.4, 75.3, 94.5, 114.4, 123.9, 130.5, 154.6, 159.3, 170.7; HRMS calcd for [C₁₂H₁₂Cl₃NO₅Na]⁺ [(M + Na)⁺]: 377.9673; found: 377.9676.

### 2,2,2-Trichloroethyl (2-(3-methoxyphenyl)acetoxy)carbamate

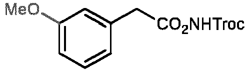

DCC coupling of 2-(3-methoxyphenyl)acetic acid with TrocNHOH gave 2,2,2-trichloroethyl (2-(3-methoxyphenyl)acetoxy)carbamate as a colorless oil (chromatography on silica gel, eluent: EtOAc/n-hexane = 1/10): IR (film) vₘₐₓ: 3267, 1750, 1257, 1092, 754, 718 cm⁻¹; ¹H NMR (300 MHz, CDCl₃) δ 3.77 (s, 2H), 3.80 (s, 3H), 4.79 (s, 2H), 6.80-6.93 (m, 3H), 7.21-7.30 (m, 1H), 8.28-8.40 (br, 1H); ¹³C NMR (75 MHz, CDCl₃) δ 38.6, 55.4, 75.3, 94.5, 113.5, 115.1, 121.7, 130.0, 133.3, 154.6, 160.0, 170.3; HRMS calcd for [C₁₂H₁₂Cl₃NO₅Na]⁺ [(M + Na)⁺]: 377.9673; found: 377.9670.

### 2,2,2-Trichloroethyl (2-(2-methoxyphenyl)acetoxy)carbamate

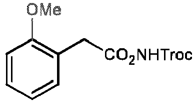

DCC coupling of 2-(2-methoxyphenyl)acetic acid with TrocNHOH gave 2,2,2-trichloroethyl (2-(2-methoxyphenyl)acetoxy)carbamate as a white solid (chromatography on silica gel, eluent: EtOAc/n-hexane = 1/8): IR (film) vₘₐₓ: 3188, 1757, 1463, 1247, 1110, 744, 709 cm⁻¹; ¹H NMR (300 MHz, CDCl₃) δ 3.81 (s, 2H), 3.83 (s, 3H), 4.80 (s, 2H), 6.83-6.91 (m, 1H), 6.91-7.00 (m, 1H), 7.17-7.25 (m, 1H), 7.26-7.35 (m, 1H), 8.17-8.43 (br, 1H); ¹³C NMR (75 MHz, CDCl₃) δ 33.4, 55.6, 75.3, 94.6, 110.7, 120.8, 121.0, 129.4, 131.1, 154.6, 157.6, 170.7; HRMS calcd for [C₁₂H₁₂Cl₃NO₅Na]⁺ [(M + Na)⁺]: 377.9673; found: 377.9682.

### 2,2,2-Trichloroethyl (2-(benzo[d][1,3]dioxol-5-yl)acetoxy)carbamate

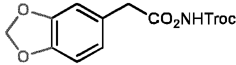

DCC coupling of 2-(benzo[d][1,3]dioxol-5-yl)acetic acid with TrocNHOH gave 2,2,2-trichloroethyl (2-(benzo[d][1,3]dioxol-5-yl)acetoxy)carbamate as a colorless oil (chromatography on silica gel, eluent: EtOAc/n-hexane = 1/8): IR (film) vₘₐₓ: 3279, 1752, 1494, 1244, 1093, 1036, 714 cm⁻¹; ¹H NMR (300 MHz, CDCl₃) δ 3.71 (s, 2H), 4.79 (s, 2H), 5.95 (s, 2H), 6.70-6.78 (m, 2H), 6.78-6.83 (m, 1H), 8.24-8.36 (br, 1H); ¹³C NMR (75 MHz, CDCl₃) δ 38.2, 75.3, 94.5, 101.4, 108.6, 109.8, 122.8, 125.4, 147.4, 148.2, 154.6, 170.4; HRMS calcd for [C₁₂H₁₀Cl₃NO₆Na]⁺ [(M + Na)⁺]: 391.9466; found: 391.9463.

### 2,2,2-Trichloroethyl (2-(4-((((9H-fluoren-9-yl)methoxy)carbonyl)oxy)phenyl)acetoxy)carbamate

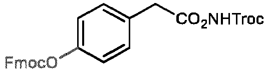

DCC coupling of 2-(4-((((9*H*-fluoren-9-yl)methoxy)carbonyl)oxy)phenyl)acetic acid with TrocNHOH gave 2,2,2-trichloroethyl (2-(4-((((9*H*-fluoren-9-yl)methoxy)carbonyl)oxy)phenyl)acetoxy)carbamate as a colorless oil (chromatography on silica gel, eluent: EtOAc/n-hexane = 1/6): IR (film) vₘₐₓ: 1756, 1216, 1110, 738 cm⁻¹ ; ¹H NMR (300 MHz, CDCl₃) δ 3.80 (s, 2H), 4.33 (t, *J* = 7.3 Hz, 1H), 4.54 (d, *J* = 7.3 Hz, 2H), 4.80 (s, 2H), 7.12-7.22 (m, 2H), 7.29-7.39 (m, 4H), 7.39-7.50 (m, 2H), 7.59-7.70 (m, 2H), 7.74-7.85 (m, 2H), 8.26-8.40 (br, 1H); ¹³C NMR (75 MHz, CDCl₃) δ 37.9, 46.8, 70.7, 75.3, 94.5, 120.3, 121.6, 125.3, 127.4, 128.2, 129.8, 130.6, 141.5, 143.3, 150.8, 153.6, 154.6, 170.0; HRMS calcd for [C₂₆H₂₀Cl₃NO₇Na]⁺ [(M + Na)⁺]: 586.0198; found: 586.0193.

### 2,2,2-Trichloroethyl (2-(4-(hydroxymethyl)phenyl)acetoxy)carbamate

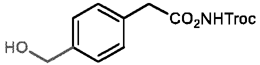

EDCI coupling of 2-(4-(hydroxymethyl)phenyl)acetic acid with TrocNHOH gave 2,2,2-trichloroethyl (2-(4-(hydroxymethyl)phenyl)acetoxy)carbamate as a colorless oil (chromatography on silica gel, eluent: EtOAc/n-hexane = 1/2): IR (film) vₘₐₓ: 3516, 3268, 1744, 1216, 1115, 1091, 717 cm⁻¹ ; ¹H NMR (300 MHz, CDCl₃) δ 2.00-2.11 (br, 1H), 3.78 (s, 2H), 4.65 (s, 2H), 4.77 (s, 2H), 7.24-7.30 (m, 2H), 7.30-7.35 (m, 2H), 8.54-8.69 (br, 1H); ¹³C NMR (75 MHz, CDCl₃) δ 38.2, 65.0, 75.2, 94.5, 127.6, 129.6, 131.3, 140.5, 154.6, 170.4; HRMS calcd for [C₁₂H₁₂Cl₃NO₅Na]⁺ [(M + Na)⁺]: 377.9673; found: 377.9675.

### 2,2,2-Trichloroethyl (2-(4-iodophenyl)acetoxy)carbamate

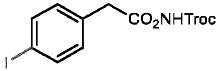

DCC coupling of 2-(4-iodophenyl)acetic acid with TrocNHOH gave 2,2,2-trichloroethyl (2-(4-iodophenyl)acetoxy)carbamate as a white solid (chromatography on silica gel, eluent: EtOAc/n-hexane = 1/10): IR (film) vₘₐₓ: 3209, 1759, 1458, 1228, 1125, 725 cm⁻¹ ; ¹H NMR (300 MHz, CDCl₃) δ 3.74 (s, 2H), 4.79 (s, 2H), 7.01-7.10 (m, 2H), 7.63-7.72 (m, 2H), 8.26-8.39 (br, 1H); ¹³C NMR (75 MHz, CDCl₃) δ 38.1, 75.3, 93.6, 94.4, 131.4, 131.5, 138.1, 154.6, 169.9; HRMS calcd for [C₁₁H₉Cl₃INO₄Na]⁺ [(M + Na)⁺]: 473.8534; found: 473.8538.

### 2,2,2-Trichloroethyl (2-(4-bromophenyl)acetoxy)carbamate

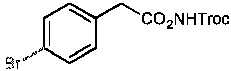

DCC coupling of 2-(4-bromophenyl)acetic acid with TrocNHOH gave 2,2,2-trichloroethyl (2-(4-bromophenyl)acetoxy)carbamate as a white solid (chromatography on silica gel, eluent: EtOAc/n-hexane = 1/10): IR (film) vₘₐₓ: 3332, 1748, 1442, 1209, 1104, 744 cm⁻¹ ; ¹H NMR (300 MHz, CDCl₃) δ 3.76 (s, 2H), 4.79 (s, 2H), 7.14-7.23 (m, 2H), 7.43-7.53 (m, 2H), 8.26-8.38 (br, 1H); ¹³C NMR (75 MHz, CDCl₃) δ 38.0, 75.4, 94.4, 122.1, 130.9, 131.2, 132.1, 154.6, 169.9; HRMS calcd for [C₁₁H₉BrCl₃NO₄Na]⁺ [(M + Na)⁺]: 427.8673; found: 427.8692.

### 2,2,2-Trichloroethyl (2-(4-chlorophenyl)acetoxy)carbamate

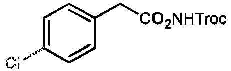

DCC coupling of 2-(4-chlorophenyl)acetic acid with TrocNHOH gave 2,2,2-trichloroethyl (2-(4-chlorophenyl)acetoxy)carbamate as a white solid (chromatography on silica gel, eluent: EtOAc/*n*-hexane = 1/10): IR (film) vₘₐₓ: 3331, 1747, 1442, 1209, 1094, 746 cm⁻¹; ¹H NMR (300 MHz, CDCl₃) δ 3.77 (s, 2H), 4.79 (s, 2H), 7.21-7.29 (m, 2H), 7.29-7.37 (m, 2H), 8.27-8.39 (br, 1H); ¹³C NMR (75 MHz, CDCl₃) δ 37.9, 75.4, 94.4, 129.2, 130.4, 130.8, 134.0, 154.6, 170.0; HRMS calcd for [C₁₁H₉Cl₄NO₄Na]⁺ [(M + Na)⁺]: 383.9148; found: 383.9157.

### 2,2,2-Trichloroethyl (2-(4-fluorophenyl)acetoxy)carbamate

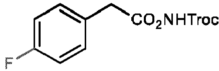

DCC coupling of 2-(4-fluorophenyl)acetic acid with TrocNHOH gave 2,2,2-trichloroethyl (2-(4-fluorophenyl)acetoxy)carbamate as a white solid (chromatography on silica gel, eluent: EtOAc/n-hexane = 1/10): IR (film) vₘₐₓ: 3180, 1725, 1510, 1130, 1096, 734 cm⁻¹; ¹H NMR (300 MHz, CDCl₃) δ 3.77 (s, 2H), 4.78 (s, 2H), 6.97-7.09 (m, 2H), 7.22-7.33 (m, 2H), 8.25-8.42 (br, 1H); ¹³C NMR (75 MHz, CDCl₃) δ 37.7, 75.3, 94.5, 115.9 (d, *J* = 21.7 Hz), 127.7 (d, *J* = 3.3 Hz), 131.1 (d, *J* = 8.2 Hz), 154.6, 162.5 (d, *J* = 246.2 Hz), 170.3; ¹⁹F NMR (282 MHz, CDCl₃) δ -115.0; HRMS calcd for [C₁₁H₉Cl₃FNO₄Na]⁺ [(M + Na)⁺]: 365.9473; found: 365.9478.

### 2,2,2-Trichloroethyl (2-(4-(trifluoromethyl)phenyl)acetoxy)carbamate

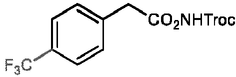

DCC coupling of 2-(4-(trifluoromethyl)phenyl)acetic acid with TrocNHOH gave 2,2,2-trichloroethyl (2-(4-(trifluoromethyl)phenyl)acetoxy)carbamate as a colorless oil (chromatography on silica gel, eluent: EtOAc/n-hexane = 1/10): IR (film) vₘₐₓ: 3182, 1725, 1322, 1117, 1062 cm⁻¹; ¹H NMR (300 MHz, CDCl₃) δ 3.87 (s, 2H), 4.79 (s, 2H), 7.40-7.49 (m, 2H), 7.57-7.66 (m, 2H), 8.29-8.41 (br, 1H); ¹³C NMR (75 MHz, CDCl₃) δ 38.3, 75.4, 94.4, 124.1 (q, *J* = 272.4 Hz), 126.0 (q, *J* = 3.8 Hz), 129.9, 130.4 (q, *J* = 32.8 Hz), 135.9, 154.6, 169.7; ¹⁹F NMR (282 MHz, CDCl₃) δ -63.2; HRMS calcd for [C₁₂H₉Cl₃F₃NO₄Na]⁺ [(M + Na)⁺]: 415.9441; found: 415.9455.

### 2,2,2-Trichloroethyl (2-(4-nitrophenyl)acetoxy)carbamate

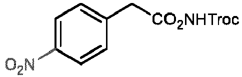

DCC coupling of 2-(4-nitrophenyl)acetic acid with TrocNHOH gave 2,2,2-trichloroethyl (2-(4-nitrophenyl)acetoxy)carbamate as a light yellow oil (chromatography on silica gel, eluent: EtOAc/n-hexane = 1/6): IR (film) vₘₐₓ: 3284, 1752, 1519, 1344, 1097, 716 cm⁻¹; ¹H NMR (300 MHz, CDCl₃) δ 3.92 (s, 2H), 4.79 (s, 2H), 7.42-7.59 (m, 2H), 8.14-8.29 (m, 2H), 8.29-8.43 (br, 1H); ¹³C NMR (75 MHz, CDCl₃) δ 38.2, 75.4, 94.4, 124.2, 130.5, 139.1, 147.8, 154.5, 169.2; HRMS calcd for [C₁₁H₉Cl₃N₂O₆Na]⁺ [(M + Na)⁺]: 392.9418; found: 392.9423.

### 2,2,2-Trichloroethyl (2-(4-acetylphenyl)acetoxy)carbamate

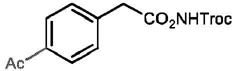

EDCI coupling of 2-(4-acetylphenyl)acetic acid with TrocNHOH gave 2,2,2-trichloroethyl (2-(4-acetylphenyl)acetoxy)carbamate as a white solid (chromatography on silica gel, eluent: EtOAc/n-hexane = 1/3): IR (film) vₘₐₓ: 3246, 1749, 1667, 1121, 1093, 704 cm⁻¹; ¹H NMR (300 MHz, CDCl₃) δ 2.60 (s, 3H), 3.87 (s, 2H), 4.79 (s, 2H), 7.37-7.46 (m, 2H), 7.90-7.99 (m, 2H), 8.30-8.43 (br, 1H); ¹³C NMR (75 MHz, CDCl₃) δ 26.8, 38.5, 75.4, 94.4, 129.0, 129.8, 136.7, 137.1, 154.5, 169.7, 197.7; HRMS calcd for [C₁₃H₁₂Cl₃NO₅Na]⁺ [(M + Na)⁺]: 389.9673; found: 389.9683.

### Methyl 4-(2-oxo-2-((((2,2,2-trichloroethoxy)carbonyl)amino)oxy)ethyl)benzoate

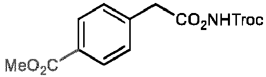

DCC coupling of 2-(4-(methoxycarbonyl)phenyl)acetic acid with TrocNHOH gave methyl 4-(2-oxo-2-((((2,2,2-trichloroethoxy)carbonyl)amino)oxy)ethyl)benzoate as a white solid (chromatography on silica gel, eluent: EtOAc/n-hexane = 1/5): IR (film) vₘₐₓ: 3236, 1753, 1690, 1291, 1118, 1090, 742 cm⁻¹; ¹H NMR (300 MHz, CDCl₃) δ 3.84 (s, 2H), 3.90 (s, 3H), 4.78 (s, 2H), 7.34-7.42 (m, 2H), 7.96-8.04 (m, 2H), 8.48-8.60 (br, 1H); ¹³C NMR (75 MHz, CDCl₃) δ 38.5, 52.3, 75.3, 94.5, 129.5, 129.8, 130.2, 137.0, 154.6, 166.9, 169.7; HRMS calcd for [C₁₃H₁₂Cl₃NO₆Na]⁺ [(M + Na)⁺]: 405.9622; found: 405.9638.

### 2,2,2-Trichloroethyl (2-(4-pivalamidophenyl)acetoxy)carbamate

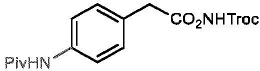

EDCI coupling of 2-(4-pivalamidophenyl)acetic acid with TrocNHOH gave 2,2,2-trichloroethyl (2-(4-pivalamidophenyl)acetoxy)carbamate as a white solid (chromatography on silica gel, eluent: EtOAc/n-hexane = 1/4): IR (film) vₘₐₓ: 3371, 3126, 2959, 1742, 1525, 1124, 1074, 714 cm⁻¹ ; ¹H NMR (300 MHz, CD₃CN) δ 1.25 (s, 9H), 3.76 (s, 2H), 4.82 (s, 2H), 7.18-7.29 (m, 2H), 7.48-7.60 (m, 2H), 7.86-8.04 (br, 1H), 9.12-9.36 (br, 1H); ¹³C NMR (75 MHz, CD₃CN) δ 27.7, 38.2, 40.2, 75.5, 95.8, 121.7, 129.0, 130.6, 139.3, 155.7, 171.3, 177.8; HRMS calcd for [C₁₆H₂₀Cl₃N₂O₅]⁺ [(M + H)⁺]: 425.0432; found: 425.0428.

### 2,2,2-Trichloroethyl (2-(4-azidophenyl)acetoxy)carbamate

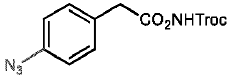

DCC coupling of 2-(4-azidophenyl)acetic acid with TrocNHOH gave 2,2,2-trichloroethyl (2-(4-azidophenyl)acetoxy)carbamate as a light yellow oil (chromatography on silica gel, eluent: EtOAc/n-hexane = 1/10): IR (film) vₘₐₓ: 3270, 2112, 1750, 1284, 1113, 1090, 716 cm⁻¹; ¹H NMR (300 MHz, CDCl₃) δ 3.78 (s, 2H), 4.79 (s, 2H), 6.96-7.05 (m, 2H), 7.27-7.34 (m, 2H), 8.29-8.46 (br, 1H); ¹³C NMR (75 MHz, CDCl₃) δ 37.9, 75.3, 94.5, 119.6, 128.5, 130.9, 139.9, 154.6, 170.2; HRMS calcd for [C₁₁H₉Cl₃N₄O₄Na]⁺ [(M + Na)⁺]: 388.9582; found: 388.9581.

### 2,2,2-Trichloroethyl (2-(4-ethynylphenyl)acetoxy)carbamate

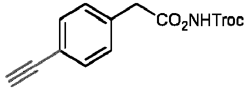

DCC coupling of 2-(4-ethynylphenyl)acetic acid with TrocNHOH gave 2,2,2-trichloroethyl (2-(4-ethynylphenyl)acetoxy)carbamate as a colorless oil (chromatography on silica gel, eluent: EtOAc/n-hexane = 1/10): IR (film) vₘₐₓ: 3289, 1750, 1213, 1092, 717 cm⁻¹; ¹H NMR (300 MHz, CDCl₃) δ 3.09 (s, 1H), 3.80 (s, 2H), 4.79 (s, 2H), 7.23-7.31 (m, 2H), 7.43-7.51 (m, 2H), 8.27-8.40 (br, 1H); ¹³C NMR (75 MHz, CDCl₃) δ 38.4, 75.3, 77.9, 83.2, 94.4, 121.9, 129.5, 132.6, 132.7, 154.6, 169.9; HRMS calcd for [C₁₃H₁₀Cl₃NO₄Na]⁺ [(M +Na)⁺]: 371.9568; found: 371.9580.

### 2,2,2-Trichloroethyl (2-(naphthalen-2-yl)acetoxy)carbamate

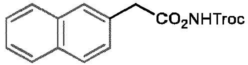

DCC coupling of 2-(naphthalen-2-yl)acetic acid with TrocNHOH gave 2,2,2-trichloroethyl (2-(naphthalen-2-yl)acetoxy)carbamate as a white solid (chromatography on silica gel, eluent: EtOAc/n-hexane = 1/8): IR (film) vₘₐₓ: 3322, 1749, 1445, 1206, 1098, 807, 719 cm⁻¹; ¹H NMR (300 MHz, CDCl₃) δ 3.96 (s, 2H), 4.79 (s, 2H), 7.39-7.45 (m, 1H), 7.45-7.54 (m, 2H), 7.74-7.79 (m, 1H), 7.79-7.88 (m, 3H), 8.27-8.41 (br, 1H); ¹³C NMR (75 MHz, CDCl₃) δ 38.8, 75.3, 94.5, 126.4, 126.6, 127.1, 127.8, 127.9, 128.5, 128.8, 129.4, 132.8, 133.6, 154.6, 170.4; HRMS calcd for [C₁₅H₁₂Cl₃NO₄Na]⁺ [(M + Na)⁺]: 397.9724; found: 397.9734.

### 2,2,2-Trichloroethyl (2-(naphthalen-1-yl)acetoxy)carbamate

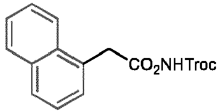

DCC coupling of 2-(naphthalen-1-yl)acetic acid with TrocNHOH gave 2,2,2-trichloroethyl (2-(naphthalen-1-yl)acetoxy)carbamate as a white solid (chromatography on silica gel, eluent: EtOAc/n-hexane = 1/4): IR (film) vₘₐₓ: 3200, 1761, 1221, 1121, 776 cm⁻¹; ¹H NMR (300 MHz, CDCl₃) δ 4.25 (s, 2H), 4.77 (s, 2H), 7.41-7.48 (m, 2H), 7.48-7.61 (m, 2H), 7.80-7.86 (m, 1H), 7.86-7.92 (m, 1H), 7.94-8.01 (m, 1H), 8.21-8.32 (br, 1H); ¹³C NMR (75 MHz, CDCl₃) δ 36.3, 75.3, 94.5, 123.6, 125.6, 126.2, 126.9, 128.4, 128.5, 128.9, 129.0, 132.0, 134.0, 154.6, 170.4; HRMS calcd for [C₁₅H₁₂Cl₃NO₄Na]⁺ [(M + Na)⁺]: 397.9724; found: 397.9735.

### 2,2,2-Trichloroethyl (2-(thiophen-3-yl)acetoxy)carbamate

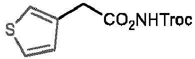

DCC coupling of 2-(thiophen-3-yl)acetic acid with TrocNHOH gave 2,2,2-trichloroethyl (2-(thiophen-3-yl)acetoxy)carbamate as a white solid (chromatography on silica gel, eluent: EtOAc/n-hexane = 1/10): IR (film) vₘₐₓ: 3215, 1757, 1230, 1106, 726 cm⁻¹; ¹H NMR (300 MHz, CDCl₃) δ 3.85 (s, 2H), 4.80 (s, 2H), 7.07 (dd, *J* = 4.9, 1.0 Hz, 1H), 7.20-7.25 (m, 1H), 7.32 (dd, *J* = 4.9, 3.0 Hz, 1H), 8.23-8.40 (br, 1H); ¹³C NMR (75 MHz, CDCl₃) δ 33.2, 75.4, 94.5, 123.9, 126.4, 128.4, 131.3, 154.6, 169.9; HRMS calcd for [C₉H₈Cl₃NO₄SNa]⁺ [(M + Na)⁺]: 353.9132; found: 353.9140.

### Ethyl 3-(2-oxo-2-((((2,2,2-trichloroethoxy)carbonyl)amino)oxy)ethyl)-1H-indole-1-carboxylate

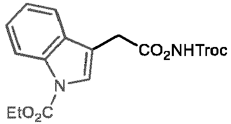

EDCI coupling of 2-(1-(ethoxycarbonyl)-1*H*-indol-3-yl)acetic acid with TrocNHOH gave ethyl 3-(2-oxo-2-((((2,2,2-trichloroethoxy)carbonyl)amino)oxy)ethyl)-1*H*-indole-1-carboxylate as a yellow oil (chromatography on silica gel, eluent: EtOAc/n-hexane = 1/6): IR (film) vₘₐₓ: 3261, 1731, 1250, 1084, 748 cm⁻¹; ¹H NMR (300 MHz, CDCl₃) δ 1.46 (t, *J* = 7.1 Hz, 3H), 3.90 (s, 2H), 4.48 (q, *J* = 7.1 Hz, 2H), 4.79 (s, 2H), 7.22-7.32 (m, 1H), 7.32-7.42 (m, 1H), 7.47-7.58 (m, 1H), 7.67 (s, 1H), 8.06-8.24 (m, 1H), 8.32-8.51 (br, 1H); ¹³C NMR (75 MHz, CDCl₃) δ 14.5, 28.6, 63.5, 75.3, 94.5, 111.8, 115.5, 119.0, 123.2, 124.7, 125.2, 129.7, 135.5, 150.9, 154.6, 169.8; HRMS calcd for [C₁₆H₁₅Cl₃N₂O₆Na]⁺ [(M + Na)⁺]: 458.9888; found: 458.9889.

### 2,2,2-Trichloroethyl (2-(11-oxo-6,11-dihydrodibenzo[b,e]oxepin-2-yl)acetoxy)carbamate

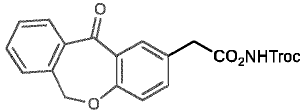

EDCI coupling of Isoxepac with TrocNHOH gave 2,2,2-trichloroethyl (2-(11-oxo-6,11-dihydrodibenzo[*b*,*e*]oxepin-2-yl)acetoxy)carbamate as a colorless oil (chromatography on silica gel, eluent: EtOAc/*n*-hexane = 1/3): IR (film) vₘₐₓ: 3248, 1758, 1487, 1298, 1090, 757, 710 cm⁻¹; ¹H NMR (300 MHz, CDCl₃) δ 3.81 (s, 2H), 4.78 (s, 2H), 5.17 (s, 2H), 7.03 (d, *J* = 8.5 Hz, 1H), 7.28-7.39 (m, 1H), 7.39-7.51 (m, 2H), 7.51-7.64 (m, 1H), 7.87 (dd, *J* = 7.8, 1.1 Hz, 1H), 8.13 (d, *J* = 2.3 Hz, 1H), 8.43-8.69 (br, 1H); ¹³C NMR (75 MHz, CDCl₃) δ 37.5, 73.7, 75.3, 94.5, 121.6, 125.4, 125.8, 128.0, 129.4, 129.6, 132.8, 133.0, 135.6, 136.3, 140.4, 154.6, 161.0, 170.2, 190.8; HRMS calcd for [C₁₉H₁₄Cl₃NO₆Na]⁺ [(M + Na)⁺]: 479.9779; found: 479.9770.

### 2,2,2-Trichloroethyl (2-(2-((2,6-dichlorophenyl)amino)phenyl)acetoxy)carbamate

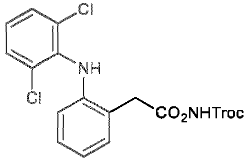

DCC coupling of Diclofenac with TrocNHOH gave 2,2,2-trichloroethyl (2-(2-((2,6-dichlorophenyl)amino)phenyl)acetoxy)carbamate as a colorless oil (chromatography on silica gel, eluent: EtOAc/*n*-hexane = 1/10): IR (film) vₘₐₓ: 3344, 1752, 1449, 1111, 744, 715 cm⁻¹ ; ¹H NMR (300 MHz, CDCl₃) δ 4.00 (s, 2H), 4.79 (s, 2H), 6.43-6.54 (br, 1H), 6.54-6.64 (m, 1H), 6.94-7.06 (m, 2H), 7.11-7.21 (m, 1H), 7.23-7.31 (m, 1H), 7.34 (d, *J* = 8.1 Hz, 2H), 8.27-8.45 (br, 1H); ¹³C NMR (75 MHz, CDCl₃) δ 35.8, 75.3, 94.4, 119.3, 122.8, 123.0, 124.4, 128.8, 129.0, 129.6, 131.2, 137.9, 142.9, 154.5, 171.0; HRMS calcd for [C₁₇H₁₃Cl₅N₂O₄Na]⁺ [(M + Na)⁺]: 506.9210; found: 506.9216.

### 2,2,2-Trichloroethyl (but-3-enoyloxy)carbamate

DCC coupling of but-3-enoic acid with TrocNHOH gave 2,2,2-trichloroethyl (but-3- 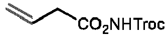 enoyloxy)carbamate as a colorless oil (chromatography on silica gel, eluent: EtOAc/n-hexane = 1/10): IR (film) vₘₐₓ: 3278, 1748, 1223, 1116, 715 cm⁻¹ ; ¹H NMR (300 MHz, CDCl₃) δ 3.27 (dt, *J* = 6.8, 1.4 Hz, 2H), 4.80 (s, 2H), 5.21-5.33 (m, 2H), 5.90 (ddt, *J* = 17.2, 10.0, 6.8 Hz, 1H), 8.18-8.64 (br, 1H); ¹³C NMR (75 MHz, CDCl₃) δ 36.4, 75.3, 94.5, 120.4, 128.0, 154.6, 170.3; HRMS calcd for [C₇H₈Cl₃NO₄Na]⁺ [(M + Na)⁺]: 297.9411; found: 297.9423.

### 2,2,2-Trichloroethyl (E)-(hex-3-enoyloxy)carbamate

DCC coupling of (*E*)-hex-3-enoic acid with TrocNHOH gave 2,2,2-trichloroethyl (*E*)-(hex-3- 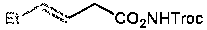 enoyloxy)carbamate as a colorless oil (chromatography on silica gel, eluent: EtOAc/n-hexane = 1/10): IR (film) vₘₐₓ: 3277, 2965, 1748, 1115, 717 cm⁻¹; ¹H NMR (300 MHz, CDCl₃) δ 1.00 (t, *J* = 7.5 Hz, 3H), 1.99-2.15 (m, 2H), 3.20 (dq, *J* = 6.9, 1.2 Hz, 2H), 4.80 (s, 2H), 5.49 (dtt, *J* = 15.4, 6.9, 1.5 Hz, 1H), 5.71 (dtt, *J* = 15.4, 6.3, 1.2 Hz, 1H), 8.25-8.40 (br, 1H); ¹³C NMR (75 MHz, CDCl₃) δ 13.4, 25.6, 35.4, 75.3, 94.5, 118.4, 138.4, 154.6, 170.9; HRMS calcd for [C₉H₁₂Cl₃NO₄Na]⁺ [(M +Na)⁺]: 325.9724; found: 325.9729.

### 2,2,2-Trichloroethyl (E)-((5-methylhex-3-enoyl)oxy)carbamate

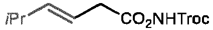

DCC coupling of (*E*)-5-methylhex-3-enoic acid with TrocNHOH gave 2,2,2-trichloroethyl (*E*)-((5-methylhex-3-enoyl)oxy)carbamate as a colorless oil (chromatography on silica gel, eluent: EtOAc/n-hexane = 1/10): IR (film) vₘₐₓ: 3357, 2964, 1806, 1761, 1463, 1223, 1117, 1070, 827, 727 cm⁻¹; ¹H NMR (300 MHz, CDCl₃) δ 0.99 (d, *J* = 6.8 Hz, 6H), 2.22-2.39 (m, 1H), 3.19 (dt, *J* = 6.7, 1.1 Hz, 2H), 4.80 (s, 2H), 5.45 (dtd, *J* = 15.5, 6.7, 1.2 Hz, 1H), 5.63 (ddt, *J* = 15.5, 6.5, 1.1 Hz, 1H), 8.28-8.44 (br, 1H); ¹³C NMR (75 MHz, CDCl₃) δ 22.2, 31.2, 35.4, 75.3, 94.5, 116.6, 143.6, 154.6, 170.9; HRMS calcd for [C₁₀H₁₄Cl₃NO₄Na]⁺ [(M + Na)⁺]: 339.9881; found: 339.9884.

### 2,2,2-Trichloroethyl (E)-(dec-3-enoyloxy)carbamate

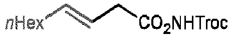

DCC coupling of (*E*)-dec-3-enoic acid with TrocNHOH gave 2,2,2-trichloroethyl (*E*)-(dec-3-enoyloxy)carbamate as a colorless oil (chromatography on silica gel, eluent: EtOAc/n-hexane = 1/15): IR (film) vₘₐₓ: 3279, 2926, 1752, 1098, 718 cm⁻¹; ¹H NMR (300 MHz, CDCl₃) δ 0.82-0.94 (m, 3H), 1.19-1.43 (m, 8H), 1.96-2.11 (m, 2H), 3.20 (dq, *J* = 6.7, 1.1 Hz, 2H), 4.80 (s, 2H), 5.49 (dtt, *J* = 15.3, 6.7, 1.2 Hz, 1H), 5.66 (dtt, *J* = 15.3, 6.6, 1.1 Hz, 1H), 8.26-8.40 (br, 1H); ¹³C NMR (75 MHz, CDCl₃) δ 14.2, 22.7, 28.9, 29.1, 31.8, 32.6, 35.4, 75.3, 94.5, 119.2, 137.0, 154.6, 170.9; HRMS calcd for [C₁₃H₂₀Cl₃NO₄Na]⁺ [(M + Na)⁺]: 382.0350; found: 382.0354.

### 2,2,2-Trichloroethyl (E)-((4-phenylbut-3-enoyl)oxy)carbamate

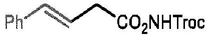

DCC coupling of (*E*)-4-phenylbut-3-enoic acid with TrocNHOH gave 2,2,2-trichloroethyl (*E*)-((4-phenylbut-3-enoyl)oxy)carbamate as a light yellow oil (chromatography on silica gel, eluent: EtOAc/*n*-hexane = 1/10): IR (film) vₘₐₓ: 3187, 1799, 1721, 1514, 1263, 1138, 1079, 719 cm⁻¹ ; ¹H NMR (300 MHz, CDCl₃) δ 3.43 (dd, *J* = 7.0, 1.3 Hz, 2H), 4.81 (s, 2H), 6.25 (dt, *J* = 15.9, 7.0 Hz, 1H), 6.58 (dt, *J* = 15.9, 1.3 Hz, 1H), 7.21-7.44 (m, 5H), 8.31-8.45 (br, 1H); ¹³C NMR (75 MHz, CDCl₃) δ 35.7, 75.3, 94.5, 119.2, 126.6, 128.1, 128.8, 135.2, 136.4, 154.6, 170.4; HRMS calcd for [C₁₃H₁₂Cl₃NO₄Na]⁺ [(M + Na)⁺]: 373.9724; found: 373.9731.

### 2,2,2-Trichloroethyl (E)-(hexa-3,5-dienoyloxy)carbamate

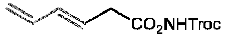

DCC coupling of (*E*)-hexa-3,5-dienoic acid with TrocNHOH gave 2,2,2-trichloroethyl (*E*)-(hexa-3,5-dienoyloxy)carbamate as a colorless oil (chromatography on silica gel, eluent: EtOAc/n-hexane = 1/15): IR (film) vₘₐₓ: 3246, 1751, 1225, 1112, 715 cm⁻¹ ; ¹H NMR (300 MHz, CDCl₃) δ 3.30 (dd, *J* = 7.2, 1.0 Hz, 2H), 4.80 (s, 2H), 5.12 (dd, *J* = 10.0, 1.6 Hz, 1H), 5.21 (dd, *J* = 16.2, 1.6 Hz, 1H), 5.74 (dt, *J* = 15.0, 7.2 Hz, 1H), 6.22 (ddt, *J* = 15.0, 10.5, 1.0 Hz, 1H), 6.33 (dt, *J* = 16.2, 10.3 Hz, 1H), 8.28-8.46 (br, 1H); ¹³C NMR (75 MHz, CDCl₃) δ 35.3, 75.3,94.5, 118.2, 122.9, 135.97, 136.01, 154.6, 170.3; HRMS calcd for [C₉H₁₀Cl₃NO₄Na]⁺ [(M+Na)⁺]: 323.9568; found: 323.9568.

### 2,2,2-Trichloroethyl ((4-phenylbut-3-ynoyl)oxy)carbamate

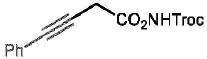

DCC coupling of 4-phenylbut-3-ynoic acid with TrocNHOH gave 2,2,2-trichloroethyl ((4-phenylbut-3-ynoyl)oxy)carbamate as a colorless oil (chromatography on silica gel, eluent: EtOAc/n-hexane = 1/10): IR (film) vₘₐₓ: 3204, 1758, 1132, 719 cm⁻¹; ¹H NMR (300 MHz, CDCl₃) δ 3.71 (s, 2H), 4.82 (s, 2H), 7.27-7.36 (m, 3H), 7.39-7.51 (m, 2H), 8.32-8.52 (br, 1H); ¹³C NMR (75 MHz, CDCl₃) δ 24.6, 75.4, 78.6, 84.6, 94.4, 122.5, 128.4, 128.7, 132.0, 154.5, 167.3; HRMS calcd for [C₁₃H₁₀C1lNO₄Na]⁺ [(M + Na)⁺]: 371.9568; found: 371.9575.

### 2,2,2-Trichloroethyl (hex-3-ynoyloxy)carbamate

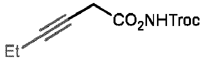

DCC coupling of hex-3-ynoic acid with TrocNHOH gave 2,2,2-trichloroethyl (hex-3-ynoyloxy)carbamate as a white solid (chromatography on silica gel, eluent: EtOAc/*n*-hexane = 1/12): IR (film) vₘₐₓ: 3206, 1762, 1460, 1204, 1125, 720 cm⁻¹ ; ¹H NMR (300 MHz, CDCl₃) δ 1.13 (t, *J* = 7.5 Hz, 3H), 2.20 (qt, *J* = 7.5, 2.4 Hz, 2H), 3.44 (t, *J* = 2.4 Hz, 2H), 4.80 (s, 2H), 8.30-8.45 (br, 1H); ¹³C NMR (75 MHz, CDCl₃) δ 12.5, 13.8, 23.9, 68.5, 75.4, 86.5, 94.4, 154.5, 168.1; HRMS calcd for [C₉H₁₀Cl₃NO₄Na]⁺ [(M + Na)⁺]: 323.9568; found: 323.9574.

### 2,2,2-Trichloroethyl ((3,3-dimethylbutanoyl)oxy)carbamate

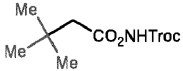

DCC coupling of 3,3-dimethylbutanoic acid with TrocNHOH gave 2,2,2-trichloroethyl ((3,3-dimethylbutanoyl)oxy)carbamate as a white solid (chromatography on silica gel, eluent: EtOAc/*n*-hexane = 1/20): IR (film) vₘₐₓ: 3286, 2961, 1791, 1741, 1473, 1253, 1123, 731 cm⁻¹; ¹H NMR (300 MHz, CDCl₃) δ 1.08 (s, 9H), 2.37 (s, 2H), 4.80 (s, 2H), 8.24-8.48 (br, 1H); ¹³C NMR (75 MHz, CDCl₃) δ 29.6, 31.3, 45.2, 75.3, 94.6, 154.7, 170.9; HRMS calcd for [C₉H₁₄Cl₃NO₄Na]⁺ [(M + Na)⁺]: 327.9881; found: 327.9889.

### 2,2,2-Trichloroethyl ((2-phenylpropanoyl)oxy)carbamate [(±)-37]

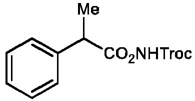

DCC coupling of 2-phenylpropanoic acid with TrocNHOH gave (±)**-37** as a colorless oil (chromatography on silica gel, eluent: EtOAc/*n*-hexane = 1/10): IR (film) vₘₐₓ: 3261, 1739, 1315, 1093, 1001, 711 cm⁻¹; ¹H NMR (300 MHz, CDCl₃) δ 1.61 (d, *J* = 7.2 Hz, 3H), 3.92 (q, *J* = 7.2 Hz, 1H), 4.75 (d, *J* = 11.9 Hz, 1H), 4.80 (d, *J* = 11.9 Hz, 1H), 7.23-7.41 (m, 5H), 8.12-8.31 (br, 1H); ¹³C NMR (75 MHz, CDCl₃) δ 18.6, 43.4, 75.3, 94.5, 127.7, 127.9, 129.0, 138.5, 154.6, 173.4; HRMS calcd for [C₁₂H₁₂Cl₃NO₄Na]⁺ [(M + Na)⁺]: 361.9724; found: 361.9734.

### 2,2,2-Trichloroethyl (R)-((2-phenylpropanoyl)oxy)carbamate [(R)-37]

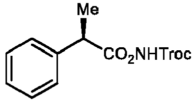

DCC coupling of (R)-2-phenylpropanoic acid (99% e.e.) with TrocNHOH gave (R)-37 as a colorless oil (chromatography on silica gel, eluent: EtOAc/n-hexane = 1/10): [α]_{D}²⁵ = -75.3 (c 1.0, CH₂Cl₂); The ¹H NMR and ¹³C NMR data of (*R*)-**37** are identical with those of (±)**-37**.

### 2,2,2-Trichloroethyl (S)-((2-phenylpropanoyl)oxy)carbamate [(S)-37]

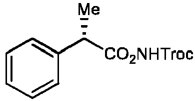

DCC coupling of (*S*)-2-phenylpropanoic acid (99% e.e.) with TrocNHOH gave (*S*)-**37** as a colorless oil (chromatography on silica gel, eluent: EtOAc/n-hexane = 1/10): [α]_{D}²⁵ = +63.0 (c 1.0, CH₂Cl₂); The ¹H NMR and ¹³C NMR data of (*S*)-**37** are identical with those of (±)-**37**.

### 2,2,2-Trichloroethyl (S)-((2-(6-methoxynaphthalen-2-yl)propanoyl)oxy)carbamate

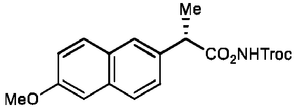

DCC coupling of (*S*)-Naproxen (99% e.e.) with TrocNHOH gave 2,2,2-trichloroethyl (*S*)-((2-(6-methoxynaphthalen-2-yl)propanoyl)oxy)carbamate as a white solid (chromatography on silica gel, eluent: EtOAc/n-hexane = 1/8): [α]_{D}²⁵ = +66.7 (c 1.0, CH₂Cl₂); IR (film) vₘₐₓ: 3250, 1760, 1224, 1108, 1064, 712 cm⁻¹; ¹H NMR (300 MHz, CDCl₃) δ 1.68 (d, *J* = 7.2 Hz, 3H), 3.91 (s, 3H), 4.05 (q, *J* = 7.2 Hz, 1H), 4.74 (d, *J* = 11.9 Hz, 1H), 4.79 (d, *J* = 11.9 Hz, 1H), 7.04-7.21 (m, 2H), 7.42 (dd, *J* = 8.4, 1.9 Hz, 1H), 7.61-7.78 (m, 3H), 8.21-8.35 (br, 1H); ¹³C NMR (75 MHz, CDCl₃) δ 18.6, 43.3, 55.4, 75.3, 94.5, 105.8, 119.4, 126.0, 126.4, 127.6, 129.0, 129.5, 133.6, 134.1, 154.6, 158.0, 173.5; HRMS calcd for [C₁₇H₁₆Cl₃NO₅Na]⁺ [(M + Na)⁺]: 441.9986; found: 441.9991.

### 2,2,2-Trichloroethyl (S,E)-((2-methylhex-3-enoyl)oxy)carbamate

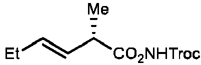

DCC coupling of (*S*,*E*)-2-methylhex-3-enoic acid (>99% e.e.) with TrocNHOH gave 2,2,2-trichloroethyl (*S,E*)-((2-methylhex-3-enoyl)oxy)carbamate as a colorless oil (chromatography on silica gel, eluent: EtOAc/n-hexane = 1/20): [α]_{D}²⁵ = +49.1 (c 1.0, CH₂Cl₂); IR (film) vₘₐₓ: 3275, 2968, 1756, 1114, 716 cm⁻¹ ; ¹H NMR (300 MHz, CDCl₃) δ 0.98 (t, *J* = 7.5 Hz, 3H), 1.33 (d, *J=* 7.1 Hz, 3H), 1.95-2.15 (m, 2H), 3.21-3.38 (m, 1H), 4.79 (s, 2H), 5.48 (ddt, *J* = 15.4, 7.7, 1.4 Hz, 1H), 5.68 (dtd, *J* = 15.4, 6.2, 0.6 Hz, 1H), 8.23-8.40 (br, 1H); ¹³C NMR (75 MHz, CDCl₃) δ 13.4, 17.5, 25.5, 40.8, 75.3, 94.6, 125.9, 135.7, 154.7, 173.9; HRMS calcd for [C₁₀H₁₄Cl₃NO₄Na]⁺ [(M + Na)⁺]: 339.9881; found: 339.9884.

### 2,2,2-Trichloroethyl (S)-((2-methylbutanoyl)oxy)carbamate

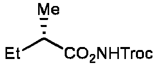

DCC coupling of (*S*)-2-methylbutanoic acid (99% e.e.) with TrocNHOH gave 2,2,2-trichloroethyl (S)-((2-methylbutanoyl)oxy)carbamate as a colorless oil (chromatography on silica gel, eluent: EtOAc/n-hexane = 1/12): [α]_{D}²⁵ = -6.2 (c 1.0, CH₂Cl₂); IR (film) vₘₐₓ: 3281, 2972, 1750, 1115, 1083, 716 cm⁻¹ ; ¹H NMR (300 MHz, CDCl₃) δ 0.96 (t, *J* = 7.4 Hz, 3H), 1.24 (d, *J* = 7.0 Hz, 3H), 1.48-1.66 (m, 1H), 1.68-1.86 (m, 1H), 2.51-2.66 (m, 1H), 4.80 (s, 2H), 8.20-8.46 (br, 1H); ¹³C NMR (75 MHz, CDCl₃) δ 11.6, 16.5, 26.7, 39.1, 75.3, 94.6, 154.8, 175.5; HRMS calcd for [C₈H₁₂Cl₃NO₄Na]⁺ [(M + Na)⁺]: 313.9724; found: 313.9729.

### 2,2,2-Trichloroethyl (S)-((2-cyclohexylpropanoyl)oxy)carbamate

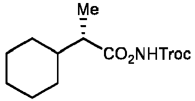

DCC coupling of (*S*)-2-cyclohexylpropanoic acid (99% e.e.) with TrocNHOH gave 2,2,2-trichloroethyl (*S*)-((2-cyclohexylpropanoyl)oxy)carbamate as a colorless oil (chromatography on silica gel, eluent: EtOAc/*n*-hexane = 1/20): [α]_{D}²⁵ = +10.3 (c 1.0, CH₂Cl₂); IR (film) vₘₐₓ: 3283, 2927, 2854, 1751, 1114, 1053, 717 cm⁻¹ ; ¹H NMR (300 MHz, CDCl₃) δ 0.92-1.34 (m, 8H), 1.54-1.84 (m, 6H), 2.46 (quintet, *J* = 7.1 Hz, 1H), 4.80 (s, 2H), 8.28-8.39 (br, 1H); ¹³C NMR (75 MHz, CDCl₃) δ 14.0, 26.2, 26.29, 26.30, 29.7, 31.1, 40.7, 43.4, 75.3, 94.6, 154.7, 175.2; HRMS calcd for [C₁₂H₁₈Cl₃NO₄Na]⁺ [(M + Na)⁺]: 368.0194; found: 368.0195.

### 2,2,2-Trichloroethyl (butyryloxy)carbamate

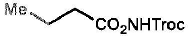

DCC coupling of butyric acid with TrocNHOH gave 2,2,2-trichloroethyl (butyryloxy)carbamate as a colorless oil (chromatography on silica gel, eluent: EtOAc/n-hexane = 1/12): IR (film) vₘₐₓ: 3278, 2967, 1749, 1228, 1119, 719 cm⁻¹ ; ¹H NMR (300 MHz, CDCl₃) δ 1.00 (t, *J* = 7.4 Hz, 3H), 1.74 (sextet, *J* = 7.4 Hz, 2H), 2.47 (t, *J* = 7.4 Hz, 2H), 4.80 (s, 2H), 8.26-8.50 (br, 1H); ¹³C NMR (75 MHz, CDCl₃) δ 13.6, 18.3, 33.5, 75.3, 94.6, 154.7, 172.4; HRMS calcd for [C₇H₁₀Cl₃NO₄Na]⁺ [(M + Na)⁺]: 299.9568; found: 299.9573.

### 2,2,2-Trichloroethyl (octanoyloxy)carbamate

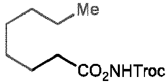

DCC coupling of octanoic acid with TrocNHOH gave 2,2,2-trichloroethyl (octanoyloxy)carbamate as a colorless oil (chromatography on silica gel, eluent: EtOAc/*n*-hexane = 1/15): IR (film) vₘₐₓ: 3281, 2927, 1751, 1231, 1120, 1089, 718 cm⁻¹; ¹H NMR (300 MHz, CDCl₃) δ 0.75-0.96 (m, 3H), 1.14-1.42 (m, 8H), 1.61-1.80 (m, 2H), 2.48 (t, *J* = 7.6 Hz, 2H), 4.80 (s, 2H), 8.18-8.47 (br, 1H); ¹³C NMR (75 MHz, CDCl₃) δ 14.2, 22.7, 24.7, 28.9, 29.0, 31.68, 31.71, 75.3, 94.6, 154.7, 172.6; HRMS calcd for [C₁₁H₁₈Cl₃NO₄Na]⁺ [(M + Na)⁺]: 356.0194; found: 356.0194.

### 2,2,2-Trichloroethyl ((5-phenylpentanoyl)oxy)carbamate

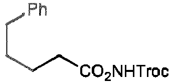

DCC coupling of 5-phenylpentanoic acid with TrocNHOH gave 2,2,2-trichloroethyl ((5-phenylpentanoyl)oxy)carbamate as a colorless oil (chromatography on silica gel, eluent: EtOAc/*n*-hexane = 1/10): IR (film) vₘₐₓ: 3282, 2936, 1753, 1112, 746, 721, 708 cm⁻¹; ¹H NMR (300 MHz, CDCl₃) δ 1.61-1.86 (m, 4H), 2.41-2.58 (m, 2H), 2.58-2.75 (m, 2H), 4.80 (s, 2H), 7.09-7.24 (m, 3H), 7.24-7.36 (m, 2H), 8.28-8.46 (br, 1H); ¹³C NMR (75 MHz, CDCl₃) δ 24.3, 30.7, 31.5, 35.5, 75.2, 94.6, 126.0, 128.49, 128.51, 141.8, 154.7, 172.3; HRMS calcd for [C₁₄H₁₆Cl₃NO₄Na]⁺ [(M + Na)⁺]: 390.0037; found: 390.0042.

### Methyl 6-oxo-6-((((2,2,2-trichloroethoxy)carbonyl)amino)oxy)hexanoate

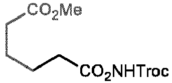

DCC coupling of 6-methoxy-6-oxohexanoic acid with TrocNHOH gave methyl 6-oxo-6-((((2,2,2-trichloroethoxy)carbonyl)amino)oxy)hexanoate as a colorless oil (chromatography on silica gel, eluent: EtOAc/n-hexane = 1/5): IR (film) vₘₐₓ: 3258, 2954, 1730, 1227, 1110, 718 cm⁻¹; ¹H NMR (300 MHz, CDCl₃) δ 1.65-1.80 (m, 4H), 2.28-2.40 (m, 2H), 2.42-2.59 (m, 2H), 3.66 (s, 3H), 4.79 (s, 2H), 8.43-8.60 (br, 1H); ¹³C NMR (75 MHz, CDCl₃) δ 24.1, 24.2, 31.3, 33.6, 51.8, 75.2, 94.6, 154.7, 172.0, 173.7; HRMS calcd for [C₁₀H₁₄Cl₃NO₆Na]⁺ [(M + Na)⁺]: 371.9779; found: 371.9780.

### 2,2,2-Trichloroethyl ((4-phenoxybutanoyl)oxy)carbamate

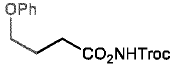

DCC coupling of 4-phenoxybutanoic acid with TrocNHOH gave 2,2,2-trichloroethyl ((4-phenoxybutanoyl)oxy)carbamate as a colorless oil (chromatography on silica gel, eluent: EtOAc/n-hexane = 1/5): IR (film) vₘₐₓ: 3285, 2956, 1754, 1237, 1115, 1047, 750, 718 cm⁻¹ ; ¹H NMR (300 MHz, CDCl₃) δ 2.20 (tt, *J* = 7.3, 6.0 Hz, 2H), 2.73 (t, *J* = 7.3 Hz, 2H), 4.04 (t, *J* = 6.0 Hz, 2H), 4.80 (s, 2H), 6.82-6.92 (m, 2H), 6.92-7.01 (m, 1H), 7.18-7.36 (m, 2H), 8.19-8.55 (br, 1H); ¹³C NMR (75 MHz, CDCl₃) δ 24.5, 28.5, 66.1, 75.3, 94.5, 114.6, 121.1, 129.6, 154.7, 158.7, 172.1; HRMS calcd for [C₁₃H₁₄Cl₃NO₃Na]⁺ [(M + Na)⁺]: 391.9830; found: 391.9835.

### 2,2,2-Trichloroethyl ((4-((tert-butoxycarbonyl)amino)butanoyl)oxy)carbamate

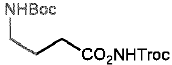

EDCI coupling of 4-((*tert*-butoxycarbonyl)amino)butanoic acid with TrocNHOH gave 2,2,2-trichloroethyl ((4-((*tert*-butoxycarbonyl)amino)butanoyl)oxy)carbamate as a white solid (chromatography on silica gel, eluent: EtOAc/n-hexane = 1/2): IR (film) vₘₐₓ: 3384, 3120, 2940, 1747, 1679, 1528, 1258, 1155, 1121, 719 cm⁻¹ ; ¹H NMR (300 MHz, CDCl₃) δ 1.43 (s, 9H), 1.81-1.96 (m, 2H), 2.52 (t, *J* = 7.3 Hz, 2H), 3.19 (q, *J* = 6.5 Hz, 2H), 4.74 (t, *J* = 6.5 Hz, 1H), 4.79 (s, 2H), 8.67-8.89 (br, 1H); ¹³C NMR (75 MHz, CDCl₃) δ 25.2, 28.5, 29.0, 39.6, 75.2, 79.7, 94.6, 154.8, 156.2, 172.1; HRMS calcd for [C₁₂H₁₉Cl₃N₂O₆Na]⁺ [(M + Na)⁺]: 415.0201; found: 415.0202.

### 2,2,2-Trichloroethyl ((3-cyclohexylpropanoyl)oxy)carbamate

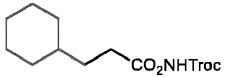

DCC coupling of 3-cyclohexylpropanoic acid with TrocNHOH gave 2,2,2-trichloroethyl ((3-cyclohexylpropanoyl)oxy)carbamate as a colorless oil (chromatography on silica gel, eluent: EtOAc/*n*-hexane = 1/15): IR (film) vₘₐₓ: 3277, 2924, 2852, 1755, 1450, 1233, 1115, 720 cm⁻¹ ; ¹H NMR (300 MHz, CDCl₃) δ 0.80-1.00 (m, 2H), 1.06-1.39 (m, 4H), 1.55-1.66 (m, 3H), 1.66-1.77 (m, 4H), 2.40-2.56 (m, 2H), 4.80 (s, 2H), 8.22-8.45 (br, 1H); ¹³C NMR (75 MHz, CDCl₃) δ 26.2, 26.6, 29.3, 32.0, 33.0, 37.2, 75.3, 94.6, 154.7, 172.9; HRMS calcd for [C₁₂H₁₈Cl₃NO₄Na]⁺ [(M + Na)⁺]: 368.0194; found: 368.0195.

### 2,2,2-Trichloroethyl ((3-cyclopentylpropanoyl)oxy)carbamate

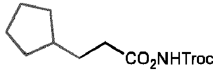

DCC coupling of 3-cyclopentylpropanoic acid with TrocNHOH gave 2,2,2-trichloroethyl ((3-cyclopentylpropanoyl)oxy)carbamate as a colorless oil (chromatography on silica gel, eluent: EtOAc/*n*-hexane = 1/15): IR (film) vₘₐₓ: 3278, 2949, 2866, 1754, 1227, 1117, 720 cm⁻¹; ¹H NMR (300 MHz, CDCl₃) δ 0.96-1.19 (m, 2H), 1.43-1.65 (m, 4H), 1.65-1.87 (m, 5H), 2.38-2.58 (m, 2H), 4.80 (s, 2H), 8.29-8.46 (br, 1H); ¹³C NMR (75 MHz, CDCl₃) δ 25.2, 30.9, 31.0, 32.4, 39.6, 75.3, 94.6, 154.7, 172.6; HRMS calcd for [C₁₁H₁₆Cl₃NO₄Na]⁺ [(M + Na)⁺]: 354.0037; found: 354.0039.

### 2,2,2-Trichloroethyl (2-cyclohexylacetoxy)carbamate

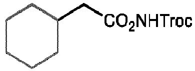

DCC coupling of 2-cyclohexylacetic acid with TrocNHOH gave 2,2,2-trichloroethyl (2-cyclohexylacetoxy)carbamate as a colorless oil (chromatography on silica gel, eluent: EtOAc/n-hexane = 1/15): IR (film) vₘₐₓ: 3275, 2926, 2852, 1749, 1214, 1113, 717 cm⁻¹ ; ¹H NMR (300 MHz, CDCl₃) δ 0.92-1.10 (m, 2H), 1.10-1.36 (m, 3H), 1.61-1.93 (m, 6H), 2.36 (d, *J=* 7.0 Hz, 2H), 4.80 (s, 2H), 8.24-8.42 (br, 1H); ¹³C NMR (75 MHz, CDCl₃) δ 26.0, 26.1, 33.0, 34.9, 39.3, 75.3, 94.6, 154.7, 171.8; HRMS calcd for [C₁₁H₁₆Cl₃NO₄Na]⁺ [(M + Na)⁺]: 354.0037; found: 354.0040.

### (((R)-4-((3R,5R,8R,9S,10S,13R,14S,17R)-3-Hydroxy-10,13-dimethylhexadecahydro-1H-cyclopenta[a]phenanthren-17-yl)pentanoyl)oxy)carbamate

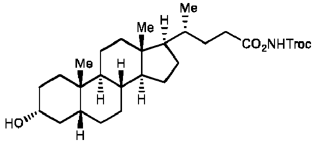

EDCI coupling of lithocholic acid with TrocNHOH gave (((*R*)-4-((3*R*,5*R*,8*R*,9*S*,10*S*,13*R*,14*S*,17*R*)-3-hydroxy-10,13-dimethylhexadecahydro-1*H*-cyclopenta[*a*]phenanthren-17-yl)pentanoyl)oxy)carbamate as a white solid (chromatography on silica gel, eluent: EtOAc/*n*-hexane = 1/2): IR (film) vₘₐₓ: 3484, 3167, 2928, 2858, 1744, 1116, 1066, 1029, 764 cm⁻¹; ¹H NMR (300 MHz, CDCl₃) δ 0.64 (s, 3H), 0.85-0.95 (m, 6H), 1.00-1.99 (m, 27H), 2.32-2.45 (m, 1H), 2.45-2.60 (m, 1H), 3.54-3.71 (m, 1H), 4.79 (s, 2H), 8.48-8.68 (br, 1H); ¹³C NMR (75 MHz, CDCl₃) δ 12.1, 18.3, 21.0, 23.5, 24.3, 26.5, 27.3, 28.3, 28.7, 30.6, 30.8, 34.7, 35.4, 35.5, 36.0, 36.5, 40.3, 40.6, 42.2, 42.9, 56.0, 56.6, 72.0, 75.2, 94.6, 154.7, 173.0; HRMS calcd for [C₂₇H₄₂Cl₃NO₅Na]⁺ [(M + Na)⁺]: 588.2021; found: 588.2026.

### 2,2,2-Trichloroethyl ((2-(p-tolyl)propanoyl)oxy)carbamate

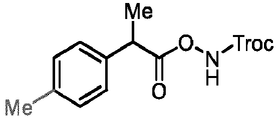

DCC coupling of 2-(p-tolyl)propanoic acid with TrocNHOH gave the product as a white solid (chromatography on silica gel, eluent: EtOAc/n-hexane = 1/10): **¹H NMR** (300 MHz, CDCl₃) δ 8.21 (s, 1H), 7.25 - 7.12 (m, 4H), 4.81 (d, *J* = 12.0 Hz, 1H), 4.79 (d, *J* = 12.0 Hz, 1H), 3.89 (q, *J* = 7.2 Hz, 1H), 2.34 (s, 3H), 1.59 (d, *J* = 7.2 Hz, 3H). **¹³C NMR** (75 MHz, CDCl₃) δ 173.56, 154.62, 137.66, 135.57, 129.69, 127.56, 94.51, 75.28, 43.00, 21.18, 18.56. **IR (film):** v (cm⁻¹) 3239, 1790, 1729, 1480, 1243, 1102, 759, 564 cm⁻¹. **HRMS (ESI, *m*/*z)*** calcd for [C₁₃H₁₄Cl₃NO₄Na] ⁺ [(M + Na) ⁺]: 375.9881, found: 375.9881.

### 2,2,2-Trichloroethyl ((2-(4-(tert-butyl)phenyl)propanoyl)oxy)carbamate

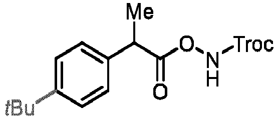

DCC coupling of 2-(4-(*tert*-butyl)phenyl)propanoic acid with TrocNHOH gave the product as a colorless oil (chromatography on silica gel, eluent: EtOAc/n-hexane = 1/10): **¹H NMR** (300 MHz, CDCl₃) δ 8.23 (s, 1H), 7.41 - 7.22 (m, 4H), 4.80 (d, *J* = 12.0 Hz, 1H), 4.75 (d, *J* = 12.0 Hz, 1H), 3.90 (q, *J* = 7.2 Hz, 1H), 1.60 (d, *J* = 7.2 Hz, 3H), 1.31 (s, 9H). **¹³C NMR** (75 MHz, CDCl₃) δ 173.56, 154.64, 150.84, 135.46, 127.35, 125.93, 94.50, 75.28, 42.90, 34.64, 31.44, 18.52. **IR (film):** v (cm⁻¹) 3296, 2962, 1750, 1454, 1321, 1226, 1110, 716, 565 cm⁻¹. **HRMS (ESI, *m*/*z)*** calcd for [C₁₆H₂₀Cl₃NO₄Na] ⁺ [(M + Na) ⁺]: 418.0350, found: 418.0350.

### 2,2,2-Trichloroethyl ((2-(4-isobutylphenyl)propanoyl)oxy)carbamate

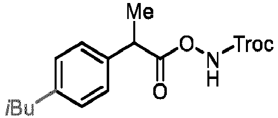

DCC coupling of 2-(4-isobutylphenyl)propanoic acid with TrocNHOH gave the product as a white solid (chromatography on silica gel, eluent: EtOAc/n-hexane = 1/10): **¹H NMR** (300 MHz, CDCl₃) δ 8.24 (s, 1H), 7.34 - 7.26 (m, 2H), 7.21 - 7.12 (m, 2H), 4.85 (d, *J* = 12.0 Hz, 1H), 4.80 (d, *J* = 12.0 Hz, 1H), 3.94 (q, *J* = 7.2 Hz, 1H), 2.50 (d, *J* = 7.2 Hz, 2H), 1.83 - 1.96 (m, 1H), 1.64 (d, *J* = 7.2 Hz, 3H), 0.95 (d, *J* = 6.6 Hz, 6H). **¹³C NMR** (75 MHz, CDCl₃) δ 173.46, 154.48, 141.32, 135.62, 129.59, 127.26, 94.38, 75.16, 45.03, 42.89, 30.15, 22.38, 18.42. **IR (film):** v (cm⁻¹) 3258, 2952, 1741, 1384, 1098, 892, 810, 563, 464 cm⁻¹. **HRMS (ESI, *m*/*z*)** calcd for [C₁₆H₂₀Cl₃NO₄Na] ⁺ [(M + Na) ⁺]: 418.0350, found: 418.0350.

### 2,2,2-Trichloroethyl ((2-(4-fluorophenyl)propanoyl)oxy)carbamate

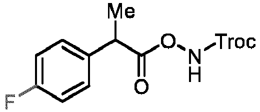

DCC coupling of 2-(4-fluorophenyl)propanoic acid with TrocNHOH gave the product as a colorless oil (chromatography on silica gel, eluent: EtOAc/n-hexane = 1/10): **¹H NMR** (300 MHz, CDCl₃) δ 8.22 (s, 1H), 7.36 - 7.22 (m, 2H), 7.09 - 6.92 (m, 2H), 4.76 (s, 2H), 3.89 (q, *J* = 7.2 Hz, 1H), 1.57 (d, *J* = 7.2 Hz, 3H). **¹³C NMR** (75 MHz, CDCl₃) δ 173.15, 162.31 (d, *J*_{C-F} = 246.5 Hz), 154.52, 134.13 (d, *J*= 3.0 Hz), 129.23 (d, *J*_{C-F} = 8.2 Hz), 115.77 (d, *J*_{C-F} = 21 Hz), 94.34, 75.18, 42.53, 18.55. **¹⁹F NMR** (282 MHz, CDCl₃) δ -114.47. **IR (film):** v (cm⁻¹) 3180, 2983, 1725, 1511, 1259, 1130, 1097, 854, 776, 587, 403 cm⁻¹. **HRMS (ESI, *m*/*z*)** calcd for [C₁₂H₁₁Cl₃FNO₄Na] ⁺ [(M + Na) ⁺]: 379.9630, found: 379.9630.

### 2,2,2-Trichloroethyl ((2-(4-chlorophenyl)propanoyl)oxy)carbamate

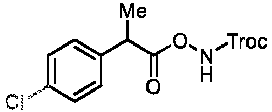

DCC coupling of 2-(4-chlorophenyl)propanoic acid with TrocNHOH gave the product as a white solid (chromatography on silica gel, eluent: EtOAc/n-hexane = 1/10): **¹H NMR** (300 MHz, CDCl₃) δ 8.25 (s, 1H), 7.37-7.23 (m, 4H), 4.80 (d, *J=* 12.0 Hz, 1H), 4.75 (d, *J* = 12.0 Hz, 1H), 3.90 (q, *J* = 7.2 Hz, 1H), 1.59 (d, J= 7.0 Hz, 3H). **¹³C** NMR (75 MHz, CDCl₃) δ 173.06, 154.57, 136.96, 133.91, 129.19, 129.11, 94.45, 75.32, 42.82, 18.55. IR (film): v (cm⁻¹) 3238, 1788, 1731, 1484, 1243, 1088, 1051, 719, 516 cm⁻¹. HRMS (ESI, *m*/*z)* calcd for [C₁₂H₁₁Cl₄NO₄Na] ⁺ [(M + Na) ⁺]: 395.9334, found: 395.9334.

### 2,2,2-Trichloroethyl ((2-(4-bromophenyl)propanoyl)oxy)carbamate

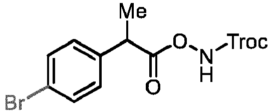

DCC coupling of 2-(4-bromophenyl)propanoic acid with TrocNHOH gave the product as a white solid (chromatography on silica gel, eluent: EtOAc/n-hexane = 1/10): **¹H** NMR (300 MHz, CDCl₃) δ 8.22 (s, 1H), 7.50 - 7.41 (m, 2H), 7.27 - 7.14 (m, 2H), 4.75 (s, 2H), 3.93-3.78 (m, 1H), 1.57 (d, *J* = 6.0 Hz, 3H). **¹³C** NMR (75 MHz, CDCl₃) δ 172.97, 154.56, 137.49, 132.16, 129.46, 122.00, 94.45, 75.33,42.90, 18.50. **IR (film):** v (cm⁻¹) 3210, 1793, 1732, 1487, 1266, 1117, 1024, 981, 719, 522 cm⁻¹. **HRMS (ESI, *m*/*z)*** calcd for [C₁₂H₁₁BrCl₃NO₄Na] ⁺ [(M + Na) ⁺]: 439.8829, found: 439.8829.

### 2,2,2-Trichloroethyl ((2-(4-(trifluoromethyl)phenyl)propanoyl)oxy)carbamate

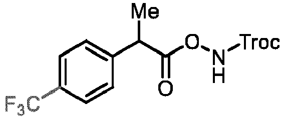

DCC coupling of 2-(4-(trifluoromethyl)phenyl)propanoic acid with TrocNHOH gave the product as a white solid (chromatography on silica gel, eluent: EtOAc/n-hexane = 1/10): **¹H NMR** (300 MHz, CDCl₃) δ 8.24 (s, 1H), 7.62 (d, *J* = 8.2 Hz, 2H), 7.47 (d, *J* = 8.1 Hz, 2H), 4.80 (d, *J* = 12.0 Hz, 1H), 4.75 (d, *J* = 12.0 Hz, 1H), 3.99 (q, *J* = 7.2 Hz, 1H), 1.63 (d, *J* = 7.2 Hz, 3H).**¹³C NMR** (75 MHz, CDCl₃) δ 172.76, 154.55, 142.42(d, *J_{C-F}* = 1.5 Hz), 130.33 (d, *J_{C-F}* = 32.5 Hz), 128.22, 126.02 (q, *J_{C-F}* = 3.8 Hz), 122.30, 94.43, 75.36, 43.30 18.57. **¹⁹F NMR** (282 MHz, CDCl₃) δ -62.65. **IR (film):** v (cm⁻¹) 3222, 1794, 1735, 1322, 1265, 1113, 1067, 842, 722, 605, 451 cm⁻¹. **HRMS (ESI, *m*/*z*)** calcd for [C₁₃H₁₁Cl₃F₃NO₄Na] ⁺ [(M + Na) ⁺]: 429.9598, found: 429.9598.

### 2,2,2-Trichloroethyl ((2-(4-(methylthio)phenyl)propanoyl)oxy)carbamate

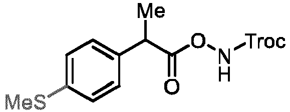

DCC coupling of 2-(4-(methylthio)phenyl)propanoic acid with TrocNHOH gave the product as a white solid (chromatography on silica gel, eluent: EtOAc/n-hexane = 1/10): **¹H NMR** (300 MHz, CDCl₃) δ 8.31 (s, 1H), 7.35 - 7.23 (m, 4H), 4.85 (d, *J* = 12.0 Hz, 1H), 4.80 (d, *J* = 12.0 Hz, 1H), 3.93 (q, *J* = 7.2 Hz, 1H), 2.52 (s, 3H), 1.63 (d, *J* = 7.2 Hz, 3H). **¹³C NMR** (75 MHz, CDCl₃) δ 173.19, 154.48, 138.22, 135.15, 128.05, 126.97, 94.36, 75.17, 42.76, 18.38, 15.79. **IR (film):** v (cm⁻¹) 3245, 1737, 1476, 1237, 1127, 809, 717, 566 cm⁻¹. **HRMS (ESI, *m*/*z*)** calcd for [C₁₃H₁₄Cl₃NO₄SNa] ⁺ [(M + Na) ⁺]: 407.9601, found: 407.9601.

### 2,2,2-Trichloroethyl ((2-(4-methoxyphenyl)propanoyl)oxy)carbamate

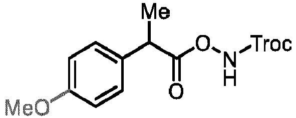

DCC coupling of 2-(4-methoxyphenyl)propanoic acid with TrocNHOH gave the product as a white solid (chromatography on silica gel, eluent: EtOAc/n-hexane = 1/10): **¹H** NMR (300 MHz, CDCl₃) δ 8.26 (s, 1H), 7.27 (d, *J* = 8.8 Hz, 2H), 6.89 (d, *J* = 8.7 Hz, 2H), 4.80 (d, *J* = 12.0 Hz, 1H), 4.75 (d, *J* = 12.0 Hz, 1H), 3.88 (q, *J =* 7.2 Hz, 1H), 3.81 (s, 3H), 1.59 (d, *J =* 7.2 Hz, 3H). **¹³C** NMR (75 MHz, CDCl₃) δ 173.65, 159.29, 154.63, 130.60, 128.77, 114.41, 94.51, 75.28, 55.43, 42.56, 18.60. IR (film): v (cm⁻¹) 3247, 1737, 1507, 1472, 1274, 1099, 812, 731, 535 cm⁻¹. HRMS (ESI, *m*/*z)* calcd for [C₁₃H₁₄Cl₃NO₅Na] ⁺ [(M + Na) ⁺]: 391.9830, found: 391.9830.

### 2,2,2-Trichloroethyl ((2-(3-phenoxyphenyl)propanoyl)oxy)carbamate

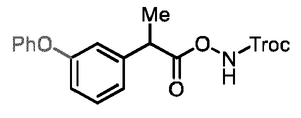

DCC coupling of 2-(3-phenoxyphenyl)propanoic acid with TrocNHOH gave the product as a colorless oil (chromatography on silica gel, eluent: EtOAc/n-hexane = 1/10): **¹H NMR** (300 MHz, CDCl₃) δ 8.27 (s, 1H), 7.40 - 7.25 (m, 3H), 7.17 - 6.98 (m, 5H), 6.95 - 6.86 (m, 1H), 4.79 (d, *J=* 12.0 Hz, 1H), 4.74(d, *J =* 12.0 Hz, 1H), 3.89 (q, *J* = 7.2 Hz, 1H), 1.59 (d, *J* = 7.2 Hz, 3H). **¹³C NMR** (75 MHz, CDCl₃) δ 173.12, 157.81, 156.95, 154.58, 140.43, 130.24, 129.95, 123.65, 122.42, 119.18, 118.25, 117.98, 94.49, 75.28, 43.24, 18.50. **IR (film):** v (cm⁻¹) 3294, 1750, 1583, 1485, 1229, 1113, 691, 568 cm⁻¹. **HRMS (ESI, *m*/*z)*** calcd for [C₁₈H₁₆Cl₃NO₅Na] ⁺ [(M + Na) ⁺]: 453.9986, found: 453.9986.

### 2,2,2-Trichloroethyl ((2-(3-benzoylphenyl)propanoyl)oxy)carbamate

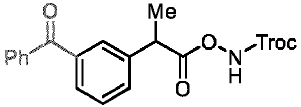

DCC coupling of 2-(3-benzoylphenyl)propanoic acid with TrocNHOH gave the product as a colorless oil (chromatography on silica gel, eluent: EtOAc/n-hexane = 1/10): **¹H NMR** (300 MHz, CDCl₃) δ 8.33 (s, 1H), 7.84 - 7.75 (m, 3H), 7.75 - 7.66 (m, 1H), 7.66 - 7.55 (m, 2H), 7.54 - 7.41 (m, 3H), 4.79 (d, *J* = 12.0 Hz, 1H), 4.74(d, *J* = 12.0 Hz, 1H), 4.00 (q, *J* = 7.2 Hz, 1H), 1.64 (d, *J* = 7.2 Hz, 3H). **¹³C NMR** (75 MHz, CDCl₃) δ 196.27, 172.88, 154.44, 138.82, 138.20, 137.33, 132.63, 131.55, 130.11, 129.61, 129.19, 128.82, 128.38, 94.35, 75.16, 43.17, 18.46. **IR (film):** v (cm⁻¹) 3271, 1763, 1653, 1448, 1282, 1114, 709, 568 cm⁻¹. **HRMS (ESI, *m*/*z*)** calcd for [C₁₉H₁₆Cl₃NO₅Na] ⁺ [(M + Na) ⁺]: 465.9986, found: 465.9986.

### 2,2,2-Trichloroethyl ((2-(2-fluoro-[1,1'-biphenyl]-4-yl)propanoyl)oxy)carbamate

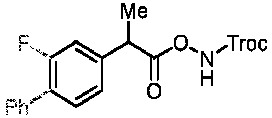

DCC coupling of 2-(2-fluoro-[1,1'-biphenyl]-4-yl)propanoic acid with TrocNHOH gave the product as a white solid (chromatography on silica gel, eluent: EtOAc/n-hexane = 1/10): **¹H NMR** (300 MHz, CDCl₃) δ 8.22 (s, 1H), 7.47 - 7.20 (m, 6H), 7.12 - 7.00 (m, 2H), 4.70 (d, *J* = 12.0 Hz, 1H), 4.66 (d, *J* = 12.0 Hz, 1H), 3.85 (q, *J* = 7.2 Hz, 1H), 1.53 (d, *J* = 7.2 Hz, 3H).**¹³C NMR** (75 MHz, CDCl₃) δ 172.91, 159.9 (d, *J_{C-F}* = 246.7 Hz), 154.62, 139.68 (d, *J_{C-F} =* 7.7 Hz), 135.36 (d, *J_{C-F} =* 1.7 Hz), 131.22 (d, *J_{C-F}* = 3.9 Hz), 129.07 (d, *J_{C-F}* = 2.8 Hz), 128.60 (d, *J_{C-F} =* 1.9 Hz), 127.96, 123.77 (d, *J_{C-F}* = 3.6 Hz), 115.53 (d, *J_{C-F} =* 23.9 Hz), 94.46, 75.32, 42.88 (d, *J_{C-F} =* 1.7 Hz), 18.47. **¹⁹F NMR** (282 MHz, CDCl₃) δ - 116.88. **IR (film):** v (cm⁻¹) 3294, 1753, 1451, 1224, 1111, 696, 569 cm⁻¹. **HRMS (ESI, *m*/*z*)** calcd for [C₁₈H₁₅Cl₃FNO₄ Na] ⁺ [(M + Na) ⁺]: 455.9943, found: 455.9943.

### 2,2,2-Trichloroethyl ((2-(3,5-dimethylphenyl)propanoyl)oxy)carbamate

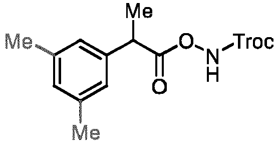

DCC coupling of 2-(3,5-dimethylphenyl)propanoic acid with TrocNHOH gave the product as a white solid (chromatography on silica gel, eluent: EtOAc/n-hexane = 1/10): **¹H NMR** (300 MHz, CDCl₃) δ 8.29 (s, 1H), 7.00 (s, 3H), 4.87 (d, J= 12.0 Hz, 1H), 4.82(d, J= 12.0 Hz, 1H), 3.91 (q, J= 7.1 Hz, 1H), 2.38 (s, 6H), 1.64 (d, *J* = 7.2 Hz, 3H). **¹³C NMR** (75 MHz, CDCl₃) δ 173.56, 154.62, 138.59, 138.45, 129.58, 125.45, 94.52, 75.28, 43.25, 21.40, 18.56. **IR (film):** v (cm⁻¹) 3230, 2961, 1736, 1486, 1239, 1084, 753, 618, 564 cm⁻¹. **HRMS (ESI, *m*/*z*)** calcd for [C₁₄H₁₆Cl₃NO₄Na] ⁺ [(M + Na) ⁺]: 390.0037, found: 390.0037.

### 2,2,2-Trichloroethyl ((2-(2,4-difluorophenyl)propanoyl)oxy)carbamate

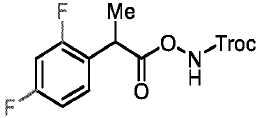

DCC coupling of 2-(2,4-difluorophenyl)propanoic acid with TrocNHOH gave the product as a colorless oil (chromatography on silica gel, eluent: EtOAc/n-hexane = 1/10): **¹H NMR** (300 MHz, CDCl₃) δ 8.27 (s, 1H), 7.40 - 7.26 (m, 1H), 7.02 - 6.76 (m, 2H), 4.81 (d, *J* = 12.0 Hz, 1H), 4.76(d, *J* = 12.0 Hz, 1H), 4.18 (q, *J* = 7.2 Hz, 1H), 1.59 (d, *J* = 7.3 Hz, 3H). **¹³C NMR** (75 MHz, CDCl₃) δ 172.68, 162.56 (dd, *J_{C-F}* = 247.5, 12.0 Hz), 160.47 (dd, *J_{C-F}* = 247.5, 12.0 Hz), 154.58, 129.74 (dd, *J_{C-F}* = 9.6, 5.2 Hz), 121.86 (dd, *J_{C-F}* = 15.0, 4.0 Hz), 111.89 (dd, *J_{C-F}* = 21.3, 3.7 Hz), 104.27 (t, *J_{C-F}* = 25.7 Hz), 94.46, 75.34, 36.13 (d, *J_{C-F}* = 2.8 Hz), 17.59. **¹⁹F NMR** (282 MHz, CDCl₃) δ -110.47, -113.44. **IR (film):** v (cm⁻¹) 3292, 1750, 1504, 1278, 1115, 716, 568 cm⁻¹. **HRMS (ESI, *m*/*z*)** calcd for [C₁₂H₁₀Cl₃F₂NO₄Na] ⁺ [(M + Na) ⁺]: 397.9536, found: 397.9536.

### 2,2,2-Trichloroethyl ((2-(benzo[d][1,3]dioxol-5-yl)propanoyl)oxy)carbamate

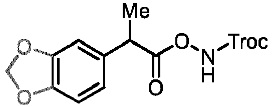

DCC coupling of 2-(benzo[*d*][1,3]dioxol-5-yl)propanoic acid with TrocNHOH gave the product as a white solid (chromatography on silica gel, eluent: EtOAc/*n*-hexane = 1/10): **¹H NMR** (300 MHz, CDCl₃) δ 8.25 (s, 1H), 6.83 (s, 1H), 6.80 - 6.71 (m, 2H), 5.94 (s, 2H), 4.83 - 4.70 (m, 2H), 3.83 (q, *J* = 6.0 Hz, 1H), 1.55 (d, *J* = 6.0 Hz, 3H). **¹³C NMR** (75 MHz, CDCl₃) δ 173.42, 154.61, 148.16, 147.33, 132.22, 121.12, 108.61, 108.08, 101.34, 94.49, 75.30, 43.03, 18.67. **IR (film):** v (cm⁻¹) 3232, 1735, 1481, 1233, 1103, 1037, 716, 566 cm⁻¹. **HRMS (ESI, *m*/*z*)** calcd for [C₁₃H₁₂Cl₃NO₆Na] ⁺ [(M + Na) ⁺]: 405.9622, found: 405.9622.

### 2,2,2-Trichloroethyl ((2-(naphthalen-2-yl)propanoyl)oxy)carbamate

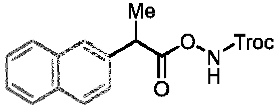

DCC coupling of 2-(naphthalen-2-yl)propanoic acid with TrocNHOH gave the product as a white solid (chromatography on silica gel, eluent: EtOAc/n-hexane = 1/10): **¹H NMR** (300 MHz, CDCl₃) δ 8.24 (s, 1H), 7.88 - 7.76 (m, 4H), 7.56 - 7.41 (m, 3H), 4.79 (d, *J* = 12.0 Hz, 1H), 4.74 (d, *J* = 12.0 Hz, 1H), 4.09 (q, *J* = 7.2 Hz, 1H), 1.70 (d, *J* = 7.2 Hz, 3H). **¹³C NMR** (75 MHz, CDCl₃) δ 173.40, 154.61, 135.91, 133.56, 132.96, 128.83, 127.99, 127.81, 126.66, 126.55, 126.33, 125.52, 94.48, 75.28, 43.55, 18.56. **IR (film):** v (cm⁻¹) 3202, 1793, 1448, 1137, 858, 712, 535, 475 cm⁻¹. **HRMS (ESI, *m*/*z*)** calcd for [C₁₆H₁₄Cl₃NO₄Na] ⁺ [(M + Na) ⁺]: 411.9881, found: 411.9881.

### 2,2,2-Trichloroethyl ((2-(thiophen-3-yl)propanoyl)oxy)carbamate

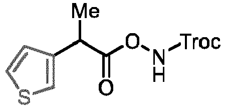

DCC coupling of 2-(thiophen-3-yl)propanoic acid with TrocNHOH gave the product as a white solid (chromatography on silica gel, eluent: EtOAc/*n*-hexane = 1/10): **¹H NMR** (300 MHz, CDCl₃) δ 8.33 (s, 1H), 7.35 - 7.26 (m, 1H), 7.25 - 7.18 (m, 1H), 7.09 (dd, *J* = 5.0, 1.4 Hz, 1H), 4.81 (d, *J* = 12.0 Hz, 1H), 4.76 (d, *J* = 12.0 Hz, 1H), 4.04 (q, *J* = 7.2 Hz, 1H), 1.61 (d, *J* = 7.2 Hz, 3H).**¹³C NMR** (75 MHz, CDCl₃) δ 173.00, 154.64, 138.48, 126.99, 126.37, 122.25, 94.48, 75.29, 38.87, 18.25. **IR (film):** v (cm⁻¹) 3259, 1762, 1380, 1313, 1093, 891, 754, 693, 563 cm⁻¹. **HRMS (ESI, *m*/*z)*** calcd for [C₁₀H₁₀Cl₃NO₄SNa] ⁺ [(M + Na) ⁺]: 367.9288, found: 367.9288.

### 2,2,2-Trichloroethyl ((2-(quinolin-6-yl)propanoyl)oxy)carbamate

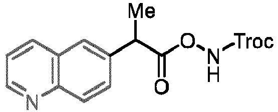

EDCI coupling of 2-(quinolin-6-yl)propanoic acid with TrocNHOH gave the product as a white solid (chromatography on silica gel, eluent: CH₂Cl₂/MeOH = 1/15): **¹H NMR** (300 MHz, CDCl₃) δ 8.88 (d, *J* = 4.5 Hz, 1H), 8.09 (d, *J* = 8.2 Hz, 1H), 7.85 (d, *J* = 8.9 Hz, 1H), 7.72 (s, 1H), 7.51 (d, *J* = 8.5 Hz, 1H), 7.38 (dd, *J=* 8.3, 4.2 Hz, 1H), 4.81 (s, 2H), 4.12 (q, *J* = 7.1 Hz, 1H), 1.67 (d, *J=* 7.0 Hz, 3H). ¹³C **NMR** (75 MHz, CDCl₃) δ 173.00, 155.01, 150.47, 147.08, 137.33, 136.68, 129.55, 129.34, 128.33, 126.49, 121.65, 94.75, 75.20, 43.41, 18.39. **IR (film):** v (cm⁻¹) 3248, 1751, 1507, 1234, 1069, 758, 709, 560 cm⁻¹. **HRMS (ESI, *m*/*z)*** calcd for [C₁₅H₁₄Cl₃N₂O₄] ⁺ [(M + H) ⁺]: 391.0014, found: 391.0014.

### 2,2,2-Trichloroethyl ((2-(6-chloro-9H-carbazol-2-yl)propanoyl)oxy)carbamate

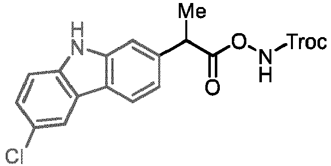

DCC coupling of 2-(6-chloro-9*H*-carbazol-2-yl)propanoic acid with TrocNHOH gave the product as a white solid (chromatography on silica gel, eluent: EtOAc/n-hexane = 1/2): **¹H NMR** (300 MHz, CDCl₃) δ 8.31 (s, 1H), 8.10 (s, 1H), 7.96 - 7.86 (m, 2H), 7.38 - 7.22 (m, 3H), 7.16 (dd, *J* = 8.1, 1.6 Hz, 1H), 4.78 (d, *J =* 12.0 Hz, 1H), 4.74 (d, *J =* 12.0 Hz, 1H), 4.04 (q, *J* = 7.2 Hz, 1H), 1.66 (d, *J* = 7.2 Hz, 3H). **¹³C NMR** (75 MHz, CDCl₃) δ 173.61, 154.71, 140.39, 138.21, 137.01, 126.20, 125.20, 124.21, 122.19, 120.94, 120.16, 119.54, 111.79, 109.94, 94.47, 75.30, 43.78, 18.89. **IR (film):** v (cm⁻¹) 3429, 3314, 1759, 1449, 1193, 997, 804, 732, 581 cm⁻¹. **HRMS (ESI, *m*/*z)*** calcd for [C₁₈H₁₄Cl₄N₂O₄Na] ⁺ [(M + Na) ⁺]: 484.9600, found: 484.9611.

### 2,2,2-Trichloroethyl ((2-phenylbutanoyl)oxy)carbamate

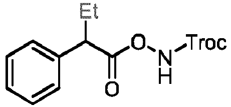

DCC coupling of 2-phenylbutanoic acid with TrocNHOH gave the product as a white solid (chromatography on silica gel, eluent: EtOAc/n-hexane = 1/10): **¹H NMR** (300 MHz, CDCl₃) δ 8.21 (s, 1H), 7.39 - 7.26 (m, 5H), 4.80 (d, *J* = 12.0 Hz, 1H), 4.78 (d, *J* = 12.0 Hz, 1H), 3.65 (t, *J* = 7.7 Hz, 1H), 2.27 - 2.09 (m, 1H), 2.01 - 1.80 (m, 1H), 0.95 (t, *J* = 7.4 Hz, 3H). **¹³C NMR** (75 MHz, CDCl₃) δ 172.99, 154.61, 137.16, 128.97, 128.19, 127.97, 94.51, 75.28, 51.10, 26.89, 12.09. **IR (film):** v (cm⁻¹) 3344, 1746, 1381, 1094, 1046, 939, 841, 718, 569 cm⁻¹. **HRMS (ESI, *m*/*z)*** calcd for [C₁₃H₁₄Cl₃NO₄Na]⁺ [(M + Na)⁺]: 375.9881, found: 375.9881.

### 2,2,2-Trichloroethyl ((2-phenylpentanoyl)oxy)carbamate

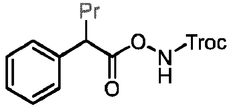

DCC coupling of 2-phenylpentanoic acid with TrocNHOH gave the product as a white solid (chromatography on silica gel, eluent: EtOAc/n-hexane = 1/15): **¹H NMR** (300 MHz, CDCl₃) δ 8.24 (s, 1H), 7.37-7.28 (m, 5H), 4.79 (d, *J* = 12.0 Hz, 1H), 4.75 (d, *J* = 12.0 Hz, 1H), 3.75 (t, *J* = 7.7 Hz, 1H), 2.22 - 2.04 (m, 1H), 1.92 - 1.79 (m, 1H), 1.40 - 1.27 (m, 2H), 0.93 (t, *J* = 7.3 Hz, 3H). **¹³C NMR** (75 MHz, CDCl₃) δ 173.06, 154.61, 137.33, 128.96, 128.17, 127.93, 94.50, 75.27, 49.13, 35.61, 20.65, 13.81. **IR (film):** v (cm⁻¹) 3351, 1751, 1319, 1183, 1104, 753, 695, 512 cm⁻¹. **HRMS (ESI, *m*/*z)*** calcd for [C₁₄H₁₆Cl₃NO₄Na]⁺ [(M + Na)⁺]: 390.0037, found: 390.0037.

### 2,2,2-Trichloroethyl ((2-phenyloctanoyl)oxy)carbamate

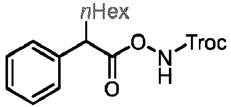

DCC coupling of 2-phenyloctanoic acid with TrocNHOH gave the product as a colorless oil (chromatography on silica gel, eluent: EtOAc/n-hexane = 1/15): **¹H NMR** (300 MHz, CDCl₃) δ 8.17 (s, 1H), 7.33 - 7.18 (m, 5H), 4.79 (d, *J* = 12.0 Hz, 1H), 4.75 (d, *J* = 12.0 Hz, 1H), 3.66 (t, *J* = 7.7 Hz, 1H), 2.16 - 1.99 (m, 1H), 1.89 - 1.74 (m, 1H), 1.31 - 1.09 (m, 8H), 0.80 (t, *J* = 6.8 Hz, 3H). **¹³C NMR** (75 MHz, CDCl₃) δ 173.07, 154.59, 137.36, 128.96, 128.16, 127.93, 94.51, 75.27, 49.40, 33.54, 31.63, 29.01, 27.37, 22.67, 14.14. **IR (film):** v (cm⁻¹) 3296, 2928, 1752, 1453, 1221, 1114, 893, 720, 697, 571 cm⁻¹. **HRMS (ESI, *m*/*z)*** calcd for [C₁₇H₂₂Cl₃NO₄Na]⁺ [(M + Na)⁺]: 432.0507, found: 432.0511.

### 2,2,2-Trichloroethyl ((3-methyl-2-phenylbutanoyl)oxy)carbamate

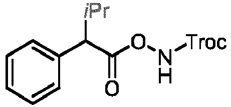

DCC coupling of 3-methyl-2-phenylbutanoic acid with TrocNHOH gave the product as a white solid (chromatography on silica gel, eluent: EtOAc/n-hexane = 1/15): **¹H NMR** (300 MHz, CDCl₃) δ 8.24 (s, 1H), 7.37 - 7.23 (m, 5H), 4.77 (s, 2H), 3.35 (d, *J* = 10.5 Hz, 1H), 2.50 - 2.30 (m, 1H), 1.11 (d, *J* = 6.5 Hz, 3H), 0.76 (d, *J* = 6.7 Hz, 3H). **¹³C NMR** (75 MHz, CDCl₃) δ 172.85, 154.58, 137.30, 128.87, 128.69, 128.00, 94.51, 75.25, 57.45, 32.32, 21.40, 20.27. **IR (film):** *v* (cm⁻¹) 3292, 1746, 1388, 1105, 1047, 892, 698, 610 cm⁻¹. **HRMS (ESI, *m*/*z)*** calcd for [C₁₄H₁₆Cl₃NO₄Na]⁺ [(M + Na)⁺]: 390.0037, found: 390.0037.

### 2,2,2-Trichloroethyl (2-cyclopentyl-2-phenylacetoxy)carbamate

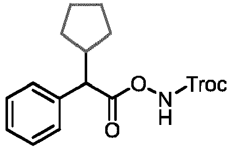

DCC coupling of 2-cyclopentyl-2-phenylacetic acid with TrocNHOH gave the product as a white solid (chromatography on silica gel, eluent: EtOAc/n-hexane = 1/15): **¹H NMR** (300 MHz, CDCl₃) δ 8.23 (s, 1H), 7.39 - 7.23 (m, 5H), 4.77 (s, 2H), 3.47 (d, *J* = 11.1 Hz, 1H), 2.72 - 2.52 (m, 1H), 2.04 - 1.92 (m, 1H), 1.76 - 1.20 (m, 6H), 1.12 - 0.99 (m, 1H). **¹³C NMR** (75 MHz, CDCl₃) δ 172.82, 154.59, 137.19, 128.88, 128.46, 127.94, 94.52, 75.26, 55.32, 43.60, 31.51, 30.91, 25.29, 24.88. **IR(film):** v (cm⁻¹) 3347, 1753, 1324, 1162, 1105, 890, 696, 576 cm⁻¹. **HRMS (ESI, *m*/*z)*** calcd for [C₁₆H₁₈Cl₃NO₄Na] ⁺ [(M + Na) ⁺]: 416.0194, found: 416.0194.

### 2,2,2-Trichloroethyl (2-cyclohexyl-2-phenylacetoxy)carbamate

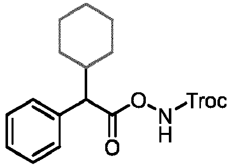

DCC coupling of 2-cyclohexyl-2-phenylacetic acid with TrocNHOH gave the product as a white solid (chromatography on silica gel, eluent: EtOAc/n-hexane = 1/15): **¹H NMR** (300 MHz, CDCl₃) δ 8.24 (s, 1H), 7.36 - 7.23 (m, 5H), 4.76 (s, 2H), 3.42 (d, *J=* 10.5 Hz, 1H), 2.17 - 1.99 (m, 1H), 1.96 - 1.86 (m, 1H), 1.81 - 1.72 (m, 1H), 1.68 - 1.60 (m, 2H), 1.41 - 1.04 (m, 5H), 0.88 - 0.70 (m, **1H). ¹³C NMR** (75 MHz, CDCl₃) δ 172.80, 154.57, 136.10, 128.85, 128.78, 127.94, 94.51, 75.27, 56.29, 41.34, 31.90, 30.47, 26.28, 26.00, 25.98. **IR (film):** v (cm⁻¹) 3347, 1765, 1311, 1077, 992, 721, 698 cm⁻¹. **HRMS (ESI, *m*/*z)*** calcd for [C₁₇H₂₀Cl₃NO₄Na] ⁺ [(M + Na) ⁺]: 430.0350, found: 430.0352.

### 2,2,2-Trichloroethyl ((1,2,3,4-tetrahydronaphthalene-1-carbonyl)oxy)carbamate

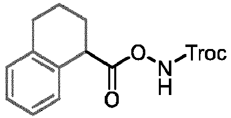

DCC coupling of 1,2,3,4-tetrahydronaphthalene-1-carboxylic acid with TrocNHOH gave the product as a colorless oil (chromatography on silica gel, eluent: EtOAc/n-hexane = 1/10): **¹H NMR** (300 MHz, CDCl₃) δ 8.33 (s, 1H), 7.29 - 7.09 (m, 4H), 4.81 (s, 2H), 4.04 (t, *J* = 5.8 Hz, 1H), 2.95 - 2.70 (m, 2H), 2.32 - 1.93 (m, 3H), 1.90 - 1.73 (m, 1H). **¹³C NMR**(75 MHz, CDCl₃) δ 173.86, 154.75, 137.51, 131.39, 129.78, 129.53, 127.61, 126.23, 94.53, 75.33, 42.71, 29.00, 26.70, 20.47. **IR (film):** v (cm⁻¹) 3292, 2941, 1749, 1450, 1225, 1113, 740, 570 cm⁻¹. **HRMS (ESI, *m*/*z)*** calcd for [C₁₄H₁₄Cl₃NO₄Na] ⁺ [(M + Na) ⁺]: 387.9881, found: 387.9881.

### 2,2,2-Trichloroethyl(((6R)-6-((3R,8R,9S,10S,13R,14S,17R)-3-((tert-butyldimethylsilyl)oxy)-10,13-dimethylhexadecahydro-1H-cyclopenta[a]phenanthren-17-yl)-2-phenylheptanoyl)oxy)carbamate

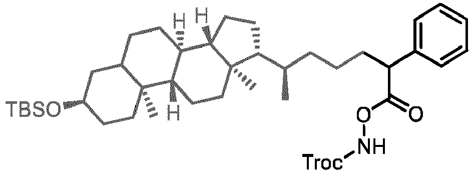

DCC coupling of (6*R*)-6-((3*R*,8*R*,9*S*,10*S*,13*R*,14*S*,17*R*)-3-((*tert*-butyldimethylsilyl)oxy)-10,13-dimethylhexadecahydro-1*H*-cyclopenta[*a*]phenanthren-17-yl)-2-phenylheptanoic acid with TrocNHOH gave product (dr:1/1) as a white solid (chromatography on silica gel, eluent: EtOAc/n-hexane = 1/10): **¹H NMR** (300 MHz, CDCl₃) δ 8.16 (s, 1H), 7.29 - 7.18 (m, 5H), 4.70 (s, 2H), 3.71 - 3.61 (m, 1H), 3.58 - 3.44 (m, 1H), 2.15 - 1.63 (m, 7H), 1.55 - 0.89 (m, 23H), 0.85 - 0.72 (m, 15H), 0.54 (d, *J=* 3.3 Hz, 3H), -0.01 (s, 6H). **¹³C NMR** (75 MHz, CDCl₃) δ 173.13, 173.08, 154.56, 137.43, 137.30, 128.97, 128.21, 128.14, 127.94, 94.53, 75.28, 73.00, 56.57, 56.43, 56.39, 49.44, 42.85, 42.47, 40.40, 40.31, 37.09, 36.03, 35.75, 35.73, 35.68, 35.62, 34.75, 34.02, 33.92, 31.19, 28.47, 27.47, 26.57, 26.14, 24.38, 24.13, 24.03, 23.55, 20.97, 18.68, 18.65, 18.48, 12.17, -4.43. **IR (film):** v (cm⁻¹) 2929, 2857, 1768, 1450, 1376, 1253, 1077, 869, 725, 576 cm⁻¹. **HRMS (ESI, *m*/*z)*** calcd for [C₄₁H₆₄Cl₃NO₅SiNa] ⁺ [(M + Na) ⁺]: 806.3512, found: 806.3512.

### 2,2,2-Trichloroethyl (E)-((2-methyl-4-phenylbut-3-enoyl)oxy)carbamate

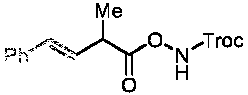

DCC coupling of 2-methyl-4-phenylbut-3-enoic acid with TrocNHOH gave the product (Z/E:1/8.5) as a white solid (chromatography on silica gel, eluent: EtOAc/n-hexane = 1/10): **¹H NMR** (300 MHz, CDCl₃) δ 8.35 (s, 1H), 7.44 - 7.16 (m, 5H), 6.59 (d, *J* = 15.9 Hz, 1H), 6.28 (dd, *J* = 15.9, 7.9 Hz, 1H), 4.83 (s, 2H), 3.85 - 3.43 (m, 1H), 1.59 - 1.38 (m, 3H). **¹³C NMR** (75 MHz, CDCl₃) δ 173.37, 154.67, 136.46, 132.85, 128.75, 128.07, 126.59, 126.45, 94.50, 75.32, 41.16, 17.47. **IR (film):** v (cm⁻¹) 3241, 1790, 1729, 1488, 1259, 1097, 970, 719, 560 cm⁻¹. **HRMS (ESI, *m*/*z)*** calcd for [C₁₄H₁₄Cl₃NO₄Na] ⁺ [(M + Na) ⁺]: 387.9881, found: 387.9881.

### 3.2. Synthesis of N-Boc-protected azanyl esters

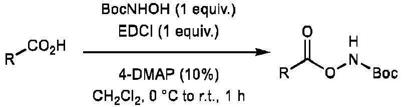

N Boc-protected azanyl esters were prepared by EDCI coupling of carboxylic acids with BocNHOH. The data of three representatives are shown below.

### tert-Butyl (2-phenylacetoxy)carbamate

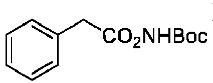

EDCI coupling gave the product as a yellow oil (chromatography on silica gel, eluent: EtOAc/n-hexane = 1/6): ¹H NMR (300 MHz, CDCl₃) δ 7.91 (s, 1H), 7.39-7.27 (m, 5H), 3.77 (s, 2H), 1.46 (s, 9H). ¹³C NMR (75 MHz, CDCl₃) δ 170.8, 155.4, 132.3, 129.3, 128.7, 127.5, 83.3, 38.6, 28.0. HRMS (ESI) calculated for [C₁₃H₁₇NNaO4]⁺ [M + Na]⁺: 274.1050, found: 274.1054.

### tert-Butyl ((5-phenylpentanoyl)oxy)carbamate

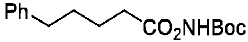

EDCI coupling gave the product as a colorless oil (chromatography on silica gel, eluent: EtOAc/n-hexane = 1/10): ¹H NMR (300 MHz, CDCl₃) δ 7.94 (s, 1H), 7.31-7.15 (m, 5H), 2.64 (t, *J* = 7.0 Hz, 2H), 2.47 (t, *J* = 7.1 Hz, 2H), 1.79-1.63 (m, 4H), 1.49 (s, 9H). ¹³C NMR(75 MHz, CDCl₃) δ 172.7, 155.5, 141.8, 128.3, 128.3, 125.8, 83.1, 35.3, 31.6, 30.6, 28.0, 24.2. HRMS (ESI) calculated for [C₁₆H₂₃NNaO₄]⁺ [M + Na]⁺: 316.1519, found: 316.1524.

### tert-Butyl ((2-phenylpropanoyl)oxy)carbamate

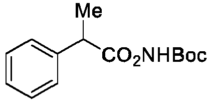

EDCI coupling gave the product as a white gum (chromatography on silica gel, eluent: EtOAc/n-hexane = 1/6): ¹H NMR (300 MHz, CDCl₃) δ 7.77 (s, 1H), 7.39-7.27 (m, 5H), 3.89 (q, *J* = 7.2 Hz, 1H), 1.59 (d, *J* = 7.3 Hz, 3H), 1.44 (s, 9H). ¹³C NMR (75 MHz, CDCl₃) δ 173.8, 155.4, 138.8, 128.8, 127.6 (2C), 83.2, 43.4, 27.9, 18.5. HRMS (ESI) Exact mass calculated for [C₁₃H₁₇NNaO4]⁺ [M + Na]⁺: 288.1206, found: 288.1209.

### 4. Ruthenium-Catalyzed 1,3-Nitrogen Migration to Access N-Troc-Protected α-Monosubstituted and α,α-Disubstituted α-Amino Acids

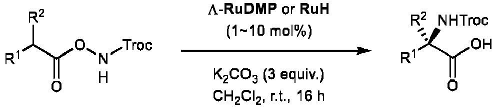

General procedures: to a Schlenk tube was added the substrate (1 equiv.), K₂CO₃ (3 equiv.) and the indicated A-ruthenium catalyst (1~10 mol%). The tube was evacuated and backfilled with N₂ for three times. Dichloromethane (0.05 M) was added, and the tube was sealed. The reaction mixture was stirred at room temperature (25 °C) for 16 hours. To quench the reaction, brine (10 mL) and concentrated hydrochloric acid (1 mL/mmol substrate) was added, and the mixture was diluted with water (1 mL). The mixture was extracted with EtOAc for three times and the combined organic layer was dried over anhydrous sodium sulfate. After filtration, the solvent was evaporated under reduced pressure, and the residue was purified by column chromatography on silica gel using indicated solvent as the eluent.

### (R)-2-Phenyl-2-(((2,2,2-trichloroethoxy)carbonyl)amino)acetic acid (2e)

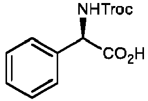

Catalyzed by 1 mol% Λ-**RuDMP**, the 1,3-nitrogen shift of 2,2,2-trichloroethyl (2-phenylacetoxy)carbamate (49.0 mg, 0.15 mmol) gave **2e** as a white solid (chromatography on silica gel, eluent: EtOAc/n-hexane = 1/5 with 0.1% TFA, 44.8 mg, 91% yield) with 95% e.e. [DAICEL CHIRALPAK IG column, Agilent HPLC 1260, iPrOH/n-hexane = 10/90 (v/v) with 0.1% TFA, 1.0 mL/min, 25 °C, 210 nm; t₁ = 15.6 min, t₂ = 20.9 min]: [α]_{D}²⁵ = -132.4 (*c* 1.0, MeOH, 95% e.e.); IR (film) *v*ₘₐₓ: 1692, 1425, 1042, 717 cm⁻¹; ¹H NMR (300 MHz, CD₃OD) δ 4.77 (d, *J* = 12.2 Hz, 1H), 4.80 (d, *J* = 12.2 Hz, 1H), 5.29 (s, 1H), 7.30-7.40 (m, 3H), 7.40-7.49 (m, 2H); ¹³C NMR (75 MHz, CD₃OD) δ 59.8, 75.6, 97.0, 128.6, 129.4, 129.7, 138.1, 156.2, 173.5; HRMS calcd for [C₁₁H₁₀Cl₃NO₄Na]⁺ [(M + Na)⁺]: 347.9568; found: 347.9576.

### (R)-2-(p-Tolyl)-2-(((2,2,2-trichloroethoxy)carbonyl)amino)acetic acid (3)

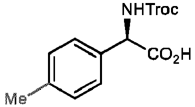

Catalyzed by 1 mol% A-**RuDMP**, the 1,3-nitrogen shift of 2,2,2-trichloroethyl (2-(p-tolyl)acetoxy)carbamate (51.1 mg, 0.15 mmol) gave **3** as a white solid (chromatography on silica gel, eluent: EtOAc/n-hexane = 1/5 with 0.1% TFA, 43.8 mg, 86% yield) with 97% e.e. [DAICEL CHIRALPAK IG column, Agilent HPLC 1260, *i*PrOH/n-hexane = 10/90 (v/v) with 0.1% TFA, 1.0 mL/min, 25 °C, 210 nm; t₁ = 15.4 min, t₂ = 23.6 min]: [α]_{D}²⁵ = -119.5 (c 1.0, MeOH, 97% e.e.); IR (film) *v*ₘₐₓ: 2924, 1717, 1509, 1041, 814, 716 cm⁻¹; ¹H NMR (300 MHz, CD₃OD) δ 2.32 (s, 3H), 4.77 (d, *J* = 12.2 Hz, 1H), 4.79 (d, *J* = 12.2 Hz, 1H), 5.24 (s, 1H), 7.13-7.22 (m, 2H), 7.26-7.35 (m, 2H); ¹³C NMR (75 MHz, CD₃OD) δ 21.2, 59.5, 75.6, 97.0, 128.6, 130.3, 135.0, 139.4, 156.2, 173.8; HRMS calcd for [C₁₂H₁₂Cl₃NO₄Na]⁺ [(M + Na)⁺]: 361.9724; found: 361.9727.

### (R)-2-([1,1'-Biphenyl]-4-yl)-2-(((2,2,2-trichloroethoxy)carbonyl)amino)acetic acid (4)

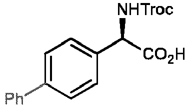

Catalyzed by 1 mol% Λ-**RuDMP**, the 1,3-nitrogen shift of 2,2,2-trichloroethyl (2-([1,1'-biphenyl]-4-yl)acetoxy)carbamate (60.4 mg, 0.15 mmol) gave **4** as a white solid (chromatography on silica gel, eluent: EtOAc/n-hexane = 1/5 with 0.1% TFA, 51.9 mg, 86% yield) with 95% e.e. [DAICEL CHIRALPAK IG column, Agilent HPLC 1260, iPrOH/n-hexane = 10/90 (v/v) with 0.1% TFA, 1.0 mL/min, 25 °C, 210 nm; t₁ = 19.6 min, t₂ = 31.1 min]: [α]_{D}²⁵ = -121.3 (c 1.0, MeOH, 95% e.e.); IR (film) *v*ₘₐₓ: 1724, 1690, 1506, 1342, 1039, 730 cm⁻¹; ¹H NMR (300 MHz, CD₃OD) δ 4.79 (d, *J* = 12.2 Hz, 1H), 4.81 (d, *J* = 12.2 Hz, 1H), 5.35 (s, 1H), 7.28-7.36 (m, 1H), 7.37-7.46 (m, 2H), 7.47-7.54 (m, 2H), 7.56-7.66 (m, 4H); ¹³C NMR (75 MHz, CD₃OD) δ 59.5, 75.7, 97.0, 128.0, 128.3, 128.5, 129.1, 129.9, 137.1, 141.8, 142.6, 156.2, 173.5; HRMS calcd for [C₁₇H₁₄Cl₃NO₄Na]⁺ [(M + Na)⁺]: 423.9881; found: 423.9880.

### (R)-2-(4-Methoxyphenyl)-2-(((2,2,2-trichloroethoxy)carbonyl)amino)acetic acid (5)

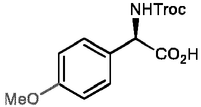

Catalyzed by 1 mol% Λ-**RuDMP**, the 1,3-nitrogen shift of 2,2,2-trichloroethyl (2-(4-methoxyphenyl)acetoxy)carbamate (53.5 mg, 0.15 mmol) gave **5** as a white solid (chromatography on silica gel, eluent: EtOAc/n-hexane = 1/3 with 0.1% TFA, 51.6 mg, 96% yield) with 96% e.e. [DAICEL CHIRALPAK IG column, Agilent HPLC 1260, iPrOH/n-hexane = 20/80 (v/v) with 0.1% TFA, 1.0 mL/min, 25 °C, 210 nm; t₁ = 11.4 min, t₂ = 14.9 min]: [α]_{D}²⁵ = -112.9 (c 1.0, MeOH, 96% e.e.); IR (film) *v*ₘₐₓ: 1721, 1506, 1246, 810 cm⁻¹; ¹H NMR (300 MHz, CD₃OD) δ 3.78 (s, 3H), 4.77 (d, *J* = 12.2 Hz, 1H), 4.79 (d, *J* = 12.2 Hz, 1H), 5.21 (s, 1H), 6.87-6.95 (m, 2H), 7.30-7.38 (m, 2H); ¹³C NMR (75 MHz, CD₃OD) δ 55.8, 59.2, 75.6, 97.0, 115.1, 129.9 (2C), 156.2, 161.2, 173.9; HRMS calcd for [C₁₂H₁₂Cl₃NO₅Na]⁺ [(M + Na)⁺]: 377.9673; found: 377.9683.

### (R)-2-(3-Methoxyphenyl)-2-(((2,2,2-trichloroethoxy)carbonyl)amino)acetic acid (6)

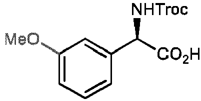

Catalyzed by 1 mol% Λ-**RuDMP**, the 1,3-nitrogen shift of 2,2,2-trichloroethyl (2-(3-methoxyphenyl)acetoxy)carbamate (53.5 mg, 0.15 mmol) gave **6** as a white solid (chromatography on silica gel, eluent: EtOAc/n-hexane = 1/4 with 0.1% TFA, 46.4 mg, 87% yield) with 96% e.e. [DAICEL CHIRALPAK IG column, Agilent HPLC 1260, iPrOH/n-hexane = 20/80 (v/v) with 0.1% TFA, 1.0 mL/min, 25 °C, 210 nm; t₁ = 9.1 min, t₂ = 12.6 min]: [α]_{D}²⁵ = -111.7 (*c* 1.0, MeOH, 96% e.e.); IR (film) vₘₐₓ: 1724, 1498, 1190, 1034, 808, 707 cm⁻¹; ¹HNMR (300 MHz, CD₃OD) δ 3.79 (s, 3H), 4.78 (d, *J =* 12.2 Hz, 1H), 4.80 (d, *J* = 12.2 Hz, 1H), 5.27 (s, 1H), 6.88 (ddd, *J* = 8.2, 2.4, 0.9 Hz, 1H), 6.96-7.05 (m, 2H), 7.27 (t, *J* = 8.2 Hz, 1H); ¹³C NMR (75 MHz, CD₃OD) δ 55.7, 59.7, 75.6, 97.0, 114.2, 115.0, 120.8, 130.8, 139.4, 156.2, 161.4, 173.5; HRMS calcd for [C₁₂H₁₂Cl₃NO₅Na]⁺ [(M + Na)⁺]: 377.9673; found: 377.9672.

### (R)-2-(2-Methoxyphenyl)-2-(((2,2,2-trichloroethoxy)carbonyl)amino)acetic acid (7)

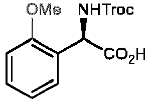

Catalyzed by 1 mol% A-**RuDMP**, the 1,3-nitrogen shift of 2,2,2-trichloroethyl (2-(2-methoxyphenyl)acetoxy)carbamate (53.5 mg, 0.15 mmol) gave 7 as a white solid (chromatography on silica gel, eluent: EtOAc/n-hexane = 1/3 with 0.1% TFA, 42.0 mg, 79% yield) with 85% e.e. [DAICEL CHIRALPAK IG column, Agilent HPLC 1260, iPrOH/n-hexane = 20/80 (v/v) with 0.1% TFA, 1.0 mL/min, 25 °C, 210 nm; t₁ = 10.6 min, t₂ = 14.0 min]: [α]_{D}²⁵ = -92.8 (c 1.0, MeOH, 85% e.e.); IR (film) vₘₐₓ: 1745, 1693, 1456, 1156, 1024, 727 cm⁻¹; ¹H NMR (300 MHz, CD₃OD) δ 3.84 (s, 3H), 4.77 (d, *J =* 12.2 Hz, 1H), 4.79 (d, *J =* 12.2 Hz, 1H), 5.56 (s, 1H), 6.88-6.97 (m, 1H), 6.97-7.04 (m, 1H), 7.25-7.38 (m, 2H); ¹³C NMR (75 MHz, CD₃OD) δ 55.1, 56.1, 75.6, 97.0, 112.3, 121.7, 126.6, 130.4, 130.9, 156.3, 158.5, 174.1; HRMS calcd for [C₁₂H₁₂Cl₃NO₅Na]⁺ [(M + Na)⁺]: 377.9673; found: 377.9683.

### (R)-2-(Benzo[d][1,3]dioxol-5-yl)-2-(((2,2,2-trichloroethoxy)carbonyl)amino)acetic acid (8)

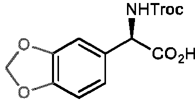

Catalyzed by 1 mol% Λ-**RuDMP**, the 1,3-nitrogen shift of 2,2,2-trichloroethyl (2-(benzo[d][1,3]dioxol-5-yl)acetoxy)carbamate (37.1 mg, 0.1 mmol) gave **8** as a white solid (chromatography on silica gel, eluent: EtOAc/*n*-hexane = 1/4 with 0.1% TFA, 20.6 mg, 56% yield) with 92% e.e. [DAICEL CHIRALPAK IG column, Agilent HPLC 1260, *i*PrOH/n-hexane = 20/80 (v/v) with 0.1% TFA, 1.0 mL/min, 25 °C, 210 nm; t₁ = 11.5 min, t₂ = 14.6 min]: [α]_{D}²⁵ = -104.0 (c 0.5, MeOH, 92% e.e.); IR (film) *v*ₘₐₓ: 1723, 1245, 1171, 1039, 815 cm⁻¹; ¹H NMR (300 MHz, CD₃OD) δ 4.77 (d, *J* = 12.3 Hz, 1H), 4.79 (d, *J* = 12.3 Hz, 1H), 5.19 (s, 1H), 5.95 (s, 2H), 6.76-6.82 (m, 1H), 6.87-6.93 (m, 2H); ¹³C NMR (75 MHz, CD₃OD) δ 59.4, 75.6, 97.0, 102.6, 108.9, 109.2, 122.4, 131.8, 149.2, 149.4, 156.1, 173.6; HRMS calcd for [C₁₂H₁₀Cl₃N0₆Na]⁺ [(M + Na)⁺]: 391.9466; found: 391.9465.

### (R)-2-(4-((((9H-Fluoren-9-yl)methoxy)carbonyl)oxy)phenyl)-2-(((2,2,2-trichloroethoxy)carbonyl)amino)acetic acid (9)

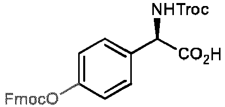

Catalyzed by 1 mol% Λ-**RuDMP**, the 1,3-nitrogen shift of 2,2,2-trichloroethyl (2-(4-((((9*H*-fluoren-9-yl)methoxy)carbonyl)oxy)phenyl)acetoxy)carbamate (56.5 mg, 0.1 mmol) gave **9** as a white solid (chromatography on silica gel, eluent: EtOAc/n-hexane = 1/3 with 0.1% TFA, 47.1 mg, 83% yield) with 93% e.e. [DAICEL CHIRALPAK IG column, Agilent HPLC 1260, *i*PrOH/n-hexane = 20/80 (v/v) with 0.1% TFA, 1.0 mL/min, 25 °C, 210 nm; t₁ = 17.6 min, t₂ = 21.1 min]: [α]_{D}²⁵ = -57.0 (*c* 1.0, MeOH, 93% e.e.); IR (film) *v*ₘₐₓ: 1749, 1715, 1255, 1216, 733 cm⁻¹; ¹H NMR (300 MHz, CD₃OD) δ 4.27 (t, *J* = 6.7 Hz, 1H), 4.55 (d, *J =* 6.7 Hz, 2H), 4.77 (d, *J =* 12.3 Hz, 1H), 4.79 (d, *J =* 12.3 Hz, 1H), 5.33 (s, 1H), 7.07-7.16 (m, 2H), 7.26-7.36 (m, 2H), 7.36-7.43 (m, 2H), 7.43-7.50 (m, 2H), 7.56-7.65 (m, 2H), 7.75-7.84 (m, 2H); ¹³C NMR (75 MHz, CD₃OD) δ 48.0, 59.1, 71.4, 75.7, 96.9, 121.1, 122.4, 126.1, 128.2, 129.0, 129.9, 136.1, 142.7, 144.6, 152.5, 154.8, 156.2, 173.2; HRMS calcd for [C₂₆H₂₀Cl₃NO₇Na]⁺ [(M + Na)⁺]: 586.0198; found: 586.0194.

### Methyl (R)-2-(4-(hydroxymethyl)phenyl)-2-(((2,2,2-trichloroethoxy)carbonyl)amino)acetate (10)

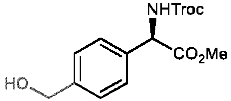

Catalyzed by 1 mol% Λ-**RuDMP**, the 1,3-nitrogen shift of 2,2,2-trichloroethyl (2-(4-(hydroxymethyl)phenyl)acetoxy)carbamate (53.5 mg, 0.15 mmol) and the methylation by trimethylsilyldiazomethane gave **10** as a colorless oil (chromatography on silica gel, eluent: EtOAc/n-hexane = 1/1, 35.7 mg, 64% yield for two steps) with 98% e.e. [DAICEL CHIRALCEL OD-H column, Agilent HPLC 1260, iPrOH/n-hexane = 10/90 (v/v), 1.0 mL/min, 25 °C, 210 nm; t₁ = 23.5 min, t₂ = 25.3 min]: [α]_{D}²⁵ = -98.3 (c 1.0, MeOH, 98% e.e.); IR (film) *v*ₘₐₓ: 3411, 1725, 1511, 1218, 1034, 818, 709 cm⁻¹; ¹H NMR (300 MHz, CDCl₃) δ 1.80-2.00 (br, 1H), 3.74 (s, 3H), 4.68 (s, 2H), 4.69 (d, *J* = 12.0 Hz, 1H), 4.71 (d, *J* = 12.0 Hz, 1H), 5.37 (d, *J* = 7.2 Hz, 1H), 6.18 (d, *J* = 7.2 Hz, 1H), 7.28-7.45 (m, 4H); ¹³C NMR (75 MHz, CDCl₃) δ 53.1, 58.0, 64.9, 74.9, 95.4, 127.5, 127.7, 135.6, 141.7, 153.7, 171.0; HRMS calcd for [C₁₃H₁₄Cl₃NO₅Na]⁺ [(M + Na)⁺]: 391.9830; found: 391.9834.

### (R)-2-(4-Iodophenyl)-2-(((2,2,2-trichloroethoxy)carbonyl)amino)acetic acid (11)

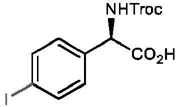

Catalyzed by 1 mol% Λ-**RuDMP**, the 1,3-nitrogen shift of 2,2,2-trichloroethyl (2-(4-iodophenyl)acetoxy)carbamate (67.9 mg, 0.15 mmol) gave **11** as a white solid (chromatography on silica gel, eluent: EtOAc/n-hexane = 1/5 with 0.1% TFA, 62.0 mg, 91% yield) with 94% e.e. [DAICEL CHIRALPAK IG column, Agilent HPLC 1260, iPrOH/n-hexane = 10/90 (v/v) with 0.1% TFA, 1.0 mL/min, 25 °C, 210 nm; t₁ = 14.4 min, t₂ = 18.3 min]: [α]_{D}²⁵ = -87.7 (c 1.0, MeOH, 94% e.e.); IR (film) *v*ₘₐₓ: 1724, 1508, 1207, 1035, 816 cm⁻¹; ¹H NMR (300 MHz, CD₃OD) δ 4.77 (d, *J* = 12.3 Hz, 1H), 4.79 (d, *J* = 12.3 Hz, 1H), 5.27 (s, 1H), 7.13-7.27 (m, 2H), 7.62-7.78 (m, 2H); ¹³C NMR (75 MHz, CD₃OD) δ 59.2, 75.6, 94.6, 96.9, 130.6, 138.0, 138.9, 156.1, 172.9; HRMS calcd for [C₁₁H₉Cl₃INO₄Na]⁺ [(M + Na)⁺]: 473.8534; found: 473.8548.

### (R)-2-(4-Bromophenyl)-2-(((2,2,2-trichloroethoxy)carbonyl)amino)acetic acid (12)

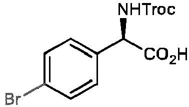

Catalyzed by 1 mol% Λ-**RuDMP**, the 1,3-nitrogen shift of 2,2,2-trichloroethyl (2-(4-bromophenyl)acetoxy)carbamate (60.8 mg, 0.15 mmol) gave **12** as a white solid (chromatography on silica gel, eluent: EtOAc/n-hexane = 1/5 with 0.1% TFA, 56.6 mg, 93% yield) with 94% e.e. [DAICEL CHIRALPAK IG column, Agilent HPLC 1260, *i*PrOH/n-hexane = 10/90 (v/v) with 0.1% TFA, 1.0 mL/min, 25 °C, 210 nm; t₁ = 13.2 min, t₂ = 16.3 min]: [α]_{D}²⁵ = -91.0 (c 1.0, MeOH, 94% e.e.); IR (film) *v*ₘₐₓ: 1718, 1217, 1040, 814 cm⁻¹; ¹H NMR (300 MHz, CD₃OD) δ 4.78 (d, *J* = 12.2 Hz, 1H), 4.80 (d, *J* = 12.2 Hz, 1H), 5.29 (s, 1H), 7.30-7.41 (m, 2H), 7.48-7.57 (m, 2H); ¹³C NMR (75 MHz, CD₃OD) δ 59.1, 75.7, 96.9, 123.2, 130.6, 132.8, 137.5, 156.1, 172.9; HRMS calcd for [C₁₁H₉BrCl₃NO₄Na]⁺ [(M + Na)⁺]: 425.8673; found: 425.8671.

### (R)-2-(4-Chlorophenyl)-2-(((2,2,2-trichloroethoxy)carbonyl)amino)acetic acid (13)

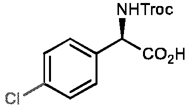

Catalyzed by 1 mol% Λ-**RuDMP**, the 1,3-nitrogen shift of 2,2,2-trichloroethyl (2-(4-chlorophenyl)acetoxy)carbamate (54.2 mg, 0.15 mmol) gave **13** as a white solid (chromatography on silica gel, eluent: EtOAc/n-hexane = 1/5 with 0.1% TFA, 50.3 mg, 93% yield) with 95% e.e. [DAICEL CHIRALPAK IG column, Agilent HPLC 1260, iPrOH/n-hexane = 10/90 (v/v) with 0.1% TFA, 1.0 mL/min, 25 °C, 210 nm; t₁ = 12.5 min, t₂ = 15.1 min]: [α]_{D}²⁵ = -93.4 (c 1.0, MeOH, 95% e.e.); IR (film) *v*ₘₐₓ: 1714, 1495, 1214, 1090, 1042, 817, 714 cm⁻¹; ¹H NMR (300 MHz, CD₃OD) δ 4.78 (d, *J* = 12.2 Hz, 1H), 4.80 (d, *J*= 12.2 Hz, 1H), 5.30 (s, 1H), 7.31-7.40 (m, 2H), 7.40-7.49 (m, 2H); ¹³C NMR (75 MHz, CD₃OD) δ 59.0, 75.6, 96.9, 129.8, 130.3, 135.2, 137.0, 156.1, 173.0; HRMS calcd for [C₁₁H₉Cl₄NO₄Na]⁺ [(M + Na)⁺]: 381.9148; found: 381.9151.

### (R)-2-(4-Fluorophenyl)-2-(((2,2,2-trichloroethoxy)carbonyl)amino)acetic acid (14)

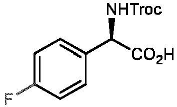

Catalyzed by 1 mol% Λ-**RuDMP**, the 1,3-nitrogen shift of 2,2,2-trichloroethyl (2-(4-fluorophenyl)acetoxy)carbamate (34.5 mg, 0.1 mmol) gave **14** as a white solid (chromatography on silica gel, eluent: EtOAc/*n*-hexane = 1/4 with 0.1% TFA, 31.6 mg, 92% yield) with 93% e.e. [DAICEL CHIRALPAK IG column, Agilent HPLC 1260, iPrOH/n-hexane = 10/90 (v/v) with 0.1% TFA, 1.0 mL/min, 25 °C, 210 nm; t₁ = 12.4 min, t₂ = 14.7 min]: [α]_{D}²⁵ = -99.0 (c 1.0, MeOH, 93% e.e.); IR (film) *v*ₘₐₓ: 1724, 1505, 1226, 1160, 1040, 811 cm⁻¹; ¹H NMR (300 MHz, CD₃OD) δ 4.78 (d, *J* = 12.2 Hz, 1H), 4.80 (d, *J* = 12.2 Hz, 1H), 5.30 (s, 1H), 7.02-7.15 (m, 2H), 7.38-7.52 (m, 2H); ¹³C NMR (75 MHz, CD₃OD) δ 59.0, 75.6, 96.9, 116.4 (d, *J* = 21.9 Hz), 130.7 (d, *J =* 8.4 Hz), 134.2 (d, *J =* 3.0 Hz), 156.1, 164.1 (d, *J* = 245.5 Hz), 173.3; ¹⁹F NMR (282 MHz, CD₃OD) δ -114.3; HRMS calcd for [C₁₁H₉Cl₃FNO₄Na]⁺ [(M + Na)⁺]: 365.9473; found: 365.9476.

### (R)-2-(((2,2,2-Trichloroethoxy)carbonyl)amino)-2-(4-(trifluoromethyl)phenyl)acetic acid (15)

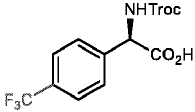

Catalyzed by 1 mol% Λ-**RuDMP**, the 1,3-nitrogen shift of 2,2,2-trichloroethyl (2-(4-(trifluoromethyl)phenyl)acetoxy)carbamate (59.2 mg, 0.15 mmol) gave **15** as a white solid (chromatography on silica gel, eluent: EtOAc/n-hexane = 1/4 with 0.1% TFA, 51.6 mg, 87% yield) with 86% e.e. [DAICEL CHIRALPAK IG column, Agilent HPLC 1260, *i*PrOH/n-hexane = 10/90 (v/v) with 0.1% TFA, 1.0 mL/min, 25 °C, 210 nm; t₁ = 8.0 min, t₂ = 9.1 min]: [α]_{D}²⁵ = -88.3 (c 1.0, MeOH, 86% e.e.); IR (film) *v*ₘₐₓ: 1726, 1696, 1541, 1324, 1161, 1121, 814 cm⁻¹; ¹H NMR (300 MHz, CD₃OD) δ 4.79 (d, *J =* 12.2 Hz, 1H), 4.81 (d, *J* = 12.2 Hz, 1H), 5.42 (s, 1H), 7.60-7.65 (m, 2H), 7.65-7.71 (m, 2H); ¹³C NMR (75 MHz, CD₃OD) δ 59.3, 75.7, 96.9, 125.5 (q, *J* = 271.1 Hz), 126.6 (q, *J* = 3.8 Hz), 129.4, 131.4 (q, *J* = 32.4 Hz), 142.7, 156.2, 172.6; ¹⁹F NMR (282 MHz, CD₃OD) δ -62.5; HRMS calcd for [C₁₂H₉Cl₃F₃NO₄Na]⁺ [(M + Na)⁺]: 415.9441; found: 415.9446.

### (R)-2-(4-Nitrophenyl)-2-(((2,2,2-trichloroethoxy)carbonyl)amino)acetic acid (16)

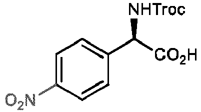

Catalyzed by 2 mol% Λ-**RuDMP**, the 1,3-nitrogen shift of 2,2,2-trichloroethyl (2-(4-nitrophenyl)acetoxy)carbamate (37.2 mg, 0.1 mmol) gave **16** as a white solid (chromatography on silica gel, eluent: EtOAc/n-hexane = 1/3 with 0.1% TFA, 27.3 mg, 73% yield) with 92% e.e. [DAICEL CHIRALPAK IG column, Agilent HPLC 1260, *i*PrOH/n-hexane = 10/90 (v/v) with 0.1% TFA, 1.0 mL/min, 25 °C, 210 nm; t₁ = 21.1 min, t₂ = 25.3 min]: [α]_{D}²⁵ = -132.5 (*c* 1.0, MeOH, 92% e.e.); IR (film) *v*ₘₐₓ: 1716, 1519, 1344, 698 cm⁻¹; ¹H NMR(300 MHz, CD₃OD) δ 4.79 (d, *J* = 12.2 Hz, 1H), 4.81 (d, *J* = 12.2 Hz, 1H), 5.48 (s, 1H), 7.64-7.77 (m, 2H), 8.21-8.31 (m, 2H); ¹³C NMR (75 MHz, CD₃OD) δ 59.2, 75.7, 96.9, 124.7, 129.9, 145.6, 149.2, 156.2, 172.1; HRMS calcd for [C₁₁H₉Cl₃N₂O₆Na]⁺ [(M + Na)⁺]: 392.9418; found: 392.9423.

### (R)-2-(4-Acetylphenyl)-2-(((2,2,2-trichloroethoxy)carbonyl)amino)acetic acid (17)

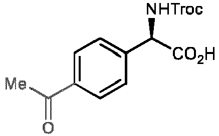

Catalyzed by 1 mol% Λ-**RuDMP**, the 1,3-nitrogen shift of 2,2,2-trichloroethyl (2-(4-acetylphenyl)acetoxy)carbamate (55.3 mg, 0.15 mmol) gave **17** as a white solid (chromatography on silica gel, eluent: EtOAc/n-hexane = 1/3 with 0.1% TFA, 43.8 mg, 79% yield) with 92% e.e. [DAICEL CHIRALPAK IG column, Agilent HPLC 1260, *i*PrOH/*n*-hexane = 20/80 (v/v) with 0.1% TFA, 1.0 mL/min, 25 °C, 210 nm; t₁ = 14.6 min, t₂ = 18.1 min]: [α]_{D}²⁵ = -137.3 (c 1.0, MeOH, 92% e.e.); IR (film) *v*ₘₐₓ: 1718, 1676, 1415, 1266, 1039, 816 cm⁻¹; ¹H NMR (300 MHz, CD₃OD, as a mixture of **17** and the corresponding hemiacetal formed by **17** and CD₃OD, the ratio of **17/17**•CD₃OD is 3.4/1) δ 2.60 (s, 3H), 4.78 (d, *J* = 12.2 Hz, 1H), 4.80 (d, *J=* 12.2 Hz, 1H), 5.40 (s, 1H), 7.54-7.62 (m, 2H), 7.94-8.03 (m, 2H); ¹³C NMR (75 MHz, CD₃OD, as a mixture of **17** and the corresponding hemiacetal formed by **17** and CD₃OD) δ 26.7, 59.4, 75.7, 96.9, 129.0, 129.8, 138.2, 143.6, 156.2, 172.7, 200.0; HRMS calcd for [C₁₃H₁₂Cl₃NO₅Na]⁺ [(M + Na)⁺]: 389.9673; found: 389.9672.

### (R)-2-(4-(Methoxycarbonyl)phenyl)-2-(((2,2,2-trichloroethoxy)carbonyl)amino)acetic acid (18)

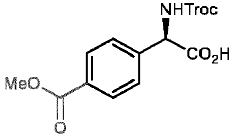

Catalyzed by 1 mol% Λ-**RuDMP**, the 1,3-nitrogen shift of methyl 4-(2-oxo-2-((((2,2,2-trichloroethoxy)carbonyl)amino)oxy)ethyl)benzoate (57.7 mg, 0.15 mmol) gave **18** as a white solid (chromatography on silica gel, eluent: EtOAc/n-hexane = 1/3 with 0.1% TFA, 52.4 mg, 91% yield) with 93% e.e. [DAICEL CHIRALPAK IG column, Agilent HPLC 1260, iPrOH/n-hexane = 20/80 (v/v) with 0.1% TFA, 1.0 mL/min, 25 °C, 210 nm; t₁ = 11.5 min, t₂ = 13.8 min]: [α]_{D}²⁵ = -103.9 (c 1.0, MeOH, 93% e.e.); IR (film) *v*ₘₐₓ: 1728, 1674, 1437, 1292, 766 cm⁻¹; ¹H NMR (300 MHz, CD₃OD) δ 3.89 (s, 3H), 4.78 (d, *J* = 12.3 Hz, 1H), 4.80 (d, *J =* 12.3 Hz, 1H), 5.40 (s, 1H), 7.51-7.61 (m, 2H), 7.94-8.06 (m, 2H); ¹³C NMR (75 MHz, CD₃OD) δ 52.7, 59.4, 75.7, 96.9, 128.8, 130.8, 131.2, 143.5, 156.2, 168.1, 172.7; HRMS calcd for [C₁₃H₁₂Cl₃NO₆Na]⁺ [(M + Na)⁺]: 405.9622; found: 405.9624.

### (R)-2-(4-Pivalamidophenyl)-2-(((2,2,2-trichloroethoxy)carbonyl)amino)acetic acid (19)

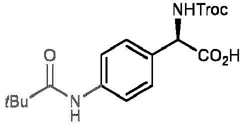

Catalyzed by 1 mol% Λ-**RuDMP**, the 1,3-nitrogen shift of 2,2,2-trichloroethyl (2-(4-pivalamidophenyl)acetoxy)carbamate (63.9 mg, 0.15 mmol) gave **19** as a white solid (chromatography on silica gel, eluent: EtOAc/n-hexane = 1/2 with 0.1% TFA, 59.7 mg, 93% yield) with 96% e.e. [DAICEL CHIRALPAK IA column, Agilent HPLC 1260, iPrOH/n-hexane = 20/80 (v/v) with 0.1% TFA, 1.0 mL/min, 25 °C, 210 nm; t₁ = 11.6 min, t₂ = 15.7 min]: [α]_{D}²⁵ = -135.7 (c 1.0, MeOH, 96% e.e.); IR (film) *v*ₘₐₓ: 1728, 1696, 1653, 1424, 1374, 1337, 1152, 821 cm⁻¹; ¹H NMR (300 MHz, CD₃OD) δ 1.29 (s, 9H), 4.77 (d, *J* = 12.2 Hz 1H), 4.80 (d, *J* = 12.2 Hz 1H), 5.26 (s, 1H), 7.31-7.42 (m, 2H), 7.48-7.60 (m, 2H); ¹³C NMR (75 MHz, CD₃OD) δ 27.7, 40.5, 59.3, 75.6, 96.9, 122.7, 129.0, 133.8, 139.9, 156.2, 173.6, 179.9; HRMS calcd for [C₁₆H₁₉Cl₃N₂O₅Na]⁺ [(M + Na)⁺]: 447.0252; found: 447.0255.

### (R)-2-(4-Azidophenyl)-2-(((2,2,2-trichloroethoxy)carbonyl)amino)acetic acid (20)

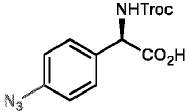

Catalyzed by 1 mol% Λ-**RuDMP**, the 1,3-nitrogen shift of 2,2,2-trichloroethyl (2-(4-azidophenyl)acetoxy)carbamate (55.1 mg, 0.15 mmol) gave **20** as a white solid (chromatography on silica gel, eluent: EtOAc/n-hexane = 1/4 with 0.1% TFA, 52.1 mg, 95% yield) with 97% e.e. [DAICEL CHIRALPAK IG column, Agilent HPLC 1260, iPrOH/n-hexane = 10/90 (v/v) with 0.1% TFA, 1.0 mL/min, 25 °C, 210 nm; t₁ = 17.9 min, t₂ = 23.3 min]: [α]_{D}²⁵ = -118.4 (c 1.0, MeOH, 97% e.e.); IR (film) *v*ₘₐₓ: 2122, 2096, 1713, 1502, 1284, 1040, 812, 717 cm⁻¹; ¹H NMR (300 MHz, CD₃OD) δ 4.77 (d, *J =* 12.3 Hz, 1H), 4.79 (d, *J =* 12.3 Hz, 1H), 5.29 (s, 1H), 7.01-7.11 (m, 2H), 7.40-7.50 (m, 2H); ¹³C NMR (75 MHz, CD₃OD) δ 59.1, 75.6, 96.9, 120.2, 130.3, 135.0, 141.6, 156.1, 173.3; HRMS calcd for [C₁₁H₉Cl₃N₄O₄Na]⁺ [(M + Na)⁺]: 388.9582; found: 388.9582.

### (R)-2-(4-Ethynylphenyl)-2-(((2,2,2-trichloroethoxy)carbonyl)amino)acetic acid (21)

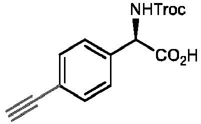

Catalyzed by 1 mol% Λ-**RuDMP**, the 1,3-nitrogen shift of 2,2,2-trichloroethyl (2-(4-ethynylphenyl)acetoxy)carbamate (52.6 mg, 0.15 mmol) gave **21** as a white solid (chromatography on silica gel, eluent: EtOAc/n-hexane = 1/5 with 0.1% TFA, 46.4 mg, 88% yield) with 94% e.e. [DAICEL CHIRALPAK IG column, Agilent HPLC 1260, iPrOH/n-hexane = 10/90 (v/v) with 0.1% TFA, 1.0 mL/min, 25 °C, 210 nm; t₁ = 15.2 min, t₂= 19.2 min]: [α]_{D}²⁵ = -127.7 (c 1.0, MeOH, 94% e.e.); IR (film) *v*ₘₐₓ: 3292, 1713, 1504, 1215, 1042, 717 cm⁻¹; ¹H NMR (300 MHz, CD₃OD) δ 3.50 (s, 1H), 4.78 (d, *J=* 12.3 Hz, 1H), 4.80 (d, *J=* 12.3 Hz, 1H), 5.32 (s, 1H), 7.37-7.44 (m, 2H), 7.44-7.51 (m, 2H); ¹³C NMR (75 MHz, CD₃OD) δ 59.4, 75.7, 79.3, 83.9, 96.9, 123.8, 128.8, 133.3, 138.8, 156.2, 173.0; HRMS calcd for [C₁₃H₁₀Cl₃NO₄Na]⁺ [(M + Na)⁺]: 371.9568; found: 371.9571.

### (R)-2-(Naphthalen-2-yl)-2-(((2,2,2-trichloroethoxy)carbonyl)amino)acetic acid (22)

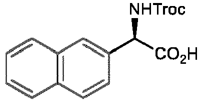

Catalyzed by 1 mol% Λ-**RuDMP**, the 1,3-nitrogen shift of 2,2,2-trichloroethyl (2-(naphthalen-2-yl)acetoxy)carbamate (56.5 mg, 0.15 mmol) gave **22** as a white solid (chromatography on silica gel, eluent: EtOAc/n-hexane = 1/4 with 0.1% TFA, 51.8 mg, 92% yield) with 96% e.e. [DAICEL CHIRALPAK IG column, Agilent HPLC 1260, iPrOH/n-hexane = 10/90 (v/v) with 0.1% TFA, 1.0 mL/min, 25 °C, 210 nm; t₁ = 15.8 min, t₂ = 23.0 min]: [α]_{D}²⁵ = -161.6 (c 1.0, MeOH, 96% e.e.); IR (film) *v*ₘₐₓ: 1724, 1693, 1504, 1182, 1039, 815, 736 cm⁻¹; ¹H NMR (300 MHz, CD₃OD) δ 4.79 (d, *J* = 12.3 Hz, 1H), 4.80 (d, *J* = 12.3 Hz, 1H), 5.49 (s, 1H), 7.43-7.51 (m, 2H), 7.51-7.59 (m, 1H), 7.79-7.89 (m, 3H), 7.89-7.95 (m, 1H); ¹³C NMR (75 MHz, CD₃OD) δ 59.9, 75.6, 97.0, 126.2, 127.4 (2C), 127.8, 128.7, 129.0, 129.5, 134.6, 134.7, 135.4, 156.2, 173.5; HRMS calcd for [C₁₅H₁₂Cl₃NO₄Na]⁺ [(M + Na)⁺]: 397.9724; found: 397.9724.

### (R)-2-(Naphthalen-1-yl)-2-(((2,2,2-trichloroethoxy)carbonyl)amino)acetic acid (23)

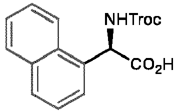

Catalyzed by 1 mol% Λ-**RuDMP**, the 1,3-nitrogen shift of 2,2,2-trichloroethyl (2-(naphthalen-1-yl)acetoxy)carbamate (56.5 mg, 0.15 mmol) gave **23** as a white solid (chromatography on silica gel, eluent: EtOAc/*n*-hexane = 1/4 with 0.1% TFA, 52.2 mg, 92% yield) with 95% e.e. [DAICEL CHIRALPAK IG column, Agilent HPLC 1260, *i*PrOH/*n*-hexane = 20/80 (v/v) with 0.1% TFA, 1.0 mL/min, 25 °C, 210 nm; t₁ = 9.0 min, t₂ = 14.1 min]: [α]_{D}²⁵ = -125.0 (c 1.0, MeOH, 95% e.e.); IR (film) *v*ₘₐₓ: 1710, 1413, 1338, 1039, 770 cm⁻¹; ¹H NMR (300 MHz, CD₃OD) δ 4.77 (d, *J* = 12.2 Hz, 1H), 4.83 (d, *J* = 12.2 Hz, 1H), 6.11 (s, 1H), 7.41-7.60 (m, 4H), 7.82-7.94 (m, 2H), 8.12-8.22 (m, 1H); ¹³C NMR (75 MHz, CD₃OD) δ 56.4, 75.6, 97.0, 124.4, 126.3, 126.8, 127.0, 127.7, 129.9, 130.2, 132.5, 133.9, 135.5, 156.4, 174.1; HRMS calcd for [C₁₅H₁₂Cl₃NO₄Na]⁺ [(M + Na)⁺]: 397.9724; found: 397.9733.

### (R)-2-(Thiophen-3-yl)-2-(((2,2,2-trichloroethoxy)carbonyl)amino)acetic acid (24)

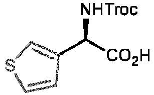

Catalyzed by 1 mol% Λ-**RuDMP**, the 1,3-nitrogen shift of 2,2,2-trichloroethyl (2-(thiophen-3-yl)acetoxy)carbamate (49.9 mg, 0.15 mmol) gave **24** as a white solid (chromatography on silica gel, eluent: EtOAc/n-hexane = 1/4 with 0.1% TFA, 42.1 mg, 84% yield) with 95% e.e. [DAICEL CHIRALPAK IG column, Agilent HPLC 1260, iPrOH/n-hexane = 20/80 (v/v) with 0.1% TFA, 1.0 mL/min, 25 °C, 210 nm; t₁ = 7.6 min, t₂ = 10.7 min]: [α]_{D}²⁵ = -75.3 (c 1.0, MeOH, 95% e.e.); IR (film) *v*ₘₐₓ: 1712, 1509, 1218, 1041, 690 cm⁻¹; ¹H NMR (300 MHz, CD₃OD) δ 4.78 (d, *J =* 12.2 Hz, 1H), 4.82 (d, *J =* 12.2 Hz, 1H), 5.42 (s, 1H), 7.16 (dd, *J =* 5.0, 1.4 Hz, 1H), 7.37-7.46 (m, 2H); ¹³C NMR (75 MHz, CD₃OD) δ 55.5, 75.6, 97.0, 124.3, 127.2, 127.8, 138.0, 156.3, 173.4; HRMS calcd for [C₉H₈Cl₃NO₄SNa]⁺ [(M + Na)⁺]: 353.9132; found: 353.9138.

### (R)-2-(1-(Ethoxycarbonyl)-1H indol-3-yl)-2-(((2,2,2-trichloroethoxy)carbonyl)amino)acetic acid (25)

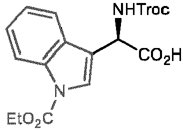

Catalyzed by 1 mol% Λ-**RuDMP**, the 1,3-nitrogen shift of ethyl 3-(2-oxo-2-((((2,2,2-trichloroethoxy)carbonyl)amino)oxy)ethyl)-1*H*-indole-1-carboxylate (65.7 mg, 0.15 mmol) gave **25** as a white solid (chromatography on silica gel, eluent: EtOAc/*n-*hexane = 1/3 with 0.1% TFA, 50.8 mg, 77% yield) with 90% e.e. [DAICEL CHIRALPAK IG column, Agilent HPLC 1260, *i*PrOH/*n*-hexane = 20/80 (v/v) with 0.1% TFA, 1.0 mL/min, 25 °C, 210 nm; t₁ = 10.2 min, t₂ = 11.7 min]: [α]_{D}²⁵ = -81.0 (*c* 1.0, MeOH, 90% e.e.); IR (film) *v*ₘₐₓ: 1724, 1245, 1088, 757 cm⁻¹; ¹H NMR (300 MHz, CD₃OD) δ 1.44 (t, *J* = 7.1 Hz, 3H), 4.46 (q, *J* = 7.1 Hz, 2H), 4.78 (d, *J* = 12.2 Hz, 1H), 4.84 (d, *J* = 12.2 Hz, 1H), 5.60 (s, 1H), 7.20-7.29 (m, 1H), 7.29-7.38 (m, 1H), 7.65-7.72 (m, 1H), 7.73 (s, 1H), 8.09-8.20 (m, 1H); ¹³C NMR (75 MHz, CD₃OD) δ 14.6, 52.2, 64.6, 75.6, 97.0, 116.1, 118.2, 120.7, 124.1, 125.4, 126.0, 129.8, 136.9, 152.1, 156.4, 173.1; HRMS calcd for [C₁₆H₁₅Cl₃N₂O₆Na]⁺ [(M + Na)⁺]: 458.9888; found: 458.9886.

### (R)-2-(11-Oxo-6,11-dihydrodibenzo[b,e]oxepin-2-yl)-2-(((2,2,2-trichloroethoxy)carbonyl)amino)acetic acid (26)

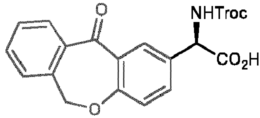

Catalyzed by 1 mol% Λ-**RuDMP**, the 1,3-nitrogen shift of 2,2,2-trichloroethyl (2-(11-oxo-6,11-dihydrodibenzo[*b*,*e*]oxepin-2-yl)acetoxy)carbamate (45.9 mg, 0.1 mmol) gave **26** as a white solid (chromatography on silica gel, eluent: EtOA*c*/*n*-hexane = 1/3 with 0.1% TFA, 39.9 mg, 87% yield) with 98% e.e. [DAICEL CHIRALPAK IG column, Agilent HPLC 1260, iPrOH/n-hexane = 20/80 (v/v) with 0.1% TFA, 1.0 mL/min, 25 °C, 210 nm; t₁ = 33.4 min, t₂ = 47.8 min]: [α]_{D}²⁵ = -120.9 (c 1.0, MeOH, 98% e.e.); IR (film) *v*ₘₐₓ: 1719, 1413, 1301, 1158, 762, 722 cm⁻¹; ¹H NMR (300 MHz, CD₃OD) δ 4.78 (d, *J* = 12.4 Hz, 1H), 4.80 (d, *J* = 12.4 Hz, 1H), 5.21 (s, 2H), 5.34 (s, 1H), 7.01-7.12 (m, 1H), 7.39-7.53 (m, 2H), 7.53-7.67 (m, 2H), 7.76-7.87 (m, 1H), 8.19-8.31 (m, 1H); ¹³C NMR (75 MHz, CD₃OD) δ 58.9, 74.6, 75.7, 96.9, 122.4, 126.4, 129.1, 130.2, 130.3, 131.8, 132.1, 134.2, 135.8, 137.4, 141.5, 156.2, 162.8, 173.2, 192.1; HRMS calcd for [C₁₉H₁₄Cl₃NO₆Na]⁺ [(M + Na)⁺]: 479.9779; found: 479.9774.

### Methyl (R)-2-(2-((2,6-dichlorophenyl)amino)phenyl)-2-(((2,2,2-trichloroethoxy)carbonyl)amino)acetate (27)

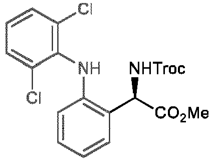

Catalyzed by 1 mol% Λ-**RuDMP**, the 1,3-nitrogen shift of 2,2,2-trichloroethyl (2-(2-((2,6-dichlorophenyl)amino)phenyl)acetoxy)carbamate (73.0 mg, 0.15 mmol) and methylation by trimethylsilyldiazomethane gave **27** as a colorless oil (chromatography on silica gel, eluent: EtOAc/n-hexane = 1/10, 40.5 mg, 54% yield for two steps) with 96% e.e. [DAICEL CHIRALPAK IA column, Agilent HPLC 1260, iPrOH/n-hexane = 10/90 (v/v), 1.0 mL/min, 25 °C, 210 nm; t₁ = 7.0 min, t₂ = 9.3 min]: [α]_{D}²⁵ = -89.8 (*c* 1.0, MeOH, 96% e.e.); IR (film) *v*ₘₐₓ: 3364, 1725, 1501, 1448, 1207, 1041, 721 cm⁻¹; ¹H NMR (300 MHz, CDCl₃) δ 3.82 (s, 3H), 4.73 (s, 2H), 5.80 (d, *J* = 6.8 Hz, 1H), 6.46-6.66 (m, 2H), 6.73-6.87 (br, 1H), 6.94-7.09 (m, 2H), 7.11-7.23 (m, 1H), 7.23-7.34 (m, 1H), 7.34-7.44 (m, 2H); ¹³C NMR (75 MHz, CDCl₃) δ 53.4, 55.2, 75.0, 95.3, 118.7, 122.6, 124.9, 125.5, 128.4, 129.0, 129.4, 130.2, 137.3, 142.2, 154.1, 171.6; HRMS calcd for [C₁₈H₁₅Cl₅N₂O₄Na]⁺ [(M + Na)⁺]: 522.9337; found: 522.9334.

### (R)-2-(((2,2,2-Trichloroethoxy)carbonyl)amino)but-3-enoic acid (28)

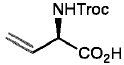

Catalyzed by 1 mol% Λ-**RuDMP**, the 1,3-nitrogen shift of 2,2,2-trichloroethyl (but-3-enoyloxy)carbamate (55.3 mg, 0.2 mmol) gave **28** as a white solid (chromatography on silica gel, eluent: EtOAc/*n*-hexane = 1/4 with 0.1% TFA, 34.5 mg, 62% yield) with 73% e.e. [DAICEL CHIRALPAK IG column, Agilent HPLC 1260, iPrOH/n-hexane = 10/90 (v/v) with 0.1% TFA, 1.0 mL/min, 25 °C, 210 nm; t₁ = 10.5 min, t₂ = 15.2 min]: [α]_{D}²⁵ = -8.7 (c 1.0, MeOH, 73% e.e.); IR (film) *v*ₘₐₓ: 1729, 1686, 1460, 1344, 1104, 726 cm⁻¹; ¹H NMR (300 MHz, CD₃OD) δ 4.76 (d, *J* = 12.2 Hz, 1H), 4.79-4.81 (m, 1H), 4.84 (d, *J* = 12.2 Hz, 1H), 5.27 (dt, *J* = 10.4, 1.1 Hz, 1H), 5.39 (ddd, *J=* 17.2, 1.5, 0.7 Hz, 1H), 6.01 (ddd, *J* = 17.2, 10.4, 5.8 Hz, 1H); ¹³C NMR (75 MHz, CD₃OD) δ 58.0, 75.6, 97.0, 118.0, 133.7, 156.4, 173.3; HRMS calcd for [C₇H₈Cl₃NO₄Na]⁺ [(M + Na)⁺]: 297.9411; found: 297.9414.

### (R,E)-2-(((2,2,2-Trichloroethoxy)carbonyl)amino)hex-3-enoic acid (29)

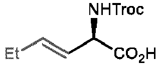

Catalyzed by 1 mol% Λ-**RuDMP**, the 1,3-nitrogen shift of 2,2,2-trichloroethyl (E)-(hex-3-enoyloxy)carbamate (45.7 mg, 0.15 mmol) gave **29** as a colorless oil (chromatography on silica gel, eluent: EtOAc/n-hexane = 1/5 with 0.1% TFA, 37.9 mg, 83% yield) with 84% e.e. [DAICEL CHIRALPAK IG column, Agilent HPLC 1260, iPrOH/n-hexane = 10/90 (v/v) with 0.1% TFA, 1.0 mL/min, 25 °C, 210 nm; t₁ = 8.4 min, t₂ = 11.4 min]: [α]_{D}²⁵ = -46.4 (c 1.0, MeOH, 84% e.e.); IR (film) *v*ₘₐₓ: 3319, 2965, 1705, 1523, 1244, 713 cm⁻¹; ¹H NMR (300 MHz, CD₃OD) δ 1.01 (t, *J* = 7.5 Hz, 3H), 2.00-2.19 (m, 2H), 4.64-4.71 (m, 1H), 4.75 (d, *J* = 12.2 Hz, 1H), 4.82 (d, *J* = 12.2 Hz, 1H), 5.56 (ddt, *J=* 15.5, 6.7, 1.4 Hz, 1H), 5.89 (dtd, *J* = 15.5, 6.4, 0.9 Hz, 1H); ¹³C NMR (75 MHz, CD₃OD) δ 13.5, 26.2, 57.6, 75.6, 97.0, 124.2, 137.6, 156.3, 174.1; HRMS calcd for [C₉H₁₂Cl₃NO₄Na]⁺ [(M + Na)⁺]: 325.9724; found: 325.9726.

### (R,E)-5-Methyl-2-(((2,2,2-trichloroethoxy)carbonyl)amino)hex-3-enoic acid (30)

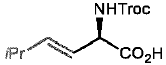

Catalyzed by 1 mol% Λ-**RuDMP**, the 1,3-nitrogen shift of 2,2,2-trichloroethyl (*E*)-((5-methylhex-3-enoyl)oxy)carbamate (47.8 mg, 0.15 mmol) gave **30** as a white solid (chromatography on silica gel, eluent: EtOAc/n-hexane = 1/5 with 0.1% TFA, 42.0 mg, 88% yield) with 81% e.e. [DAICEL CHIRALPAK IG column, Agilent HPLC 1260, *i*PrOH/*n*-hexane = 10/90 (v/v) with 0.1% TFA, 1.0 mL/min, 25 °C, 210 nm; t₁ = 7.5 min, t₂ = 8.7 min]: [α]_{D}²⁵ = -45.9 (c 1.0, MeOH, 81% e.e.); IR (film) *v*ₘₐₓ: 2962, 1731, 1692, 1464, 1342, 1187, 1097, 728 cm⁻¹; ¹H NMR (300 MHz, CD₃OD) δ 1.01 (d, *J* = 6.8 Hz, 6H), 2.23-2.41 (m, 1H), 4.65-4.71 (m, 1H), 4.74 (d, *J* = 12.2 Hz, 1H), 4.83 (d, *J* = 12.2 Hz, 1H), 5.53 (ddd, *J* = 15.5, 6.6, 1.2 Hz, 1H), 5.81 (ddd, *J* = 15.5, 6.7, 1.2 Hz, 1H); ¹³C NMR (75 MHz, CD₃OD) δ 22.4 (2C), 32.1, 57.6, 75.6, 97.0, 122.4, 142.8, 156.3, 174.1; HRMS calcd for [C₁₀H₁₄Cl₃NO₄Na]⁺ [(M + Na)⁺]: 339.9881; found: 339.9884.

### (R,E)-2-(((2,2,2-Trichloroethoxy)carbonyl)amino)dec-3-enoic acid (31)

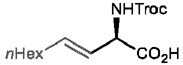

Catalyzed by 1 mol% Λ-**RuDMP**, the 1,3-nitrogen shift of 2,2,2-trichloroethyl (*E*)-(dec-3-enoyloxy)carbamate (54.1 mg, 0.15 mmol) gave **31** as a white solid (chromatography on silica gel, eluent: EtOAc/*n*-hexane = 1/6 with 0.1% TFA, 46.2 mg, 85% yield) with 82% e.e. [DAICEL CHIRALPAK IG column, Agilent HPLC 1260, iPrOH/n-hexane = 10/90 (v/v) with 0.1% TFA, 1.0 mL/min, 25 °C, 210 nm; t₁ = 7.4 min, t₂ = 11.6 min]: [α]_{D}²⁵ = -46.4 (c 1.0, MeOH, 82% e.e.); IR (film) *v*ₘₐₓ: 2956, 2924, 1723, 1698, 1529, 1195, 1102, 1052, 812, 711 cm⁻¹; ¹H NMR (300 MHz, CD₃OD) δ 0.82-0.96 (m, 3H), 1.21-1.48 (m, 8H), 2.00-2.15 (m, 2H), 4.64-4.71 (m, 1H), 4.74 (d, *J* = 12.2 Hz, 1H), 4.83 (d, *J* = 12.2 Hz, 1H), 5.56 (ddt, *J* = 15.4, 6.6, 1.2 Hz, 1H), 5.81 (dtd, *J* = 15.4, 6.8, 0.9 Hz, 1H); ¹³C NMR (75 MHz, CD₃OD) δ 14.4, 23.6, 29.8, 30.0, 32.8, 33.2, 57.6, 75.6, 97.0, 125.2, 136.2, 156.2, 174.0; HRMS calcd for [C₁₃H₂₀Cl₃NO₄Na]⁺ [(M + Na)⁺]: 382.0350; found: 382.0349.

### (R,E)-4-Phenyl-2-(((2,2,2-trichloroethoxy)carbonyl)amino)but-3-enoic acid (32)

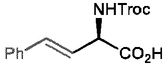

Catalyzed by 1 mol% Λ-**RuDMP**, the 1,3-nitrogen shift of 2,2,2-trichloroethyl (E)-((4-phenylbut-3-enoyl)oxy)carbamate (52.9 mg, 0.15 mmol) gave **32** as a yellow solid (chromatography on silica gel, eluent: EtOAc/n-hexane = 1/4 with 0.1% TFA, 46.8 mg, 88% yield) with 82% e.e. [DAICEL CHIRALPAK IG column, Agilent HPLC 1260, iPrOH/n-hexane = 10/90 (v/v) with 0.1% TFA, 1.0 mL/min, 25 °C, 210 nm; t₁ = 13.9 min, t₂ = 18.8 min]: [α]_{D}²⁵ = -84.3 (*c* 1.0, MeOH, 82% e.e.); IR (film) *v*ₘₐₓ: 1695, 1427, 1350, 1041, 720 cm⁻¹; ¹H NMR (300 MHz, CD₃OD) δ 4.78 (d, *J=* 12.2 Hz, 1H), 4.85 (d, *J* = 12.2 Hz, 1H), 4.94 (dd, *J* = 6.7, 0.8 Hz, 1H), 6.34 (dd, *J* = 16.0, 6.7 Hz, 1H), 6.73 (dd, *J* = 16.0, 0.8 Hz, 1H), 7.18-7.36 (m, 3H), 7.36-7.49 (m, 2H); ¹³C NMR (75 MHz, CD₃OD) δ 57.7, 75.6, 97.0, 124.5, 127.6, 129.1, 129.7, 134.2, 137.6, 156.4, 173.6; HRMS calcd for [C₁₃H₁₂Cl₃NO₄Na]⁺ [(M + Na)⁺]: 373.9724; found: 373.9726.

### (R,E)-2-(((2,2,2-Trichloroethoxy)carbonyl)amino)hexa-3,5-dienoic acid (33)

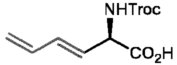

Catalyzed by 1 mol% Λ-**RuDMP**, the 1,3-nitrogen shift of 2,2,2-trichloroethyl (*E*)-(hexa-3,5-dienoyloxy)carbamate (45.4 mg, 0.15 mmol) gave **33** as a white solid (chromatography on silica gel, eluent: EtOAc/n-hexane = 1/5 with 0.1% TFA, 32.5 mg, 72% yield) with 81% e.e. [DAICEL CHIRALPAK IG column, Agilent HPLC 1260, *i*PrOH/*n*-hexane = 10/90 (v/v) with 0.1% TFA, 1.0 mL/min, 25 °C, 210 nm; t₁ = 9.4 min, t₂ = 14.1 min]: [α]_{D}²⁵ = -35.9 (c 1.0, MeOH, 81% e.e.); IR (film) *v*ₘₐₓ: 1711, 1515, 1208, 1098, 767 cm⁻¹; ¹H NMR (300 MHz, CD₃OD) δ 4.76 (d, *J* = 12.2 Hz, 1H), 4.78-4.81 (m, 1H), 4.83 (d, *J* = 12.2 Hz, 1H), 5.09-5.20 (m, 1H), 5.20-5.33 (m, 1H), 5.74-5.90 (m, 1H), 6.29-6.47 (m, 2H); ¹³C NMR (75 MHz, CD₃OD) δ 57.2, 75.6, 97.0, 119.0, 128.5, 134.9, 137.2, 156.3, 173.4; HRMS calcd for [C₉H₁₀Cl₃NO₄Na]⁺ [(M + Na)⁺]: 323.9568; found: 323.9572.

### (R)-4-Phenyl-2-(((2,2,2-trichloroethoxy)carbonyl)amino)but-3-ynoic acid (34)

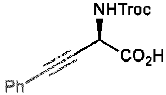

Catalyzed by 2 mol% Λ-**RuDMP** (3 equiv. of KHCO₃ was used as the base instead of K₂CO₃), the 1,3-nitrogen shift of 2,2,2-trichloroethyl ((4-phenylbut-3-ynoyl)oxy)carbamate (52.6 mg, 0.15 mmol) gave **34** as a yellow oil (chromatography on silica gel, eluent: EtOAc/*n*-hexane = 1/4 with 0.1% TFA, 24.3 mg, 46% yield) with 62% e.e. [DAICEL CHIRALPAK IG column, Agilent HPLC 1260, *i*PrOH/*n*-hexane = 10/90 (v/v) with 0.1% TFA, 1.0 mL/min, 25 °C, 210 nm; t₁ = 10.9 min, t₂ = 18.5 min]: [α]_{D}²⁵ = -31.5 (c 1.0, MeOH, 62% e.e.); IR (film) *v*ₘₐₓ: 1722, 1512, 1213, 1040, 719 cm⁻¹; ¹H NMR (300 MHz, CD₃OD) δ 4.79 (d, *J* = 12.3 Hz, 1H), 4.85 (d, *J=* 12.3 Hz, 1H), 5.26 (s, 1H), 7.26-7.39 (m, 3H), 7.40-7.52 (m, 2H); ¹³C NMR (75 MHz, CD₃OD) δ 47.8, 75.7, 83.6, 85.0, 96.9, 123.4, 129.5, 129.9, 132.8, 156.2, 170.5; HRMS calcd for [C₁₃H₁₀Cl₃NO₄Na]⁺ [(M + Na)⁺]: 371.9568; found: 371.9570.

### (R)-2-(((2,2,2-Trichloroethoxy)carbonyl)amino)hex-3-ynoic acid (35)

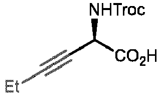

Catalyzed by 2 mol% Λ-**RuDMP** (3 equiv. of KHCO₃ was used as the base instead of K₂CO₃), the 1,3-nitrogen shift of 2,2,2-trichloroethyl (hex-3-ynoyloxy)carbamate (45.4 mg, 0.15 mmol) gave **35** as a colorless oil (chromatography on silica gel, eluent: EtOAc/*n*-hexane = 1/5 with 0.1% TFA, 28.4 mg, 63% yield) with 64% e.e. [DAICEL CHIRALPAK IG column, Agilent HPLC 1260, *i*PrOH/*n*-hexane = 10/90 (v/v) with 0.1% TFA, 1.0 mL/min, 25 °C, 210 nm; t₁ = 9.0 min, t₂ = 15.2 min]: [α]_{D}²⁵ = -31.1 (c 1.0, MeOH, 64% e.e.); IR (film) *v*ₘₐₓ: 1710, 1524, 1240, 1046, 715 cm⁻¹; ¹H NMR (300 MHz, CD₃OD) δ 1.13 (t, *J* = 7.5 Hz, 3H), 2.23 (qd, *J* = 7.5, 2.3 Hz, 2H), 4.75 (d, *J* = 12.2 Hz, 1H), 4.82 (d, *J* = 12.2 Hz, 1H), 4.94 (t, *J =* 2.3 Hz, 1H); ¹³C NMR (75 MHz, CD₃OD) δ 13.0, 13.8, 47.5, 73.9, 75.7, 87.3, 96.9, 156.1, 171.2; HRMS calcd for [C₉H₁₀Cl₃NO₄Na]⁺ [(M + Na)⁺]: 323.9568; found: 323.9570.

### (R)-3,3-Dimethyl-2-(((2,2,2-trichloroethoxy)carbonyl)amino)butanoic acid (36)

Catalyzed by 10 mol% Λ-**RuH**, the 1,3-nitrogen shift of 2,2,2-trichloroethyl ((3,3- 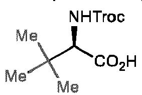 dimethylbutanoyl)oxy)carbamate (76.6 mg, 0.25 mmol) gave **36** as a colorless oil (chromatography on silica gel, eluent: EtOAc/n-hexane = 1/7 with 0.1% TFA, 15.6 mg, 20% yield) with 90% e.e. [DAICEL CHIRALCEL OD-H column, Agilent HPLC 1260, iPrOH/n-hexane = 5/95 (v/v) with 0.1% TFA, 1.0 mL/min, 25 °C, 210 nm; t₁ = 6.0 min, t₂ = 8.5 min]: [α]_{D}²⁵ = -15.4 (c 1.0, MeOH, 90% e.e.); IR (film) *v*ₘₐₓ: 2967, 1690, 1453, 1167, 812 cm⁻¹; ¹H NMR (300 MHz, CD₃OD) δ 1.04 (s, 9H), 4.05 (s, 1H), 4.74 (d, *J=* 12.2 Hz, 1H), 4.86 (d, *J=* 12.2 Hz, 1H); ¹³C NMR (75 MHz, CD₃OD) δ 27.1, 35.1, 64.4, 75.6, 97.1, 156.8, 174.2; HRMS calcd for [C₉H₁₄Cl₃NO₄Na]⁺ [(M + Na)⁺]: 327.9881; found: 327.9883.

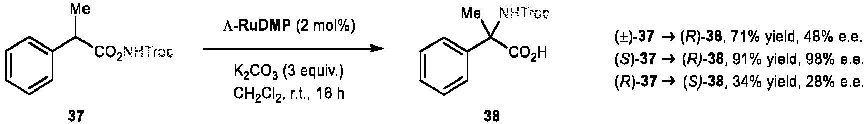

The 1,3-nitrogen shift of carbonyl α,α-disubstituted azanyl ester under ruthenium catalysis was investigated with racemic or enantiopure **37.** The reaction of racemic **37** led to **38** in 71% yield and 48% e.e., with the preferential formation of (R)-configuration product. The reaction of either configuration of enantiopure **37** was stereoretentive. However, (*S*)-**37** and Λ**-RuDMP** exhibited a matched substrate/catalyst combination as the reaction proceeded smoothly to give a virtually complete retention of the stereocenter, while (*R*)-**37** and Λ**-RuDMP** was mismatched and gave an inferior result with significant racemization of the stereocenter.

### (R)-2-Phenyl-2-(((2,2,2-trichloroethoxy)carbonyl)amino)propanoic acid [(R)-38]

Catalyzed by 2 mol% Λ**-RuDMP,** the 1,3-nitrogen shift of (*S*)-**37** (51.1 mg, 0.15 mmol) gave (*R*)-**38** as 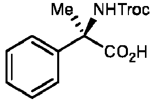 a colorless oil (chromatography on silica gel, eluent: EtOAc/*n*-hexane = 1/5 with 0.1% TFA, 46.4 mg, 91% yield) with 98% e.e. [DAICEL CHIRALPAK IG column, Agilent HPLC 1260, *i*PrOH/*n*-hexane = 10/90 (v/v) with 0.1% TFA, 1.0 mL/min, 25 °C, 210 nm; t₁ = 7.8 min, t₂ = 10.3 min]: [α]_{D}²⁵ = -52.5 (c 1.0, MeOH, 98% e.e.); IR (film) *v*ₘₐₓ: 1710, 1495, 1244, 1102, 1052, 697 cm⁻¹; ¹H NMR (300 MHz, CD₃OD) δ 1.98 (s, 3H), 4.72 (d, *J =* 12.2 Hz, 1H), 4.75 (d, *J* = 12.2 Hz, 1H), 7.14-7.42 (m, 3H), 7.44-7.62 (m, 2H); ¹³C NMR (75 MHz, CD₃OD) δ 23.5, 63.2, 75.2, 97.1, 127.2, 128.8, 129.3, 141.9, 154.8, 175.6; HRMS calcd for [C₁₂H₁₂Cl₃NO₄Na]⁺ [(M + Na)⁺]: 361.9724; found: 361.9725.

### (S)-2-Phenyl-2-(((2,2,2-trichloroethoxy)carbonyl)amino)propanoic acid [(S)-38]

Catalyzed by 2 mol% Λ-**RuDMP**, the 1,3-nitrogen shift of (*R*)-**37** gave (*S*)-**38** as a colorless oil in 34% 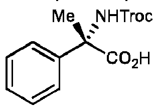 yield with 28% e.e.: [α]_{D}²⁵ =+1.2 (c 1.0, MeOH, 28% e.e.). The ¹H NMR and ¹³C NMR data of (*S*)-**38** are identical with those of (*R*)-**38**.

### (R)-2-(6-Methoxynaphthalen-2-yl)-2-(((2,2,2-trichloroethoxy)carbonyl)amino)propanoic acid (39)

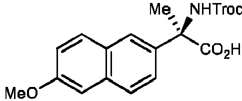

Catalyzed by 2 mol% Λ-**RuDMP**, the 1,3-nitrogen shift of 2,2,2-trichloroethyl (*S*)-((2-(6-methoxynaphthalen-2-yl)propanoyl)oxy)carbamate (42.1 mg, 0.1 mmol) gave **39** as a colorless oil (chromatography on silica gel, eluent: EtOAc/n-hexane = 1/5 with 0.1% TFA, 36.0 mg, 86% yield) with 99% e.e. [DAICEL CHIRALPAK IG column, Agilent HPLC 1260, *i*PrOH/*n*-hexane = 10/90 (v/v) with 0.1% TFA, 1.0 mL/min, 25 °C, 210 nm; t₁ = 15.4 min, t₂ = 26.4 min]: [α]_{D}²⁵ = -87.1 (*c* 1.0, MeOH, 99% e.e.); IR (film) *v*ₘₐₓ: 1710, 1491, 1264, 1197, 1099, 807, 712 cm⁻¹; ¹H NMR (300 MHz, CD₃OD) δ 2.07 (s, 3H), 3.88 (s, 3H), 4.72 (d, *J* = 12.2 Hz, 1H), 4.75 (d, *J* = 12.2 Hz, 1H), 7.12 (dd, *J* = 8.9, 2.5 Hz, 1H), 7.16-7.26 (m, 1H), 7.56 (dd, *J* = 8.8, 1.5 Hz, 1H), 7.66-7.81 (m, 2H), 7.85-7.98 (m, 1H); ¹³C NMR (75 MHz, CD₃OD) δ 23.5, 55.7, 63.2, 75.2, 97.1, 106.4, 120.0, 125.6, 126.1, 128.0, 129.9, 130.7, 135.5, 136.8, 154.8, 159.5, 175.7; HRMS calcd for [C₁₇H₁₆Cl₃NO₅Na]⁺ [(M + Na)⁺]: 441.9986; found: 441.9987.

### (R,E)-2-Methyl-2-(((2,2,2-trichloroethoxy)carbonyl)amino)hex-3-enoic acid (40)

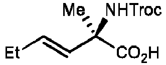

Catalyzed by 2 mol% Λ-**RuH**, the 1,3-nitrogen shift of 2,2,2-trichloroethyl (*S*,*E*)-((2-methylhex-3-enoyl)oxy)carbamate (47.8 mg, 0.15 mmol) gave **40** as a colorless oil (chromatography on silica gel, eluent: EtOAc/*n*-hexane = 1/6 with 0.1% TFA, 36.9 mg, 77% yield) with 87% e.e. [DAICEL CHIRALPAK IG column, Agilent HPLC 1260, *i*PrOH/*n*-hexane = 5/95 (v/v) with 0.1% TFA, 1.0 mL/min, 25 °C, 210 nm; t₁ = 10.2 min, t₂ = 14.0 min]: [α]_{D}²⁵ = -11.6 (*c* 1.0, MeOH, 87% e.e.); IR (film) *v*ₘₐₓ: 1712, 1500, 1331, 1109, 1053, 723 cm⁻¹; ¹H NMR (300 MHz, CD₃OD) δ 0.99 (t, *J* = 7.5 Hz, 3H), 1.57 (s, 3H), 2.00-2.16 (m, 2H), 4.73 (d, *J* = 12.2 Hz, 1H), 4.76 (d, *J* = 12.2 Hz, 1H), 5.69-5.87 (m, 2H); ¹³C NMR (75 MHz, CD₃OD) δ 13.7, 24.1, 26.3, 61.1, 75.2, 97.1, 130.2, 133.7, 155.2, 176.2; HRMS calcd for [C₁₀H₁₄Cl₃NO₄Na]⁺ [(M + Na)⁺]: 339.9881; found: 339.9886.

### (R)-2-Methyl-2-(((2,2,2-trichloroethoxy)carbonyl)amino)butanoic acid (41)

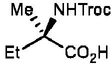

Catalyzed by 5 mol% Λ-**RuH**, the 1,3-nitrogen shift of 2,2,2-trichloroethyl (*S*)-((2-methylbutanoyl)oxy)carbamate (43.9 mg, 0.15 mmol) gave **41** as a colorless oil (chromatography on silica gel, eluent: EtOAc/n-hexane = 1/5 with 0.1% TFA, 24.9 mg, 57% yield) with 86% e.e. [DAICEL CHIRALCEL OD-H column, Agilent HPLC 1260, *i*PrOH/*n*-hexane = 10/90 (v/v) with 0.1% TFA, 1.0 mL/min, 25 °C, 210 nm; t₁ = 5.2 min, t₂ = 8.0 min]: [α]_{D}²⁵ = -17.7 (c 1.0, MeOH, 86% e.e.); IR (film) *v*ₘₐₓ: 1708, 1409, 1341, 1111, 725 cm⁻¹; ¹H NMR (300 MHz, CD₃OD) δ 0.88 (t, *J* = 7.5 Hz, 3H), 1.49 (s, 3H), 1.83-2.04 (m, 2H), 4.73 (d, *J* = 12.2 Hz, 1H), 4.76 (d, *J* = 12.2 Hz, 1H); ¹³C NMR (75 MHz, CD₃OD) δ 8.5, 22.7, 30.7, 61.1, 75.1, 97.2, 155.0, 177.3; HRMS calcd for [C₈H₁₂Cl₃NO₄Na]⁺ [(M + Na)⁺]: 313.9724; found: 313.9732.

### (R)-2-Cyclohexyl-2-(((2,2,2-trichloroethoxy)carbonyl)amino)propanoic acid (42)

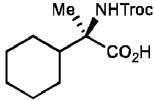

Catalyzed by 5 mol% Λ-**RuH**, the 1,3-nitrogen shift of 2,2,2-trichloroethyl (S)-((2-cyclohexylpropanoyl)oxy)carbamate (69.3 mg, 0.2 mmol) gave **42** as a colorless oil (chromatography on silica gel, eluent: EtOAc/n-hexane = 1/7 with 0.1% TFA, 17.3 mg, 25% yield) with 92% e.e. [DAICEL CHIRALPAK IG column, Agilent HPLC 1260, iPrOH/n-hexane = 5/95 (v/v) with 0.1% TFA, 1.0 mL/min, 25 °C, 210 nm; t₁ = 10.2 min, t₂ = 11.5 min]: [α]_{D}²⁵ = -6.1 (c 0.5, MeOH, 92% e.e.); IR (film) *v*ₘₐₓ: 2931, 2856, 1723, 1699, 1445, 1416, 732 cm⁻¹; ¹H NMR (300 MHz, CD₃OD) δ 0.99-1.93 (m, 5H), 1.49 (s, 3H), 1.61-1.92 (m, 6H), 4.69 (d, *J* = 12.2 Hz, 1H), 4.77 (d, *J =* 12.2 Hz, 1H); ¹³C NMR (75 MHz, CD₃OD) δ 19.2, 27.4, 27.6, 27.7, 28.4, 28.8, 46.0, 64.1, 75.2, 97.2, 155.5, 176.7; HRMS calcd for [C₁₂H₁₈Cl₃NO₄Na]⁺ [(M + Na)⁺]: 368.0194; found: 368.0200.

### 5. Iron-Catalyzed 1,3-Nitrogen Migration to Access N Troc-Protected α-Monosubstituted α-Amino Acids

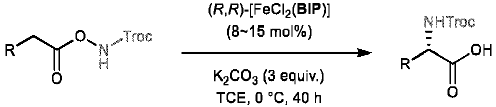

General procedures: to a Schlenk tube was added the substrate (1 equiv.), K₂CO₃ (3 equiv.) and (*R*,*R*)-[FeCl₂(**BIP**)] (8~15 mol%). 1,1,2,2-Tetrachloroethane (TCE, 0.1 M) was added, and the mixture was degassed via freeze-pump-thaw for two times. The tube was sealed, and the reaction mixture was stirred at 0 °C for 40 hours. To quench the reaction, brine (10 mL) and concentrated hydrochloric acid (1 mL/mmol substrate) was added, and the mixture was diluted with water (1 mL). The mixture was extracted with EtOAc for three times and the combined organic layer was dried over anhydrous sodium sulfate. After filtration, the solvent was evaporated under reduced pressure, and the residue was purified by column chromatography on silica gel using indicated solvent as the eluent.

### (S)-2-Phenyl-2-(((2,2,2-trichloroethoxy)carbonyl)amino)acetic acid (2e)

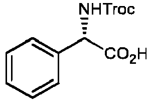

Catalyzed by 8 mol% (*R*,*R*)-[FeCl₂(**BIP**)], the 1,3-nitrogen shift of 2,2,2-trichloroethyl (2-phenylacetoxy)carbamate gave (*S*)-**2e** in 95% yield with 91% e.e.

### (S,E)-4-Phenyl-2-(((2,2,2-trichloroethoxy)carbonyl)amino)but-3-enoic acid (32)

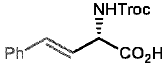

Catalyzed by 8 mol% (*R*,*R*)-[FeCl₂(**BIP**)], the 1,3-nitrogen shift of 2,2,2-trichloroethyl (E)-((4-phenylbut-3-enoyl)oxy)carbamate gave (*S*)-**32** in 44% yield with 86% e.e.

### (S)-4-Phenyl-2-(((2,2,2-trichloroethoxy)carbonyl)amino)but-3-ynoic acid (34)

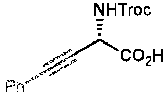

Catalyzed by 8 mol% (*R*,*R*)-[FeCl₂(**BIP**)], the 1,3-nitrogen shift of 2,2,2-trichloroethyl ((4-phenylbut-3-ynoyl)oxy)carbamate gave (*S*)-**34** in 13% yield with 44% e.e.

### (S)-2-(((2,2,2-Trichloroethoxy)carbonyl)amino)butanoic acid (43)

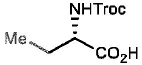

Catalyzed by 8 mol% (*R*,*R*)-[FeCl₂(**BIP**)], the 1,3-nitrogen shift of 2,2,2-trichloroethyl (butyryloxy)carbamate (55.7 mg, 0.2 mmol) gave **43** as a colorless oil (chromatography on silica gel, eluent: EtOAc/*n*-hexane = 1/5 with 0.1% TFA, 34.3 mg, 62% yield) with 91% e.e. [DAICEL CHIRALPAK IG column, Agilent HPLC 1260, *i*PrOH/*n*-hexane = 10/90 (v/v) with 0.1% TFA, 1.0 mL/min, 25 °C, 210 nm; t₁ = 9.0 min, t₂ = 14.0 min]: [α]_{D}²⁵ = -40.0 (c 1.0, MeOH, 91% e.e.); IR (film) *v*ₘₐₓ: 1723, 1686, 1460, 1393, 1105, 813, 722 cm⁻¹; ¹H NMR (300 MHz, CD₃OD) δ 1.00 (t, *J* = 7.4 Hz, 3H), 1.62-1.82 (m, 1H), 1.82-2.02 (m, 1H), 4.10 (dd, *J* = 8.6, 5.1 Hz, 1H), 4.74 (d, *J* = 12.2 Hz, 1H), 4.83 (d, *J* = 12.2 Hz, 1H); ¹³C NMR (75 MHz, CD₃OD) δ 10.6, 26.0, 57.0, 75.5, 97.1, 156.7, 175.5; HRMS calcd for [C₇H₁₀Cl₃NO₄Na]⁺ [(M + Na)⁺]: 299.9568; found: 299.9567.

### (S)-2-(((2,2,2-Trichloroethoxy)carbonyl)amino)octanoic acid (44)

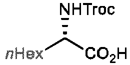

Catalyzed by 8 mol% (*R*,*R*)-[FeCl₂(**BIP**)], the 1,3-nitrogen shift of 2,2,2-trichloroethyl (octanoyloxy)carbamate (66.9 mg, 0.2 mmol) gave **44** as a colorless oil (chromatography on silica gel, eluent: EtOAc/n-hexane = 1/6 with 0.1% TFA, 50.5 mg, 75% yield) with 92% e.e. [DAICEL CHIRALPAK IG column, Agilent HPLC 1260, *i*PrOH/*n*-hexane = 5/95 (v/v) with 0.1% TFA, 1.0 mL/min, 25 °C, 210 nm; t₁ = 13.4 min, t₂ = 20.8 min]: [α]_{D}²⁵ = +9.5 (*c* 1.0, MeOH, 92% e.e.); IR (film) *v*ₘₐₓ: 2957, 2926, 1719, 1688, 1395, 1117, 810, 720 cm⁻¹; ¹H NMR (300 MHz, CD₃OD) δ 0.71-1.04 (m, 3H), 1.16-1.56 (m, 8H), 1.60-1.78 (m, 1H), 1.78-1.96 (m, 1H), 4.15 (dd, *J* = 9.1, 4.8 Hz, 1H), 4.73 (d, *J* = 12.2 Hz, 1H), 4.84 (d, *J* = 12.2 Hz, 1H); ¹³C NMR (75 MHz, CD₃OD) δ 14.4, 23.6, 26.8, 29.8, 32.7, 32.8, 55.5, 75.5, 97.1, 156.7, 175.7; HRMS calcd for [C₁₁H₁₈Cl₃NO₄Na]⁺ [(M + Na)⁺]: 356.0194; found: 356.0194.

### (S)-5-Phenyl-2-(((2,2,2-trichloroethoxy)carbonyl)amino)pentanoic acid (45)

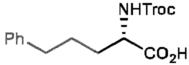

Catalyzed by 8 mol% (*R*,*R*)-[FeCl₂(**BIP**)], the 1,3-nitrogen shift of 2,2,2-trichloroethyl ((5-phenylpentanoyl)oxy)carbamate (73.7 mg, 0.2 mmol) gave **45** as a colorless oil (chromatography on silica gel, eluent: EtOAc/n-hexane = 1/5 with 0.1% TFA, 43.9 mg, 60% yield) with 91% e.e. [DAICEL CHIRALPAK IG column, Agilent HPLC 1260, *i*PrOH/*n*-hexane = 10/90 (v/v) with 0.1% TFA, 1.0 mL/min, 25 °C, 210 nm; t₁ = 10.7 min, t₂ = 14.0 min]: [α]_{D}²⁵ = -26.3 (c 1.0, MeOH, 91% e.e.); IR (film) *v*ₘₐₓ: 1710, 1416, 1336, 1097, 1050, 724, 699 cm⁻¹; ¹H NMR (300 MHz, CD₃OD) δ 1.62-1.80 (m, 3H), 1.80-1.99 (m, 1H), 2.54-2.73 (m, 2H), 4.21 (dd, *J* = 8.3, 4.6 Hz, 1H), 4.72 (d, *J =* 12.2 Hz, 1H), 4.84 (d, *J =* 12.2 Hz, 1H), 7.09-7.20 (m, 3H), 7.20-7.31 (m, 2H); ¹³C NMR (75 MHz, CD₃OD) δ 28.8, 32.3, 36.2, 55.3, 75.5, 97.1, 126.8, 129.3, 129.4, 143.2, 156.7, 175.6; HRMS calcd for [C₁₄H₁₆Cl₃NO₄Na]⁺ [(M + Na)⁺]: 390.0037; found: 390.0037.

### (S)-6-Methoxy-6-oxo-2-(((2,2,2-trichloroethoxy)carbonyl)amino)hexanoic acid (46)

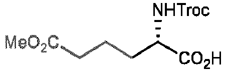

Catalyzed by 8 mol% (*R*,*R*)-[FeCl₂(**BIP**)], the 1,3-nitrogen shift of methyl 6-oxo-6-((((2,2,2-trichloroethoxy)carbonyl)amino)oxy)hexanoate (70.1 mg, 0.2 mmol) gave **46** as a colorless oil (chromatography on silica gel, eluent: EtOAc/n-hexane = 1/2 with 0.1% TFA, 49.2 mg, 70% yield) with 90% e.e. [DAICEL CHIRALCEL OD-H column, Agilent HPLC 1260, *i*PrOH/*n*-hexane = 10/90 (v/v) with 0.1% TFA, 1.0 mL/min, 25 °C, 210 nm; t₁ = 7.7 min, t₂ = 9.0 min]: [α]_{D}²⁵ = -33.2 (*c* 1.0, MeOH, 90% e.e.); IR (film) *v*ₘₐₓ: 1709, 1430, 1204, 724 cm⁻¹; ¹H NMR (300 MHz, CD₃OD) δ 1.59-1.82 (m, 3H), 1.82-1.99 (m, 1H), 2.27-2.47 (m, 2H), 3.66 (s, 3H), 4.17 (dd, *J* = 8.5, 4.6 Hz, 1H), 4.74 (d, *J* = 12.2 Hz, 1H), 4.84 (d, *J* = 12.2 Hz, 1H); ¹³C NMR (75 MHz, CD₃OD) δ 22.4, 31.9, 34.1, 52.1, 55.2, 75.5, 97.1, 156.7, 175.3, 175.4; HRMS calcd for [C₁₀H₁₄Cl₃NO₆Na]⁺ [(M + Na)⁺]: 371.9779; found: 371.9778.

### O-Phenyl-N-((2,2,2-trichloroethoxy)carbonyl)-L-homoserine (47)

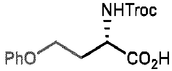

Catalyzed by 15 mol% (*R*,*R*)-[FeCl₂(**BIP**)], the 1,3-nitrogen shift of 2,2,2-trichloroethyl ((4-phenoxybutanoyl)oxy)carbamate (55.6 mg, 0.15 mmol) gave **47** as a colorless oil (chromatography on silica gel, eluent: EtOAc/n-hexane = 1/4 with 0.1% TFA, 29.7 mg, 53% yield) with 90% e.e. [DAICEL CHIRALPAK IG column, Agilent HPLC 1260, iPrOH/n-hexane = 20/80 (v/v) with 0.1% TFA, 1.0 mL/min, 25 °C, 210 nm; t₁ = 6.6 min, t₂ = 8.1 min]: [α]_{D}²⁵ = +6.8 (c 1.0, MeOH, 90% e.e.); IR (film) *v*ₘₐₓ: 1713, 1497, 1233, 1103, 1048, 751, 723, 691 cm⁻¹; ¹H NMR (300 MHz, CD₃OD) δ 2.01-2.22 (m, 1H), 2.30-2.48 (m, 1H), 3.96-4.16 (m, 2H), 4.46 (dd, *J =* 9.5, 4.6 Hz, 1H), 4.71 (d, *J* = 12.2 Hz, 1H), 4.81 (d, *J* = 12.2 Hz, 1H), 6.81-6.99 (m, 3H), 7.13-7.31 (m, 2H); ¹³C NMR (75 MHz, CD₃OD) δ 32.3, 52.8, 65.2, 75.3, 97.0, 115.7, 121.9, 130.4, 156.7, 160.2, 175.3; HRMS calcd for [C₁₃H₁₄Cl₃NO₅Na]⁺ [(M + Na)⁺]: 391.9830; found: 391.9832.

### Methyl (S)-4-((tert-butoxycarbonyl)amino)-2-(((2,2,2-trichloroethoxy)carbonyl)amino)butanoate (48)

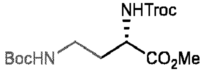

Catalyzed by 8 mol% (*R*,*R*)-[FeCl₂(**BIP**)] at room temperature for 16 hours, the 1,3-nitrogen shift of 2,2,2-trichloroethyl ((4-((*tert-*butoxycarbonyl)amino)butanoyl)oxy)carbamate (78.7 mg, 0.2 mmol) and the methylation by trimethylsilyldiazomethane gave **48** as a colorless oil (chromatography on silica gel, eluent: EtOAc/n-hexane = 1/3, 41.5 mg, 51% yield for two steps) with 90% e.e. [DAICEL CHIRALPAK IC column, Agilent HPLC 1260, iPrOH/n-hexane = 10/90 (v/v), 1.0 mL/min, 25 °C, 210 nm; t₁ = 10.0 min, t₂ = 14.3 min]: [α]_{D}²⁵ = -16.9 (c 1.0, MeOH, 90% e.e.); IR (film) *v*ₘₐₓ: 3346, 2972, 1721, 1692, 1514, 1246, 1165, 1100, 726 cm⁻¹; ¹H NMR (300 MHz, CDCl₃) δ 1.43 (s, 9H), 1.77-1.92 (m, 1H), 2.02-2.16 (m, 1H), 2.96-3.14 (m, 1H), 3.29-3.47 (m, 1H), 3.76 (s, 3H), 4.44 (dt, *J* = 8.5, 4.5 Hz, 1H), 4.69 (d, *J* = 12.1 Hz, 1H), 4.78 (d, *J* = 12.1 Hz, 1H), 4.83-5.09 (br, 1H), 5.87 (d, *J* = 8.5 Hz, 1H); ¹³C NMR (75 MHz, CDCl₃) δ 28.5, 33.2, 36.7, 51.9, 52.9, 74.8, 79.7, 95.5, 154.7, 156.1, 172.4; HRMS calcd for [C₁₃H₂₁Cl₃N₂O₆Na]⁺ [(M + Na)⁺]: 429.0357; found: 429.0363.

### (S)-3-Cyclohexyl-2-(((2,2,2-trichloroethoxy)carbonyl)amino)propanoic acid (49)

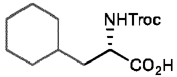

Catalyzed by 8 mol% (*R*,*R*)-[FeCl₂(**BIP**)], the 1,3-nitrogen shift of 2,2,2-trichloroethyl ((3-cyclohexylpropanoyl)oxy)carbamate (69.3 mg, 0.2 mmol) gave **49** as a colorless oil (chromatography on silica gel, eluent: EtOAc/n-hexane = 1/6 with 0.1% TFA, 39.8 mg, 57% yield) with 91% e.e. [DAICEL CHIRALPAK IG column, Agilent HPLC 1260, iPrOH/n-hexane = 10/90 (v/v) with 0.1% TFA, 1.0 mL/min, 25 °C, 210 nm; t₁ = 9.4 min, t₂ = 12.7 min]: [α]_{D}²⁵ = -28.3 (c 1.0, MeOH, 91% e.e.); IR (film) *v*ₘₐₓ: 2925, 2851, 1709, 1417, 1336, 1043, 723 cm⁻¹; ¹H NMR (300 MHz, CD₃OD) δ 0.85-1.06 (m, 2H), 1.15-1.34 (m, 3H), 1.36-1.53 (m, 1H), 1.53-1.77 (m, 6H), 1.77-1.92 (m, 1H), 4.24 (dd, *J* = 10.1, 5.0 Hz, 1H), 4.72 (d, *J* = 12.2 Hz, 1H), 4.86 (d, *J* = 12.2 Hz, 1H); ¹³C NMR (75 MHz, CD₃OD) δ 27.2, 27.4, 27.6, 33.1, 34.8, 35.4, 40.2, 53.3, 75.5, 97.2, 156.7, 176.3; HRMS calcd for [C₁₂H₁₈Cl₃NO₄Na]⁺ [(M + Na)⁺]: 368.0194; found: 368.0190.

### (S)-3-Cyclopentyl-2-(((2,2,2-trichloroethoxy)carbonyl)amino)propanoic acid (50)

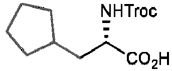

Catalyzed by 8 mol% (*R*,*R*)-[FeCl₂(**BIP**)], the 1,3-nitrogen shift of 2,2,2-trichloroethyl ((3-cyclopentylpropanoyl)oxy)carbamate (66.5 mg, 0.2 mmol) gave **50** as a colorless oil (chromatography on silica gel, eluent: EtOAc/n-hexane = 1/6 with 0.1% TFA, 43.5 mg, 65% yield) with 92% e.e. [DAICEL CHIRALPAK IG column, Agilent HPLC 1260, iPrOH/n-hexane = 10/90 (v/v) with 0.1% TFA, 1.0 mL/min, 25 °C, 210 nm; t₁ = 9.3 min, t₂ = 13.3 min]: [α]_{D}²⁵ = -11.6 (c 1.0, MeOH, 92% e.e.); IR (film) *v*ₘₐₓ: 2951, 1710, 1411, 1335, 1107, 1045, 722 cm⁻¹; ¹H NMR (300 MHz, CD₃OD) δ 1.04-1.26 (m, 2H), 1.46-1.72 (m, 4H), 1.72-1.89 (m, 4H), 1.89-2.05 (m, 1H), 4.17 (dd, *J* = 8.4, 6.3 Hz, 1H), 4.73 (d, *J* = 12.2 Hz, 1H), 4.84 (d, *J* = 12.2 Hz, 1H); ¹³C NMR (75 MHz, CD₃OD) δ 25.9, 26.1, 33.0, 33.8, 38.1, 38.8, 55.2, 75.5, 97.1, 156.7, 176.0; HRMS calcd for [C₁₁H₁₆Cl₃NO₄Na]⁺ [(M + Na)⁺]: 354.0037; found: 354.0037.

### (S)-2-Cyclohexyl-2-(((2,2,2-trichloroethoxy)carbonyl)amino)acetic acid (51)

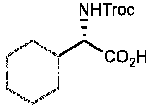

Catalyzed by 8 mol% (*R*,*R*)-[FeCl₂(**BIP**)], the 1,3-nitrogen shift of 2,2,2-trichloroethyl (2-cyclohexylacetoxy)carbamate (66.5 mg, 0.2 mmol) gave **51** as a colorless oil (chromatography on silica gel, eluent: EtOAc/n-hexane = 1/6 with 0.1% TFA, 32.0 mg, 48% yield) with 89% e.e. [DAICEL CHIRALCEL OD-H column, Agilent HPLC 1260, iPrOH/n-hexane = 5/95 (v/v) with 0.1% TFA, 1.0 mL/min, 25 °C, 210 nm; t₁ = 6.5 min, t₂ = 9.2 min]: [α]_{D}²⁵ = +4.4 (c 1.0, MeOH, 89% e.e.); IR (film) *v*ₘₐₓ: 2929, 2856, 1708, 1420, 1336, 1100, 722 cm⁻¹; ¹H NMR (300 MHz, CD₃OD) δ 1.00-1.43 (m, 5H), 1.44-1.96 (m, 6H), 4.08 (d, *J* = 6.1 Hz, 1H), 4.74 (d, *J* = 12.2 Hz, 1H), 4.84 (d, *J* = 12.2 Hz, 1H); ¹³C NMR (75 MHz, CD₃OD) δ 27.09, 27.14 (2C), 29.4, 30.8, 41.4, 60.8, 75.6, 97.1, 156.9, 174.8; HRMS calcd for [C₁₁H₁₆Cl₃NO₄Na]⁺ [(M + Na)⁺]: 354.0037; found: 354.0038.

### (S)-3,3-Dimethyl-2-(((2,2,2-trichloroethoxy)carbonyl)amino)butanoic acid (36)

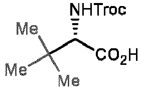

Catalyzed by 15 mol% (*R*,*R*)-[FeCl₂(**BIP**)], the 1,3-nitrogen shift of 2,2,2-trichloroethyl ((3,3-dimethylbutanoyl)oxy)carbamate gave (*S*)-**36** in 56% yield with 85% e.e.

### (2S,4R)-4-((3R,5R,8R,9S,10S,13R,14S,17R)-3-Hydroxy-10,13-dimethylhexadecahydro-1H-cyclopenta[a]phenanthren-17-yl)-2-(((2,2,2-trichloroethoxy)carbonyl)amino)pentanoic acid (52)

Catalyzed by 8 mol% (*R*,*R*)-[FeCl₂(**BIP**)] at room temperature for 16 hours, the 1,3-nitrogen shift of 2,2,2-trichloroethyl (((R)-4-((3R,5R,8R,9S,10S,13R,14S,17R)-3-hydroxy-10, 13-dimethylhexadecahydro-1*H*-cyclopenta[a]phenanthren-17-yl)pentanoyl)oxy)carbamate (85.0 mg, 0.15 mmol) gave **52** and its epimer (2-*epi*-**52**) in 77% combined yield (chromatography on silica gel, eluent: EtOAc/n-hexane = 1/3 with 0.1% TFA, 65.6 mg) with 23:1 d.r. [determined by HPLC analysis: DAICEL CHIRALCEL OD-H column, Agilent HPLC 1260, *i*PrOH/*n*-hexane = 20/80 (v/v) with 0.1% TFA, 1.0 mL/min, 25 °C, 210 nm; t₁ **(52)** = 9.7 min, t₂ (2*-epi-***52**) = 13.4 min]. 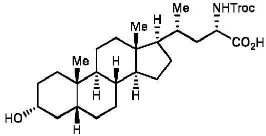 **52** (less polar, major diastereomer): [α]_{D}²⁵ = -4.9 (c 1.0, MeOH); IR (film) *v*ₘₐₓ: 2931, 2861, 1702, 1214, 1158, 1041, 724 cm⁻¹; ¹H NMR (300 MHz, CD₃OD) δ 0.69 (s, 3H), 0.94 (s, 3H), 1.05 (d, *J* = 6.4 Hz, 3H), 1.08-2.09 (m, 26H), 3.42-3.64 (m, 1H), 4.20 (dd, *J* = 8.2, 6.3 Hz, 1H), 4.71 (d, *J* = 12.2 Hz, 1H), 4.84 (d, *J* = 12.2 Hz, 1H); ¹³C NMR (75 MHz, CD₃OD) δ 12.4, 19.6, 21.9, 24.0, 25.3, 27.6, 28.4, 29.3, 31.2, 35.1, 35.7, 36.5, 37.17, 37.22, 39.8, 41.5, 41.9, 43.6, 44.0, 54.3, 57.9 (2C), 72.4, 75.5, 97.1, 156.3, 176.0; HRMS calcd for [C₂₇H₄₂Cl₃NO₅Na]⁺ [(M + Na)⁺]: 588.2021; found: 588.2019. 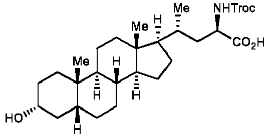 2-*epi*-**52** (more polar, minor diastereomer): [α]_{D}²⁵ = +24.0 (*c* 1.0, MeOH); IR (film) *v*ₘₐₓ: 2932, 2862, 1718, 1211, 1157, 725 cm⁻¹; ¹H NMR (300 MHz, CD₃OD) δ 0.68 (s, 3H), 0.95 (s, 3H), 0.97-1.01 (m, 3H), 1.08-2.09 (m, 26H), 3.42-3.64 (m, 1H), 4.28 (dd, *J* = 12.0, 2.5 Hz, 1H), 4.71 (d, *J* = 12.2 Hz, 1H), 4.87 (d, *J =* 12.2 Hz, 1H); ¹³C NMR (75 MHz, CD₃OD) δ 12.6, 18.3, 22.0, 24.0, 25.3, 27.6, 28.4, 29.2, 31.2, 34.0, 35.7, 36.5, 37.17, 37.24, 39.1, 41.6, 41.9, 43.5, 44.1, 53.1, 57.9 (2C), 72.4, 75.5, 97.2, 156.9, 176.4; HRMS calcd for [C₂₇H₄₂Cl₃NO₅Na]⁺ [(M + Na)⁺]: 588.2021; found: 588.2016.

### 6. Iron-Catalyzed 1,3-Nitrogen Migration to Access N Troc-Protected α,α-Disubstituted α-Amino Acids

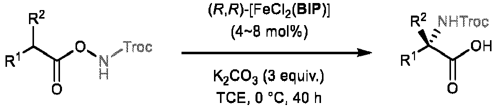

General procedures: to a Schlenk tube was added the substrate (1 equiv.), K₂CO₃ (3 equiv.) and (*R*,*R*)-[FeCl₂(**BIP**)] (4~8 mol%). 1,1,2,2-Tetrachloroethane (TCE, 0.1 M) was added, and the mixture was degassed via freeze-pump-thaw for two times. The tube was sealed, and the reaction mixture was stirred at 0 °C for 40 hours. To quench the reaction, brine (10 mL) and concentrated hydrochloric acid (1 mL/mmol substrate) was added, and the mixture was diluted with water (1 mL). The mixture was extracted with EtOAc for three times and the combined organic layer was dried over anhydrous sodium sulfate. After filtration, the solvent was evaporated under reduced pressure, and the residue was purified by column chromatography on silica gel using indicated solvent as the eluent.

### (5)-2-Phenyl-2-(((2,2,2-trichloroethoxy)carbonyl)amino)propanoic acid (53)

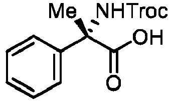

Starting from 2,2,2-trichloroethyl ((2-phenylpropanoyl)oxy)carbamate (68.0 mg, 0.20 mmol) according to the general procedure to provide **53** as a white solid (chromatography on silica gel, eluent: EtOAc/n-hexane = 1/5 with 0.1% TFA, 59.0 mg, 87% yield) and with 83% ee as determined by HPLC analysis (column: Daicel Chiralpak ODR 250 x 4.6 mm, Particle size: 10 µm, absorption: λ = 210 nm, mobile phase: CH₃CN/H₂O (0.1% TFA) = 40:60, flow rate: 1.0 mL/min, column temperature: 25 °C, retention times: tᵣ (major) = 18.3 min, tᵣ (minor) = 16.1 min). [α]_{D}²² = 15.9 (c = 1.0, MeOH). **¹H NMR** (300 MHz, MeOD) δ 7.57 - 7.48 (m, 2H), 7.40 - 7.23 (m, 3H), 4.80 - 4.65 (m, 2H), 1.98 (s, 3H). **¹³C NMR** (75 MHz, MeOD) δ 172.71, 151.92, 138.97, 126.44, 125.89, 124.33, 94.18, 72.29, 60.27, 20.60. **IR (film):** *v* (cm⁻¹)3398, 2953, 1713, 1494, 1450, 1245, 1102, 765, 696, 569 cm⁻¹. **HRMS (ESI, *m*/*z*)** calcd. for [C₁₂H₁₂Cl₃NO₄H] ⁺ [(M + H) ⁺]: 339.9905, found: 339.9905.

### (S)-2-(p-Tolyl)-2-(((2,2,2-trichloroethoxy)carbonyl)amino)propanoic acid (54)

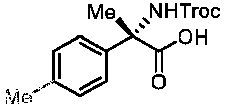

Starting from 2,2,2-trichloroethyl ((2-(*p*-tolyl)propanoyl)oxy)carbamate (70.6 mg, 0.20 mmol) according to the general procedure to provide **54** as a colorless oil (chromatography on silica gel, eluent: EtOAc/n-hexane = 1/5 with 0.1% TFA, 66.0 mg, 93% yield) and with 87% ee as determined by HPLC analysis (column: Daicel Chiralpak IG 250 x 4.6 mm, Particle size: 5 µm, absorption: λ = 210 nm, mobile phase: CH₃CN/H₂O (0.1% TFA) = 50:50, flow rate: 1.0 mL/min, column temperature: 25 °C, retention times: tᵣ (major) = 11.3 min, tᵣ (minor) = 13.0 min). **α]_{D}²²** = 23.1 (c = 1.0, MeOH). **¹H NMR** (300 MHz, MeOD) δ 7.21 - 7.12 (m, 2H), 6.92 (d, *J* = 8.3 Hz, 2H), 4.56 - 4.40 (m, 2H), 2.08 (s, 3H), 1.72 (s, 3H). **¹³C NMR** (75 MHz, MeOD) δ 173.97, 152.98, 137.04, 136.89, 128.17, 125.31, 95.30, 73.38, 61.15, 21.61, 19.23. **IR (film):** v (cm⁻¹) 3400, 2951, 1700, 1406, 1245, 1099, 1053, 815, 719, 569 cm⁻¹. **HRMS (ESI, *m*/*z)*** calcd. for [C₁₃H₁₄Cl₃NO₄Na] ⁺ [(M + Na) ⁺]: 375.9881, found: 375.9881.

### (S)-2-(4-(tert-Butyl)phenyl)-2-(((2,2,2-trichloroethoxy)carbonyl)amino)propanoic acid (55)

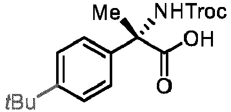

Starting from 2,2,2-trichloroethyl ((2-(4-(tert-butyl)phenyl)propanoyl)oxy)carbamate (79.0 mg, 0.20 mmol) according to the general procedure to provide **55** as a white solid (chromatography on silica gel, eluent: EtOAc/n-hexane = 1/5 with 0.1% TFA, 49.0 mg, 62% yield) and with 84% ee as determined by HPLC analysis (column: Daicel Chiralpak IG 250 x 4.6 mm, Particle size: 5 µm, absorption: λ = 210 nm, mobile phase: CH₃CN/H₂O (0.1% TFA) = 50:50, flow rate: 1.0 mL/min, column temperature: 25 °C, retention times: tᵣ (major) = 13.7 min, tᵣ (minor) = 15.9 min). **[α]_{D}²²** = 12.9 (c = 1.0, MeOH). **¹H NMR** (300 MHz, MeOD) δ 7.49 - 7.34 (m, 4H), 4.73 (s, 2H), 1.97 (s, 3H), 1.31 (s, 9H). **¹³C NMR** (75 MHz, MeOD) δ 174.90, 153.97, 151.00, 137.96, 126.07, 125.42, 96.24, 74.35, 62.08, 34.42, 30.86, 22.60. **IR (film):** v (cm⁻¹) 3331, 2958, 1703, 1495, 1234, 1097, 819, 712, 564 cm⁻¹. **HRMS (ESI, *m*/*z)*** calcd. for [C₁₆H₂₀Cl₃NO₄Na] ⁺ [(M + Na) ⁺]: 418.0350, found: 418.0350.

### (5)-2-(4-Isobutylphenyl)-2-(((2,2,2-trichloroethoxy)carbonyl)amino)propanoic acid (56)

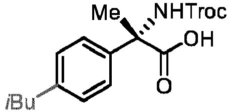

Starting from 2,2,2-trichloroethyl ((2-(4-isobutylphenyl)propanoyl)oxy)carbamate (79.0 mg, 0.20 mmol) according to the general procedure to provide **56** as a white solid (chromatography on silica gel, eluent: EtOAc/*n*-hexane = 1/5 with 0.1% TFA, 55.0 mg, 70% yield) and with 85% ee as determined by HPLC analysis (column: Daicel Chiralpak ODR 250 x 4.6 mm, Particle size: 10 µm, absorption: λ = 210 nm, mobile phase: CH₃CN/H₂O (0.1% TFA) = 50:50, flow rate: 1.0 mL/min, column temperature: 25 °C, retention times: tᵣ (major) = 17.9 min, tᵣ (minor) = 16.1 min). **[α]_{D}²²** = 24.2 (c = 127.4, MeOH, 99% ee). **¹H NMR** (300 MHz, MeOD) δ 7.50 - 7.37 (m, 2H), 7.23 - 7.05 (m, 2H), 4.80 - 4.65 (m, 2H), 2.47 (d, *J* = 7.2 Hz, 2H), 1.96 (s, 3H), 1.92-1.77 (m, 1H), 0.90 (d, *J* = 6.6 Hz, 6H). **¹³C NMR** (75 MHz, MeOD) δ 174.41, 153.48, 141.14, 137.82, 128.72, 125.64, 95.75, 73.82, 61.64, 44.56, 29.98, 22.11, 21.36. **IR (film):** v (cm⁻¹) 3277, 2954, 1711, 1493, 1246, 1100, 815, 722, 537 cm⁻¹. **HRMS (ESI, *m*/*z)*** calcd. for [C₁₆H₂₀Cl₃NO₄Na] ⁺ [(M + Na) ⁺]: 418.0350, found: 418.0350.

### (S)-2-(4-Fluorophenyl)-2-(((2,2,2-trichloroethoxy)carbonyl)amino)propanoic acid (57)

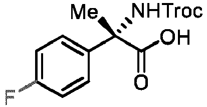

Starting from 2,2,2-trichloroethyl ((2-(4-fluorophenyl)propanoyl)oxy)carbamate (71.4 mg, 0.20 mmol) according to the general procedure to provide **57** as a colorless oil (chromatography on silica gel, eluent: EtOAc/*n*-hexane = 1/5 with 0.1% TFA, 62.0 mg, 86% yield) and with 83% ee as determined by HPLC analysis (column: Daicel Chiralpak ODR 250 x 4.6 mm, Particle size: 10 µm, absorption: λ = 210 nm, mobile phase: CH₃CN/H₂O (0.1% TFA) = 45:55, flow rate: 1.0 mL/min, column temperature: 25 °C, retention times: tᵣ (major) = 12.1 min, tᵣ (minor) = 10.3 min). **[α]_{D}²²** = 13.4 (c = 1.0, MeOH). **¹H NMR** (300 MHz, MeOD) δ 7.61 - 7.48 (m, 2H), 7.13 - 6.97 (m, 2H), 4.78 - 4.67 (m, 2H), 1.96 (s, 3H). **¹³C NMR** (75 MHz, MeOD) δ 174.57, 162.81 (d, *J*_{C-F} = 245.1 Hz), 153.00, 137.10, 128.55 (d, *J*_{C-F} = 8.3 Hz), 115.03 (d, *J*_{C-F} = 21.7 Hz), 96.22, 74.35, 61.85, 22.84. **¹⁹F NMR** (282 MHz, MeOD) δ -117.17. **IR (film):** v (cm⁻¹) 3398, 2999, 1709, 1509, 1406, 1232, 1105, 808, 720, 568 cm⁻¹. **HRMS (ESI, *m*/*z*)** calcd. for [C₁₂H₁₁Cl₃FNO₄Na] ⁺ [(M + Na) ⁺]: 379.9630, found: 379.9630.

### (S)-2-(4-Chlorophenyl)-2-(((2,2,2-trichloroethoxy)carbonyl)amino)propanoic acid (58)

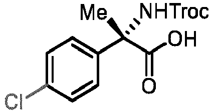

Starting from 2,2,2-trichloroethyl ((2-(4-chlorophenyl)propanoyl)oxy)carbamate (74.6 mg, 0.20 mmol) according to the general procedure to provide **58** as a colorless oil (chromatography on silica gel, eluent: EtOAc/*n*-hexane = 1/5 with 0.1% TFA, 72.0 mg, 96% yield) and with 83% ee as determined by HPLC analysis (column: Daicel Chiralpak IG 250 x 4.6 mm, Particle size: 5 µm, absorption: λ = 210 nm, mobile phase: CH₃CN/H₂O (0.1% TFA) = 50:50, flow rate: 1.0 mL/min, column temperature: 25 °C, retention times: tᵣ (major) = 10.2 min, tᵣ (minor) = 11.7 min). **[α]_{D}²²** = 12.6 (c = 1.0, MeOH). **¹H NMR** (300 MHz, MeOD) δ 7.56 - 7.46 (m, 2H), 7.39 - 7.30 (m, 2H), 4.73 (s, 2H), 1.95 (s, 3H). **¹³C NMR** (75 MHz, MeOD) δ 174.31, 153.95, 139.94, 133.79, 128.47, 128.22, 96.19, 74.37, 61.91, 22.85. **IR (film):** v (cm⁻¹) 3400, 2953, 1707, 1491, 1263, 1094, 818, 719, 569 cm⁻¹. **HRMS (ESI, *m*/*z)*** calcd. for [C₁₂H₁₁Cl₄NO₄Na] ⁺ [(M + Na) ⁺]: 395.9334, found: 395.9334.

### (S)-2-(4-Bromophenyl)-2-(((2,2,2-trichloroethoxy)carbonyl)amino)propanoic acid (59)

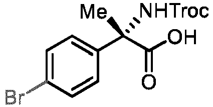

Starting from 2,2,2-trichloroethyl ((2-(4-bromophenyl)propanoyl)oxy)carbamate (83.4 mg, 0.20 mmol) according to the general procedure to provide **59** as a white solid (chromatography on silica gel, eluent: EtOAc/n-hexane = 1/5 with 0.1% TFA, 51.0 mg, 61% yield) and with 82% ee as determined by HPLC analysis (column: Daicel Chiralpak IG 250 x 4.6 mm, Particle size: 5 µm, absorption: λ = 210 nm, mobile phase: CH₃CN/H₂O (0.1% TFA) = 50:50, flow rate: 1.0 mL/min, column temperature: 25 °C, retention times: tᵣ (major) = 11.6 min, tᵣ (minor) = 13.0 min). **[α]_{D}²²** = 3.3 (c = 1.0, MeOH). **¹H NMR** (300 MHz, MeOD) δ 7.54 - 7.40 (m, 4H), 4.73 (s, 2H), 1.94 (s, 3H). **¹³C NMR** (75 MHz, MeOD) δ 174.24, 153.97, 140.48, 131.50, 128.56, 121.82, 96.22, 74.38, 61.99, 22.82. **IR (film):** v (cm⁻¹) 3307, 2954, 1708, 1487, 1183, 1008, 809, 717, 568 cm⁻¹. **HRMS (ESI, *m*/*z)*** calcd. for [C₁₂H₁₁BrCl₃NO₄Na] ⁺ [(M + Na) ⁺]: 439.8829, found: 439.8829.

### (S)-2-(((2,2,2-Trichloroethoxy)carbonyl)amino)-2-(4-(trifluoromethyl)phenyl)propanoic acid (60)

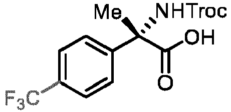

Starting from 2,2,2-trichloroethyl ((2-(4-(trifluoromethyl)phenyl)propanoyl)oxy)carbamate (81.4 mg, 0.20 mmol) according to the general procedure to provide **60** as a white solid (chromatography on silica gel, eluent: EtOAc/*n*-hexane = 1/5 with 0.1% TFA, 53.0 mg, 65% yield) and with 80% ee as determined by HPLC analysis (column: Daicel Chiralpak ODR 250 x 4.6 mm, Particle size: 10 µm, absorption: λ = 210 nm, mobile phase: CH₃CN/H₂O (0.1% TFA) = 40:60, flow rate: 1.0 mL/min, column temperature: 25 °C, retention times: tᵣ (major) = 29.8 min, tᵣ (minor) = 25.7 min). **[α]_{D}²²** = 22.4 (c = 1.0, MeOH). **¹H NMR** (300 MHz, MeOD) δ 7.77 - 7.60 (m, 4H), 4.74 (s, 2H), 1.99 (s, 3H). **¹³C NMR** (75 MHz, MeOD) δ 173.99, 154.01, 145.62, 129.95 (q, *J*_{C-F} = 32.3 Hz), 127.33, 125.27 (q, *J*_{C-F} = 3.8 Hz), 124.75 (q, *J*_{C-F} = 269.3 Hz), 96.16, 74.41, 62.22, 23.08. **¹⁹F NMR** (282 MHz, MeOD) δ -64.06. **IR (film):** v (cm⁻¹) 3332, 1713, 1497, 1323, 1058, 838, 731, 568 cm⁻¹. **HRMS (ESI, *m*/*z)*** calcd. for [C₁₃H₁₁Cl₃F₃NO₄Na] ⁺ [(M + Na) ⁺]: 429.9598, found: 429.9598.

### (S)-2-(4-(Methylthio)phenyl)-2-(((2,2,2-trichloroethoxy)carbonyl)amino)propanoic acid (61)

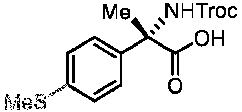

Starting from 2,2,2-trichloroethyl ((2-(4-(methylthio)phenyl)propanoyl)oxy)carbamate (77.0 mg, 0.20 mmol) according to the general procedure to provide **61** as a white solid (chromatography on silica gel, eluent: EtOAc/*n*-hexane = 1/5 with 0.1% TFA, 68.0 mg, 88% yield) and with 80% ee as determined by HPLC analysis (column: Daicel Chiralpak ODR 250 x 4.6 mm, Particle size: 10 µm, absorption: λ = 210 nm, mobile phase: CH₃CN/H₂O (0.1% TFA) = 40:60, flow rate: 1.0 mL/min, column temperature: 25 °C, retention times: tᵣ (major) = 35.3 min, tᵣ (minor) = 27.9 min). **[α]_{D}²²** = 4.8 (c = 1.0, MeOH). **¹H NMR** (300 MHz, MeOD) δ 7.49 - 7.40 (m, 2H), 7.27 - 7.19 (m, 2H), 4.73 (s, 2H), 2.45 (s, 3H), 1.95 (s, 3H). **¹³C NMR** (75 MHz, MeOD) δ 174.65, 153.93, 138.96, 137.67, 126.94, 126.38, 96.21, 74.34, 61.97, 22.57, 14.70. **IR (film):** v (cm⁻¹)3397, 1700, 1490, 1451, 1143, 1100, 820, 701, 560 cm⁻¹. **HRMS (ESI, *m*/*z)*** calcd. for [C₁₃H₁₄Cl₃NO₄SNa] ⁺ [(M + Na) ⁺]: 407.9601, found: 407.9601.

### (S)-2-(4-Methoxyphenyl)-2-(((2,2,2-trichloroethoxy)carbonyl)amino)propanoic acid (62)

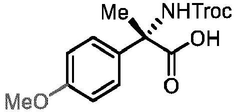

Starting from 2,2,2-trichloroethyl ((2-(4-methoxyphenyl)propanoyl)oxy)carbamate (73.6 mg, 0.20 mmol) according to the general procedure to provide **62** as a white solid (chromatography on silica gel, eluent: EtOAc/*n*-hexane = 1/5 with 0.1% TFA, 71.4 mg, 97% yield) and with 56% ee as determined by HPLC analysis (column: Daicel Chiralpak ODR 250 x 4.6 mm, Particle size: 10 µm, absorption: λ = 210 nm, mobile phase: CH₃CN/H₂O (0.1% TFA) = 40:60, flow rate: 1.0 mL/min, column temperature: 25 °C, retention times: tᵣ (major) = 22.8 min, tᵣ (minor) = 16.8 min). **[α]_{D}²²** = 17.4 (c = 1.0, MeOH). **¹H NMR** (300 MHz, MeOD) δ 7.49 - 7.39 (m, 2H), 6.94 - 6.84 (m, 2H), 4.78 - 4.65 (m, 2H), 3.77 (s, 3H), 1.95 (s, 3H). **¹³C NMR** (75 MHz, MeOD) δ 174.99, 159.85, 153.94, 132.85, 127.61, 113.82, 96.25, 74.32, 61.82, 54.87, 22.50. **IR (film):** v (cm⁻¹) 2954, 1709, 1510, 1249, 1180, 1097, 817, 721, 568 cm⁻¹. **HRMS (ESI, *m*/*z*)** calcd. for [C₁₃H₁₄Cl₃NO₅Na] ⁺ [(M + Na) ⁺]: 391.9830, found: 391.9830.

### (S)-2-(3-Phenoxyphenyl)-2-(((2,2,2-trichloroethoxy)carbonyl)amino)propanoic acid (63)

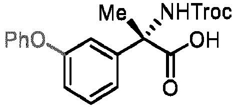

Starting from 2,2,2-trichloroethyl ((2-(3-phenoxyphenyl)propanoyl)oxy)carbamate (86.2 mg, 0.20 mmol) according to the general procedure to provide **63** as a white solid (chromatography on silica gel, eluent: EtOAc/*n*-hexane = 1/5 with 0.1% TFA, 71.0 mg, 82% yield) and with 77% ee as determined by HPLC analysis (column: Daicel Chiralpak IG 250 x 4.6 mm, Particle size: 5 µm, absorption: λ = 210 nm, mobile phase: CH₃CN/H₂O (0.1% TFA) = 40:60, flow rate: 1.0 mL/min, column temperature: 25 °C, retention times: tᵣ (major) = 78.9 min, tᵣ (minor) = 73.6 min). **[α]_{D}²²** = 36.6 (c = 1.0, MeOH). **¹H NMR** (300 MHz, MeOD) δ 7.38 - 7.24 (m, 4H), 7.21 - 7.15 (m, 1H), 7.09 (t, *J* = 7.4 Hz, 1H), 6.97 (d, *J=* 8.1 Hz, 2H), 6.93 - 6.84 (m, 1H), 4.80 - 4.63 (m, 2H), 1.94 (s, 3H). **¹³C NMR** (75 MHz, MeOD) δ 174.44, 157.71, 157.68, 153.87, 143.24, 130.46, 129.81, 123.55, 121.45, 118.92, 118.13, 117.26, 96.17, 74.31, 62.15, 22.86. **IR (film)**: v (cm⁻¹) 3333, 2953, 1709, 1485, 1243, 1101, 810, 690, 569 cm⁻¹. **HRMS (ESI, *m*/*z*)** calcd. for [C₁₈H₁₆Cl₃NO₅Na] ⁺ [(M + Na) ⁺]: 453.9986, found: 453.9986.

### (S)-2-(3-Benzoylphenyl)-2-(((2,2,2-trichloroethoxy)carbonyl)amino)propanoic acid (64)

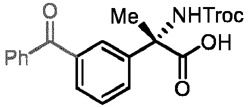

Starting from 2,2,2-trichloroethyl ((2-(3-benzoylphenyl)propanoyl)oxy)carbamate (88.6 mg, 0.20 mmol) according to the general procedure to provide **64** as a white solid (chromatography on silica gel, eluent: EtOAc/n-hexane = 1/5 with 0.1% TFA, 75.3 mg, 85% yield) and with 82% ee as determined by HPLC analysis (column: Daicel Chiralpak IG 250 x 4.6 mm, Particle size: 5 µm, absorption: λ = 210 nm, mobile phase: CH₃CN/H₂O (0.1% TFA) = 40:60, flow rate: 1.0 mL/min, column temperature: 25 °C, retention times: tᵣ (major) = 48.7 min, tᵣ (minor) = 44.8 min). **[α]_{D}²²** = 16.8 (c = 1.0, MeOH). **¹H NMR** (300 MHz, MeOD) δ 7.98 (s, 1H), 7.89 - 7.61 (m, 6H), 7.59 - 7.48 (m, 3H), 4.74 (s, 2H), 2.01 (s, 3H). **¹³C NMR** (75 MHz, MeOD) δ 197.34, 173.97, 154.00, 141.64, 137.78, 133.00, 130.98, 130.19, 129.46, 128.64, 128.16, 96.13, 74.33, 62.19, 23.24. **IR (film):** v (cm⁻¹) 3397, 2953, 1718, 1656, 1498, 1280, 1103, 818, 716, 569 cm⁻¹. **HRMS (ESI, *m*/*z)*** calcd. for [C₁₉H₁₆Cl₃NO₅Na] ⁺ [(M + Na) ⁺]: 465.9986, found: 465.9986.

### (S)-2-(2-Fluoro-[1,1'-biphenyl]-4-yl)-2-(((2,2,2-trichloroethoxy)carbonyl)amino)propanoic acid (65)

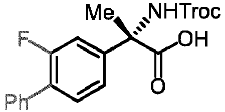

Starting from 2,2,2-trichloroethyl ((2-(2-fluoro-[1,1'-biphenyl]-4-yl)propanoyl)oxy)carbamate (86.6 mg, 0.20 mmol) according to the general procedure to provide 65 as a white solid (chromatography on silica gel, eluent: EtOAc/n-hexane = 1/5 with 0.1% TFA, 70.0 mg, 81% yield) and with 80% ee as determined by HPLC analysis (column: Daicel Chiralpak ODR 250 x 4.6 mm, Particle size: 10 µm, absorption: λ = 210 nm, mobile phase: CH₃CN/H₂O (0.1% TFA) = 50:50, flow rate: 1.0 mL/min, column temperature: 25 °C, retention times: tᵣ (major) = 19.7 min, tᵣ (minor) = 17.7 min). **[α]_{D}²²** = 94.3 (c = 1.0, MeOH). **¹H NMR** (300 MHz, MeOD) δ 7.69 - 7.27 (m, 8H), 4.76 (s, 2H), 1.99 (s, 3H). **¹³C NMR** (75 MHz, MeOD) δ 174.25, 159.89 (d, *J*_{C-F} = 244.5 Hz), 153.93, 142.87 (d, *J*_{C-F} = 7.2 Hz), 135.75 (d, *J*_{C-F} = 1.4 Hz), 130.69 (d, *J*_{C-F} = 3.9 Hz), 129.08 (d, *J*_{C-F} = 3.0 Hz), 128.61, 127.95, 122.66 (d, *J*_{C-F} = 3.6 Hz), 114.49 (d, *J*_{C-F}= 25.3 Hz), 96.18, 74.36, 61.92, **22.91.¹⁹F NMR** (282 MHz, MeOD) δ -119.76. **IR (film):** v (cm⁻¹) 3325, 2955, 1723, 1560, 1409, 1275, 1109, 720, 569 cm⁻¹. **HRMS (ESI, *m*/*z)*** calcd. for [C₁₈H₁₅Cl₃FNO₄Na] ⁺ [(M + Na) ⁺]: 455.9943, found: 455.9943.

### (5)-2-(3,5-Dimethylphenyl)-2-(((2,2,2-trichloroethoxy)carbonyl)amino)propanoic acid (66)

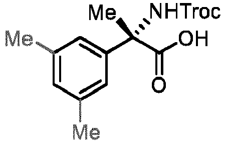

Starting from 2,2,2-trichloroethyl ((2-(3,5-dimethylphenyl)propanoyl)oxy)carbamate (73.4 mg, 0.20 mmol) according to the general procedure to provide **66** as a white solid (chromatography on silica gel, eluent: EtOAc/n-hexane = 1/5 with 0.1% TFA, 46.3 mg, 63% yield) and with 75% ee as determined by HPLC analysis (column: Daicel Chiralpak IG 250 x 4.6 mm, Particle size: 5 µm, absorption: λ = 210 nm, mobile phase: CH₃CN/H₂O (0.1% TFA) = 50:50, flow rate: 1.0 mL/min, column temperature: 25 °C, retention times: tᵣ (major) = 12.7 min, tᵣ (minor) = 9.6 min). **[α]_{D}²²** = 32.5 (c = 1.0, MeOH, 99% ee). **¹H NMR** (300 MHz, MeOD) δ 7.12 (s, 2H), 6.93 (s, 1H), 4.81 - 4.62 (m, 2H), 2.29 (s, 6H), 1.95

(s, 3H). **¹³C NMR** (75 MHz, MeOD) δ 174.89, 153.91, 140.82, 138.10, 129.42, 124.07, 96.29, 74.28, 62.19, 22.56, 20.65. **IR (film):** v (cm⁻¹) 3398, 2854, 1707, 1494, 1233, 1098, 809, 710, 568 cm⁻¹. **HRMS (ESI, *m*/*z*)** calcd. for [C₁₄H₁₆Cl₃NO₄Na]⁺ [(M + Na) ⁺]: 390.0037, found: 390.0037.

### (S)-2-(2,4-Difluorophenyl)-2-(((2,2,2-trichloroethoxy)carbonyl)amino)propanoic acid (67)

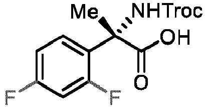

Starting from 2,2,2-trichloroethyl ((2-(2,4-difluorophenyl)propanoyl)oxy)carbamate (75.0 mg, 0.20 mmol) according to the general procedure to provide **67** as a white solid (chromatography on silica gel, eluent: EtOAc/n-hexane = 1/5 with 0.1% TFA, 52.0 mg, 69% yield) and with 83% ee as determined by HPLC analysis (column: Daicel Chiralpak ODR 250 x 4.6 mm, Particle size: 10 µm, absorption: λ = 210 nm, mobile phase: CH₃CN/H₂O (0.1% TFA) = 40:60, flow rate: 1.0 mL/min, column temperature: 25 °C, retention times: tᵣ (major) = 19.6 min, tᵣ (minor) = 16.9 min). **[α]_{D}²²** = 13.9 (c = 1.0, MeOH). **¹H NMR** (300 MHz, MeOD) δ 7.68 - 7.54 (m, 1H), 7.03 - 6.86 (m, 2H), 4.79 - 4.63 (m, 2H), 2.03 (s, 3H). **¹³C NMR** (126 MHz, MeOD) δ 173.70, 162.66 (dd, *J*_{C-F} = 248.2, 12.6 Hz), 161.67 (dd, *J*_{C-F} = 252.0, 12.6 Hz), 152.69, 129.96 (dd, *J*_{C-F} = 10.1, 5.0 Hz), 124.24 (dd, *J*_{C-F} = 12.6, 3.8 Hz), 110.02 (d, *J*_{C-F} = 22.9 Hz), 103.48 (t, *J*_{C-F} = 26.2 Hz), 95.68, 73.64, 58.78, **21.78.¹⁹F NMR** (282 MHz, MeOD) δ -109.53 (d, *J* = 8.7 Hz), -113.06 (d, *J=* 8.2 Hz). **IR (film):** v (cm⁻¹) 3400, 3086, 1716, 1501, 1274, 1099, 972, 814, 721, 568 cm⁻¹. **HRMS (ESI, *m*/*z)*** calcd. for [C₁₂H₁₀Cl₃F₂NO₄Na] ⁺ [(M + Na) ⁺]: 397.9536, found: 397.9536.

### (S)-2-(Benzo[d][1,3]dioxol-5-yl)-2-(((2,2,2-trichloroethoxy)carbonyl)amino)propanoic acid (68)

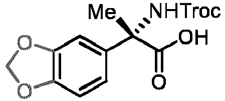

Starting from 2,2,2-trichloroethyl ((2-(benzo[d][1,3]dioxol-5-yl)propanoyl)oxy)carbamate (76.4 mg, 0.20 mmol) according to the general procedure to provide **68** as a white solid (chromatography on silica gel, eluent: EtOAc/n-hexane = 1/5 with 0.1% TFA, 58.0 mg, 76% yield) and with 68% ee as determined by HPLC analysis (column: Daicel Chiralpak ODR 250 x 4.6 mm, Particle size: 10 µm, absorption: λ = 210 nm, mobile phase: CH₃CN/H₂O (0.1% TFA) = 40:60, flow rate: 1.0 mL/min, column temperature: 25 °C, retention times: tᵣ (major) = 22.9 min, tᵣ (minor) = 18.6 min). **[α]_{D}²²** = 10.7 (c = 1.0, MeOH). **¹H NMR** (300 MHz, MeOD) δ 7.04 - 6.95 (m, 2H), 6.81 - 6.72 (m, 1H), 5.93 (s, 2H), 4.80 - 4.65 (m, 2H), 1.94 (s, 3H). **¹³C NMR** (75 MHz, MeOD) δ 174.40, 153.39, 147.82, 147.26, 134.34, 119.43, 107.36, 106.65, 101.19, 95.73, 73.82, 61.52, 22.09. **IR (film):** v (cm⁻¹) 3372, 2955, 1713, 1502, 1270, 1094, 1037, 819, 711, 563 cm⁻¹. **HRMS (ESI, *m*/*z)*** calcd. for [C₁₃H₁₂Cl₃NO₆Na] ⁺ [(M + Na) ⁺]: 405.9622, found: 405.9622.

### (S)-2-(Naphthalen-2-yl)-2-(((2,2,2-trichloroethoxy)carbonyl)amino)propanoic acid (69)

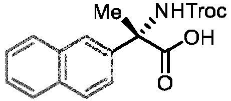

Starting from 2,2,2-trichloroethyl ((2-(naphthalen-2-yl)propanoyl)oxy)carbamate (78.0 mg, 0.20 mmol) according to the general procedure to provide **69** as a white solid (chromatography on silica gel, eluent: EtOAc/n-hexane = 1/5 with 0.1% TFA, 70.0 mg, 90% yield) and with 84% ee as determined by HPLC analysis (column: Daicel Chiralpak ODR 250 x 4.6 mm, Particle size: 10 µm, absorption: λ = 210 nm, mobile phase: CH₃CN/H₂O (0.1% TFA) = 50:50, flow rate: 1.0 mL/min, column temperature: 25 °C, retention times: tᵣ (major) = 14.2 min, tᵣ (minor) = 12.6 min). **[α]_{D}²²** = 34.7 (c = 1.0, MeOH, 99% ee). **¹H NMR** (300 MHz, MeOD) δ 8.00 (d, *J=* 2.1 Hz, 1H), 7.88 - 7.76 (m, 3H), 7.64 (dd, *J=* 8.8, 2.1 Hz, 1H), 7.51 - 7.42 (m, 2H), 4.82 - 4.66 (m, 2H), 2.09 (s, 3H). **¹³C NMR** (75 MHz, MeOD) δ 174.73, 154.01, 138.33, 133.64, 133.36, 128.39, 128.16, 127.57, 126.47, 126.41, 125.49, 124.33, 96.26, 74.34, 62.45, 22.72. **IR (film):** v (cm⁻¹) 3335, 3023, 1730, 1502, 1289, 1106, 860, 737, 569, 473 cm⁻¹. **HRMS (ESI, *m*/*z)*** calcd. for [C₁₆H₁₄Cl₃NO₄Na] ⁺ [(M + Na) ⁺]: 411.9881, found: 411.9881.

### (S)-2-(6-Methoxynaphthalen-2-yl)-2-(((2,2,2-trichloroethoxy)carbonyl)amino)propanoic acid (70)

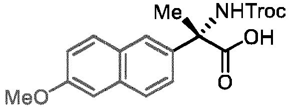

Starting from 2,2,2-trichloroethyl ((2-(6-methoxynaphthalen-2-yl)propanoyl)oxy)carbamate (83.8 mg, 0.20 mmol) according to the general procedure to provide **70** as a white solid (chromatography on silica gel, eluent: EtOAc/n-hexane = 1/5 with 0.1% TFA, 82.9 mg, 99% yield) and with 64% ee as determined by HPLC analysis (column: Daicel Chiralpak IG 250 x 4.6 mm, Particle size: 5 µm, absorption: λ = 210 nm, mobile phase: CH₃CN/H₂O (0.1% TFA) = 50:50, flow rate: 1.0 mL/min, column temperature: 25 °C, retention times: tᵣ (major) = 21.3 min, tᵣ (minor) = 15.4 min). **[α]_{D}²²** =49.4 (c = 1.0, MeOH). **¹H NMR** (300 MHz, MeOD) δ 7.91 (d, *J=* 2.2 Hz, 1H), 7.73 (dd, *J=* 9.0, 2.1 Hz, 2H), 7.58 (dd, *J=* 8.8, 2.1 Hz, 1H), 7.18 (d, *J=* 2.6 Hz, 1H), 7.11 (dd, J= 8.9, 2.5 Hz, 1H), 4.81 - 4.66 (m, 2H), 3.87 (s, 3H), 2.07 (s, 3H). **¹³C NMR** (75 MHz, MeOD) δ 174.87, 158.68, 154.00, 135.92, 134.64, 129.83, 129.05, 127.16, 125.27, 124.73, 119.19, 105.60, 96.28, 74.34, 62.34, 54.89, 22.61. **IR (film):** v (cm⁻¹) 3333, 2951, 1700, 1452, 1264, 1051, 812, 733, 567 cm⁻¹. **HRMS (ESI, *m*/*z)*** calcd. for [C₁₇H₁₆Cl₃NO₅Na] ⁺ [(M + Na) ⁺]: 441.9986, found: 441.9986.

### (S)-2-(Thiophen-3-yl)-2-(((2,2,2-trichloroethoxy)carbonyl)amino)propanoic acid (71)

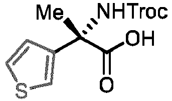

Starting from 2,2,2-trichloroethyl ((2-(thiophen-3-yl)propanoyl)oxy)carbamate (69.0 mg, 0.20 mmol) according to the general procedure to provide **71** as an oil (chromatography on silica gel, eluent: EtOAc/n-hexane = 1/5 with 0.1% TFA, 65.0 mg, 94% yield) and with 83% ee as determined by HPLC analysis (column: Daicel Chiralpak IG 250 x 4.6 mm, Particle size: 5 µm, absorption: λ = 210 nm, mobile phase: CH₃CN/H₂O (0.1% TFA) = 40:60, flow rate: 1.0 mL/min, column temperature: 25 °C, retention times: tᵣ (major) = 20.6 min, tᵣ (minor) = 19.2 min). **[α]_{D}²²** = 13.8 (c = 1.0, MeOH). **¹H NMR** (300 MHz, MeOD) δ 7.47 - 7.40 (m, 1H), 7.40 - 7.31 (m, 1H), 7.20 (dd, *J=* 5.1, 1.4 Hz, 1H), 4.83 - 4.66 (m, 2H), 1.94 (s, 3H). **¹³C NMR** (75 MHz, MeOD) δ 174.43, 154.14, 142.15, 126.61, 125.74, 122.42, 96.19, 74.38, 60.30, 23.57. **IR (film):** v (cm⁻¹) 3394, 2953, 1701, 1452, 1195, 1125, 1102, 796, 722, 568 cm⁻¹. **HRMS (ESI, *m*/*z)*** calcd. for [C₁₀H₁₀Cl₃NO₄SNa] ⁺ [(M + Na) ⁺]: 367.9288, found: 367.9288.

### (S)-2-(Quinolin-6-yl)-2-(((2,2,2-trichloroethoxy)carbonyl)amino)propanoic acid (72)

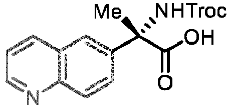

Starting from 2,2,2-trichloroethyl ((2-(quinolin-6-yl)propanoyl)oxy)carbamate (78.0 mg, 0.20 mmol) according to the general procedure to provide **72** as a white solid (chromatography on silica gel, eluent: EtOAc/n-hexane = 1/5 with 0.1% TFA, 67.0 mg, 86% yield) and with 70% ee as determined by HPLC analysis (column: Daicel Chiralpak ODR 250 x 4.6 mm, Particle size: 10 µm, absorption: λ = 210 nm, mobile phase: CH₃CN/H₂O (0.1% TFA) = 20:80, flow rate: 1.0 mL/min, column temperature: 25 °C, retention times: tᵣ (major) = 8.4 min, tᵣ (minor) = 7.5 min). **[α]_{D}²²** = 14.1 (*c* = 1.0, MeOH). **¹H NMR** (300 MHz, MeOD) δ 9.20 - 9.07 (m, 2H), 8.47 (s, 1H), 8.37 - 8.19 (m, 2H), 8.03 (dd, *J* = 8.4, 5.4 Hz, 1H), 4.74 (s, 2H), 2.10 (s, 3H). **¹³C NMR**(75 MHz, MeOD) δ 173.61, 154.14, 146.88, 145.65, 143.75, 138.67, 133.99, 129.36, 126.97, 122.37, 121.35, 96.12, 74.42, 62.36, 23.52. **IR (film):** v (cm⁻¹) 3367, 2953, 1669, 1385, 1183, 1100, 799, 718, 568 cm⁻¹. **HRMS (ESI, *m*/*z)*** calcd. for [C₁₅H₁₃Cl₃N₂O₄H] ⁺ [(M + H) ⁺]: 391.0014, found: 391.0014.

### (S)-2-(6-Chloro-9H-carbazol-2-yl)-2-(((2,2,2-trichloroethoxy)carbonyl)amino)propanoic acid (73)

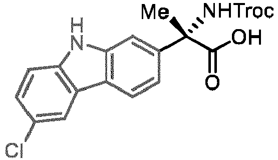

Starting from 2,2,2-trichloroethyl ((2-(6-chloro-9*H*-carbazol-2-yl)propanoyl)oxy)carbamate (92.4 mg, 0.20 mmol) according to the general procedure to provide **73** as a white solid (chromatography on silica gel, eluent: EtOAc/n-hexane = 1/5 with 0.1% TFA, 24.1 mg, 26% yield) and with 72% ee as determined by HPLC analysis (column: Daicel Chiralpak IG 250 x 4.6 mm, Particle size: 5 µm, absorption: λ = 210 nm, mobile phase: CH₃CN/H₂O (0.1% TFA) = 50:50, flow rate: 1.0 mL/min, column temperature: 25 °C, retention times: tᵣ (major) = 40.1 min, tᵣ (minor) = 29.2 min). **[α]_{D}²²** = 10.8 (*c* = 1.0, MeOH). **¹H NMR** (300 MHz, MeOD) δ 8.04 - 7.96 (m, 2H), 7.65 (d, *J=* 1.8 Hz, 1H), 7.44 - 7.27 (m, 3H), 4.81 - 4.68 (m, 2H), 2.09 (s, 3H). **¹³C NMR** (126 MHz, MeOD) δ 174.52, 153.47, 140.64, 138.94, 138.75, 125.24, 123.88, 123.72, 121.58, 119.71, 119.32, 116.95, 111.56, 108.69, 95.74, 73.84, 62.20, 22.23. **IR (film):** *v* (cm⁻¹) 3416, 2923, 2532, 1699, 1468, 1270, 1097, 809, 692, 563 cm⁻¹. **HRMS (ESI, *m*/*z*)** calcd. for [C₁₈H₁₄Cl₄N₂O₄Na] ⁺ [(M + Na) ⁺]: 484.9600, found: 484.9600.

### (5)-2-Phenyl-2-(((2,2,2-trichloroethoxy)carbonyl)amino)butanoic acid (74)

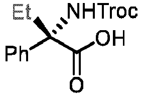

Starting from 2,2,2-trichloroethyl ((2-phenylbutanoyl)oxy)carbamate (70.6 mg, 0.20 mmol) according to the general procedure to provide **74** as a colorless oil (chromatography on silica gel, eluent: EtOAc/n-hexane = 1/5 with 0.1% TFA, 60.0 mg, 85% yield) and with 83% ee as determined by HPLC analysis (column: Daicel Chiralpak ODR 250 x 4.6 mm, Particle size: 10 µm, absorption: λ = 210 nm, mobile phase: CH₃CN/H₂O(0.1% TFA) = 50:50, flow rate: 1.0 mL/min, column temperature: 25 °C, retention times: tᵣ (major) = 12.4 min, tᵣ (minor) = 9.9 min). **[α]_{D}²²** = 66.5 (c = 1.0, MeOH). **¹H NMR** (300 MHz, MeOD) δ 7.55 - 7.47 (m, 2H), 7.38 - 7.20 (m, 3H), 4.81 - 4.64 (m, 2H), 2.70 - 2.47 (m, 2H), 0.92 (t, *J* = 7.2 Hz, 3H). **¹³C NMR** (75 MHz, MeOD) δ 174.06, 153.12, 140.61, 128.42, 127.78, 126.46, 96.31, 74.14, 66.23, 26.58, 7.88. **IR (film):** v (cm⁻¹) 3398, 2960, 1712, 1493, 1406, 1232, 1105, 725, 696, 568 cm⁻¹. **HRMS (ESI, *m*/*z)*** calcd. for [C₁₃H₁₄Cl₃NO₄Na] ⁺ [(M + Na) ⁺]: 375.9881, found: 375.9881.

### (S)-2-Phenyl-2-(((2,2,2-trichloroethoxy)carbonyl)amino)pentanoic acid (75)

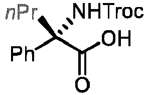

Starting from 2,2,2-trichloroethyl ((2-phenylpentanoyl)oxy)carbamate (73.4 mg, 0.20 mmol) according to the general procedure to provide **75** as a white solid (chromatography on silica gel, eluent: EtOAc/n-hexane = 1/5 with 0.1% TFA, 67.0 mg, 91% yield) and with 82% ee as determined by HPLC analysis (column: Daicel Chiralpak IG 250 x 4.6 mm, Particle size: 5 µm, absorption: λ = 210 nm, mobile phase: CH₃CN/H₂O (0.1% TFA) = 40:60, flow rate: 1.0 mL/min, column temperature: 25 °C, retention times: tᵣ (major) = 26.7 min, tᵣ (minor) = 30.2 min). **[α]_{D}²²** = 9.83 (*c* = 1.0, MeOH). **¹H NMR** (300 MHz, MeOD) δ 7.55 - 7.47 (m, 2H), 7.39 - 7.21 (m, 3H), 4.81 - 4.65 (m, 2H), 2.66 - 2.42 (m, 2H), 1.54 - 1.36 (m, 1H), 1.34 - 1.16 (m, 1H), 0.99 (t, *J* = 7.4 Hz, 3H). **¹³C NMR** (75 MHz, MeOD) δ 174.18, 153.14, 140.80, 128.42, 127.77, 126.39, 96.34, 74.15, 65.65, 35.88, 17.74, 13.54. **IR (film):** *v* (cm⁻¹) 3397, 2961, 1710, 1493, 1403, 1227, 1107, 1054, 720, 695, 568 cm⁻¹. **HRMS (ESI, *m*/*z)*** calcd. for [C₁₄H₁₆Cl₃NO₄Na] ⁺ [(M + Na) ⁺]: 390.0037, found: 390.0037.

### (S)-2-Phenyl-2-(((2,2,2-trichloroethoxy)carbonyl)amino)octanoic acid (76)

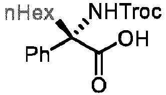

Starting from 2,2,2-trichloroethyl ((2-phenyloctanoyl)oxy)carbamate (81.8 mg, 0.20 mmol) according to the general procedure to provide **76** as a white solid (chromatography on silica gel, eluent: EtOAc/n-hexane = 1/5 with 0.1% TFA, 74.0 mg, 90% yield) and with 82% ee as determined by HPLC analysis (column: Daicel Chiralpak IG 250 x 4.6 mm, Particle size: 5 µm, absorption: λ = 210 nm, mobile phase: CH₃CN/H₂O (0.1% TFA) = 50:50, flow rate: 1.0 mL/min, column temperature: 25 °C, retention times: tᵣ (major) = 24.2 min, tᵣ (minor) = 21.3 min). **[α]_{D}²²** = 23.8 (c = 1.0, MeOH). **¹H NMR** (300 MHz, MeOD) δ 7.55 - 7.47 (m, 2H), 7.38 - 7.20 (m, 3H), 4.83 - 4.63 (m, 2H), 2.71 - 2.44 (m, 2H), 1.48 - 1.15 (m, 8H), 0.95 - 0.85 (m, 3H). **¹³C NMR** (75 MHz, MeOD) δ 174.16, 153.05, 140.76, 128.42, 127.77, 126.37, 96.36, 74.10, 65.63, 33.52, 31.94, 29.40, 24.30, 22.67, 13.53. **IR (film):** v (cm⁻¹) 3428, 2930, 1752, 1690, 1484, 1146, 922, 906, 723, 691, 571, 464 cm⁻¹. **HRMS (ESI, *m*/*z)*** calcd. for [C₁₇H₂₂Cl₃NO₄Na] ⁺ [(M + Na) ⁺]: 432.0507, found: 432.0507.

### (S)-3-Methyl-2-phenyl-2-(((2,2,2-trichloroethoxy)carbonyl)amino)butanoic acid (77)

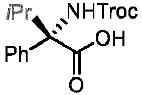

Starting from 2,2,2-trichloroethyl ((3-methyl-2-phenylbutanoyl)oxy)carbamate (73.4 mg, 0.20 mmol) according to the general procedure to provide **77** as a white solid (chromatography on silica gel, eluent: EtOAc/n-hexane = 1/5 with 0.1% TFA, 65.0 mg, 88% yield) and with 88% ee as determined by HPLC analysis (column: Daicel Chiralpak IG 250 x 4.6 mm, Particle size: 5 µm, absorption: λ = 210 nm, mobile phase: CH₃CN/H₂O(0.1% TFA) = 40:60, flow rate: 1.0 mL/min, column temperature: 25 °C, retention times: tᵣ (major) = 21.7 min, tᵣ (minor) = 30.6 min). **[α]_{D}²²** = 5.9 (c = 1.0, MeOH). **¹H NMR** (300 MHz, MeOD) δ 7.57 (d, *J=* 7.4 Hz, 2H), 7.36 - 7.19 (m, 3H), 4.77 (d, *J=* 12.0 Hz, 1H), 4.69 (d, *J* = 12.0 Hz, 1H), 2.86 - 2.67 (m, 1H), 0.96 (d, *J* = 6.0 Hz, 3H), 0.92 (d, *J* = 6.0 Hz, 3H). **¹³C NMR** (75 MHz, MeOD) δ 173.79, 154.45, 138.84, 127.82, 127.69, 127.31, 96.35, 74.37, 69.56, 35.50, 17.51. **IR (film):** v (cm⁻¹) 3285, 2972, 1704, 1400, 1321, 1121, 773, 704, 570 cm⁻¹. **HRMS (ESI, *m*/*z)*** calcd. for [C₁₄H₁₆Cl₃NO₄Na] ⁺ [(M + Na) ⁺]: 390.0037, found: 390.0037.

### (S)-2-Cyclopentyl-2-phenyl-2-(((2,2,2-trichloroethoxy)carbonyl)amino)acetic acid (78)

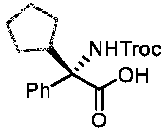

Starting from 2,2,2-trichloroethyl (2-cyclopentyl-2-phenylacetoxy)carbamate (78.6 mg, 0.20 mmol) according to the general procedure to provide **78** as a white solid (chromatography on silica gel, eluent: EtOAc/n-hexane = 1/5 with 0.1% TFA, 34.6 mg, 44% yield) and with 82% ee as determined by HPLC analysis (column: Daicel Chiralpak IG 250 x 4.6 mm, Particle size: 5 µm, absorption: λ = 210 nm, mobile phase: CH₃CN/H₂O (0.1% TFA) = 45:55, flow rate: 1.0 mL/min, column temperature: 25 °C, retention times: tᵣ (major) = 21.3 min, tᵣ (minor) = 27.3 min). **[α]_{D}²²** =2.3 (*c* = 1.0, MeOH). **¹H NMR** (300 MHz, MeOD) δ 7.61 - 7.53 (m, 2H), 7.35 - 7.18 (m, 3H), 4.76 (d, *J=* 12.0 Hz, 1H), 4.68 (d, *J* = 12.0 Hz, 1H), 3.14 - 2.82 (m, 1H), 1.82 - 1.42 (m, **8H). ¹³C NMR** (75 MHz, MeOD) δ 174.01, 154.28, 139.95, 127.95, 127.46, 127.34, 96.34, 74.34, 68.04, 27.99, 27.84, 25.38, 25.28. **IR (film):** *v* (cm⁻¹) 3286, 2953, 1709, 1494, 1399, 1200, 1106, 766, 699, 569 cm⁻¹. **HRMS (ESI, *m*/*z)*** calcd. for [C₁₆H₁₈Cl₃NO₄Na] ⁺ [(M + Na) ⁺]: 416.0194, found: 416.0194.

### (S)-2-Cyclohexyl-2-phenyl-2-(((2,2,2-trichloroethoxy)carbonyl)amino)acetic acid (79)

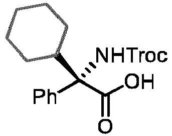

Starting from 2,2,2-trichloroethyl (2-cyclohexyl-2-phenylacetoxy)carbamate (81.4 mg, 0.20 mmol) according to the general procedure to provide **79** as a white solid (chromatography on silica gel, eluent: EtOAc/n-hexane = 1/5 with 0.1% TFA, 49.6 mg, 61% yield) and with 82% ee as determined by HPLC analysis (column: Daicel Chiralpak IB 250 x 4.6 mm, Particle size: 5 µm, absorption: λ = 210 nm, mobile phase: CH₃CN/H₂O (0.1% TFA) = 50:50, flow rate: 1.0 mL/min, column temperature: 25 °C, retention times: tᵣ (major) = 16.8 min, tᵣ (minor) = 18.3 min). **[α]_{D}²²** = 6.2 (*c* = 1.0, MeOH). **¹H NMR** (300 MHz, MeOD) δ 7.59 - 7.51 (m, 2H), 7.35 - 7.18 (m, 3H), 4.77 (d, *J* = 12.0 Hz, 1H), 4.69 (d, *J =* 12.0 Hz, 1H), 2.43 - 2.30 (m, 1H), 1.85 - 1.60 (m, 5H), 1.38 - 1.19 (m, 2H), 1.07 - 1.05 (m, 3H). ¹³C **NMR** (75 MHz, MeOD) δ 173.56, 154.44, 138.66, 127.79, 127.67, 127.25, 96.37, 74.35, 69.44, 46.01, 28.45, 26.90, 26.82, 26.54. **IR (film):** *v* (cm⁻¹) 3280, 2925, 1712, 1399, 1243, 1057, 887, 765, 651, 463 cm⁻¹. **HRMS (ESI, *m*/*z)*** calcd. for [C₁₇H₂₀Cl₃NO₄Na] ⁺ [(M + Na) ⁺]: 430.0350, found: 430.0350.

### (S)-1-(((2,2,2-Trichloroethoxy)carbonyl)amino)-1,2,3,4-tetrahydronaphthalene-1-carboxylic acid (80)

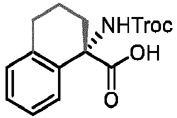

Starting from 2,2,2-trichloroethyl ((1,2,3,4-tetrahydronaphthalene-1-carbonyl)oxy)carbamate (73.0 mg, 0.20 mmol) according to the general procedure to provide **80** as a white solid (chromatography on silica gel, eluent: EtOAc/n-hexane = 1/5 with 0.1% TFA, 41.0 mg, 56% yield) and with 68% ee as determined by HPLC analysis (column: Daicel Chiralpak ODR 250 x 4.6 mm, Particle size: 10 µm, absorption: λ = 210 nm, mobile phase: CH₃CN/H₂O (0.1% TFA) = 40:60, flow rate: 1.0 mL/min, column temperature: 25 °C, retention times: tᵣ (major) = 30.9 min, tᵣ (minor) = 27.6 min). **[α]_{D}²²** = 11.2 (c = 1.0, MeOH). **¹H NMR** (300 MHz, MeOD) δ 7.46 (d, *J* = 7.7 Hz, 1H), 7.25 - 7.09 (m, 3H), 4.77 (d, *J* = 12.0 Hz, 1H), 4.69 (d, *J* = 12.0 Hz, 1H), 2.93 - 2.73 (m, 2H), 2.63 - 2.51 (m, 1H), 2.44 - 2.32 (m, 1H), 2.22 - 1.97

(m, 1H), 1.95 - 1.80 (m, 1H). **¹³C NMR** (75 MHz, MeOD) δ 175.21, 153.76, 138.73, 134.95, 129.60, 128.23, 127.53, 126.46, 96.31, 74.30, 61.36, 31.56, 29.53, 19.70. **IR (film):** *v* (cm⁻¹) 3421, 2923, 1703, 1454, 1213, 1058, 721, 569 cm⁻¹. **HRMS (ESI, *m*/*z)*** calcd. for [C₁₄H₁₄Cl₃NO₄Na] ⁺ [(M + Na) ⁺]: 387.9881, found: 387.9881.

### (2S,6R)-6-((3R,8R,9S,10S,13R,14S,17R)-3-((tert-Butyldimethylsilyl)oxy)-10,13-dimethylhexadecahydro-1H-cyclopenta[a]phenanthren-17-yl)-2-phenyl-2-(((2,2,2-trichloroethoxy)carbonyl)amino)heptanoic acid (81)

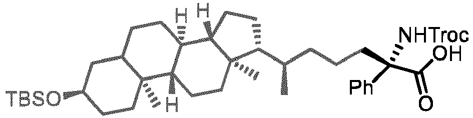

Starting from 2,2,2-trichloroethyl (((6*R*)-6-((3*R*,8*R*,9*S*,10*S*,13*R*,14*S*,17*R*)-3-((*tert*butyldimethylsilyl)oxy)-10,13-dimethylhexadecahydro-1*H*-cyclopenta[*a*]phenanthren-17-yl)-2-phenylheptanoyl)oxy)carbamate (153.4 mg, 0.20 mmol) according to the general procedure to provide **81** (dr: 90/10) as a white solid (chromatography on silica gel, eluent: EtOAc/*n*-hexane = 1/5 with 0.1% TFA, 63.0 mg, 41% yield) and with 90:10 dr as determined by HPLC analysis (column: Daicel Chiralpak ODR250 x 4.6 mm, Particle size: 10 µm, absorption: λ = 210 nm, mobile phase: CH₃CN/H₂O (0.1% TFA) = 80:20, flow rate: 1.0 mL/min, column temperature: 25 °C, retention times: tᵣ (major) = 19.9 min, tᵣ (minor) = 16.1 min). **[α]_{D}²²** = 25.6 (c = 1.0, MeOH). **¹H NMR** (300 MHz, MeOD) δ 7.89 - 7.80 (m, 2H), 7.73 - 7.55 (m, 3H), 5.19 - 4.95 (m, 2H), 4.05 - 3.83 (m, 1H), 2.96 - 2.76 (m, 2H), 2.44 - 2.08 (m, 5H), 1.96 - 1.42 (m, 24H), 1.32 - 1.23 (m, 14H), 1.04 (s, 3H), 0.42 (s, 6H). **¹³C NMR** (75 MHz, MeOD) δ 173.98, 152.87, 140.66, 128.27, 127.61, 126.21, 96.25, 73.97, 73.08, 71.39, 65.53, 56.89, 56.40, 42.86, 42.55, 40.86, 40.55, 37.08, 36.22, 36.02, 35.88, 35.55, 34.63, 33.77, 31.10, 30.17, 28.35, 27.38, 26.67, 25.45, 25.32, 24.27, 22.98, 20.95, 20.54, 18.24, 18.00, 11.57, -5.35, -6.75. **IR (film):** *v* (cm⁻¹) 3398, 2929, 1716, 1491, 1250, 1107, 1010, 800, 696, 568 cm⁻¹. **HRMS (ESI, *m*/*z*)** calcd. for [C₁₄H₁₄Cl₃NO₄Na] ⁺ [(M + Na) ⁺]: 387.9881, found: 387.9881.

### (E)-2-Methyl-4-phenyl-2-(((2,2,2-trichloroethoxy)carbonyl)amino)but-3-enoic acid (82)

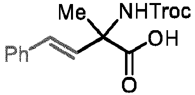

Starting from 2,2,2-trichloroethyl (E)-((2-methyl-4-phenylbut-3-enoyl)oxy)carbamate (73.0 mg, 0.20 mmol) according to the general procedure to provide **82** as a white solid (chromatography on silica gel, eluent: EtOAc/n-hexane = 1/5 with 0.1% TFA, 38.3 mg, 52% yield) and with 5% ee as determined by HPLC analysis (column: Daicel Chiralpak IG 250 x 4.6 mm, Particle size: 5 µm, absorption: λ = 210 nm, mobile phase: CH₃CN/H₂O (0.1% TFA) = 45:55, flow rate: 1.0 mL/min, column temperature: 25 °C, retention times: tᵣ (major) = 15.7 min, tᵣ (minor) = 17.6 min). **¹H NMR** (300 MHz, MeOD) δ 7.43 - 7.17 (m, 5H), 6.62 (s, 2H), 4.82 - 4.72 (m, 2H), 1.68 (s, 3H). **¹³C NMR** (75 MHz, MeOD) δ 175.14, 154.46, 137.12, 129.87, 128.77, 128.61, 127.96, 126.73, 96.34, 74.48, 60.61, 23.70. **IR (film):** *v* (cm⁻¹) 3397, 2961, 1710, 1493, 1267, 1107, 802, 720, 568 cm⁻¹. **HRMS (ESI, *m*/*z)*** calcd. for [C₁₄H₁₄Cl₃NO₄Na] ⁺ [(M + Na) ⁺]: 387.9881, found: 387.9881.

### 2-Cyclohexyl-2-(((2,2,2-trichloroethoxy)carbonyl)amino)propanoic acid (83)

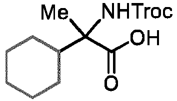

Starting from 2,2,2-trichloroethyl ((2-cyclohexylpropanoyl)oxy)carbamate (69.0 mg, 0.20 mmol) according to the general procedure to provide **83** as a white solid (chromatography on silica gel, eluent: EtOAc/*n*-hexane = 1/5 with 0.1% TFA, 29.0 mg, 42% yield) and with 10% ee as determined by HPLC analysis (column: Daicel Chiralpak IG 250 x 4.6 mm, Particle size: 5 µm, absorption: λ = 210 nm, mobile phase: CH₃CN/H₂O (0.1% TFA) = 50:50, flow rate: 1.0 mL/min, column temperature: 25 °C, retention times: tᵣ (major) = 16.2 min, tᵣ (minor) = 21.7 min). **¹H NMR** (300 MHz, MeOD) 4.80 (d, *J=* 12.0 Hz, 1H), 4.72 (d, *J=* 12.0 Hz, 1H), 1.90 - 1.60 (m, 6H), 1.49 (s, 3H), 1.39 - 1.03 (m, 5H). **¹³C NMR** (75 MHz, MeOD) δ 175.92, 154.65, 96.32, 74.36, 63.27, 44.51, 27.95, 27.56, 26.80, 26.77, 26.52, 18.38. **IR (film):** *v* (cm⁻¹) 2929, 1706, 1403, 1275, 1102, 729, 570 cm⁻¹. **HRMS (ESI, *m*/*z)*** calcd. for [C₁₂H₁₈Cl₃NO₄Na] ⁺ [(M + Na) ⁺]: 368.0194, found: 368.0194.

### 7. Iron-Catalyzed 1,3-Nitrogen Migration to Access N-Boc-Protected α-Monosubstituted and α,α-Disubstituted α-Amino Acids

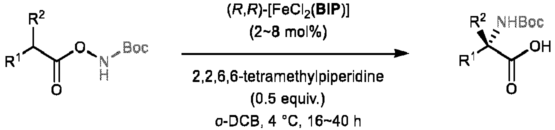

General procedures: to a Schlenk tube was added the substrate (1 equiv.) and (*R*,*R*)-[FeCl₂(**BIP**)] (2~8 mol%). 1,2-Dichlorobenzene (o-DCB, 0.1 M) and 2,2,6,6-tetramethylpiperidine (0.5 equiv.) was added, and the mixture was degassed via freeze-pump-thaw for two times. The tube was sealed, and the reaction mixture was stirred at 4 °C for 16~40 hours. To quench the reaction, brine (10 mL) and concentrated hydrochloric acid (1 mL/mmol substrate) was added, and the mixture was diluted with water (1 mL). The mixture was extracted with EtOAc for three times and the combined organic layer was dried over anhydrous sodium sulfate. After filtration, the solvent was evaporated under reduced pressure, and the residue was purified by column chromatography on silica gel using indicated solvent as the eluent. The data of three representatives are shown below.

### (S)-2-((tert-Butoxycarbonyl)amino)-2-phenylacetic acid (84)

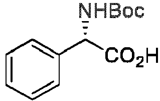

Catalyzed by 2 mol% (*R*,*R*)-[FeCl₂(**BIP**)], the 1,3-nitrogen shift of *tert*-butyl (2-phenylacetoxy)carbamate (50.3 mg, 0.2 mmol) gave **84** as a white gum (chromatography on silica gel, eluent: EtOAc/n-hexane = 1/6 with 0.2% HOAc, 45.9 mg, 91% yield): ¹H NMR (300 MHz, MeOD) δ 7.44-7.19 (m, 5H), 5.15 (s, 1H), 1.40 (s, 9H). ¹³C NMR (75 MHz, MeOD) δ 174.3, 157.6, 138.8, 129.8, 129.3, 128.7, 81.0, 59.3, 28.8. HRMS (ESI) calculated for [C₁₃H₁₇NNaO₄]⁺ [M + Na]⁺: 274.1050, found: 274.1061. Enantiomeric excess was established by HPLC analysis using a Chiralpak IG column, ee = 90% (HPLC: 210 nm, n-hexane/iPrOH = 90/10 + 0.1% TFA, flow rate 1.0 mL/min, 25 °C, tr (major) = 21.0 min, tr (minor) = 14.3 min). [α]_{D}²⁵ = +95.0 (c 1.0, MeOH).

### (S)-2-((tert-Butoxycarbonyl)amino)-5-phenylpentanoic acid (108)

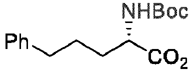

Catalyzed by 5 mol% (*R*,*R*)-[FeCl₂(**BIP**)], the 1,3-nitrogen shift of *tert*-butyl ((5-phenylpentanoyl)oxy)carbamate (58.7 mg, 0.2 mmol) gave **108** as a colorless gum (chromatography on silica gel, eluent: EtOAc/n-hexane = 1/8 with 0.2% HOAc, 39.8 mg, 68% yield): ¹H NMR (300 MHz, MeOD) δ 7.31-7.09 (m, 5H), 4.19-3.91 (m, 1H), 2.63 (t, *J* = 6.5 Hz, 2H), 1.89-1.58 (m, 4H), 1.44 (s, 9H). ¹³C NMR (75 MHz, MeOD) δ 176.4, 158.3, 143.4, 129.6, 129.5, 127.0, 80.6, 54.8, 36.4, 32.6, 29.0, 28.9. HRMS (ESI) calculated for [C₁₆H₂₃NNaO₄]⁺ [M + Na]⁺: 316.1519, found: 316.1519. Enantiomeric excess was established by HPLC analysis using a Chiralpak IG column, ee = 92% (HPLC: 210 nm, n-hexane/iPrOH = 90/10 + 0.1% TFA, flow rate 1.0 mL/min, 25 °C, tr (major) = 20.1 min, tr (minor) = 13.6 min). [α]_{D}²⁵ = +4.5 (c 1.0, MeOH).

### (S)-2-((tert-Butoxycarbonyl)amino)-2-phenylacetic acid (118)

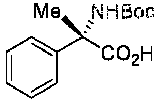

Catalyzed by 5 mol% (*R*,*R*)-[FeCl₂(**BIP**)], the 1,3-nitrogen shift of tert-butyl ((2-phenylpropanoyl)oxy)carbamate (53.1 mg, 0.2 mmol) gave **118** as a colorless gum (chromatography on silica gel, eluent: EtOAc/n-hexane = 1/6 with 0.2% HOAc, 32.0 mg, 60% yield): ¹H NMR (300 MHz, MeOD) δ 7.69-7.10 (m, 5H), 1.92 (s, 3H), 1.39 (s, 9H). ¹³C NMR (75 MHz, MeOD) δ 176.3, 156.8, 142.9, 129.4, 128.7, 127.3, 80.9, 63.0, 28.8, 24.3. HRMS (ESI) Exact mass calculated for [C₁₃H₁₇NNaO₄]⁺ [M + Na]⁺: 288.1206, found: 288.1216. Enantiomeric excess was established by HPLC analysis using a Chiralpak IG column, ee = 87% (HPLC: 210 nm, n-hexane/iPrOH = 90/10 + 0.1% TFA, flow rate 1.0 mL/min, 25 °C, tr (major) = 7.9 min, tr (minor) = 13.2 min). [α]_{D}²⁵ = +57.5° (c 1.0, MeOH).

### 8. Iron-Catalyzed 1,3-Nitrogen Migration to Access N-Boc-Protected α-Deuterated α-Amino Acids

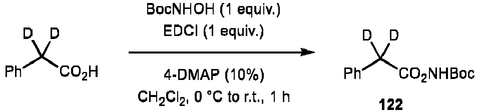

EDCI coupling of 2-phenylacetic-2,2-*d*₂ acid (synthesized by a literature procedure³) with BocNHOH gave **122** as a white gum (chromatography on silica gel, eluent: EtOAc/hexane = 1/6): ¹H NMR (300 MHz, CDCl₃) δ 8.06 (s, 1H), 7.41-7.24 (m, 5H), 1.45 (s, 9H). ¹³C NMR (75 MHz, CDCl₃) δ 170.7, 155.4, 132.2, 129.2, 128.6, 127.4, 83.1, 38.2 (m), 27.8. HRMS (ESI) calculated for [C₁₃H₁₅D₂NNaO₄]⁺ [M + Na]⁺: 276.1175, found: 276.1179.

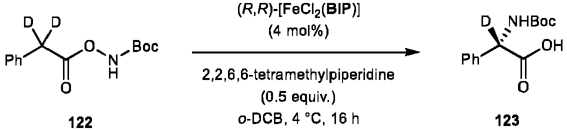

Under the general reaction condition for 1,3-nitrogen migration, the reaction of **122** (50.7 mg, 0.2 mmol) catalyzed by 4 mol% (*R*,*R*)-[FeCl₂(**BIP**)] gave **123** as a white gum (chromatography on silica gel, eluent: EtOAc/n-hexane = 1/6 with 0.2% HOAc, 47.3 mg, 94% yield): ¹H NMR (300 MHz, MeOD) δ 7.48-7.27 (m, 5H), 1.44 (s, 9H). ¹³C NMR (75 MHz, MeOD) δ 174.3, 157.6, 138.8, 129.8, 129.4, 128.7, 81.0, 59.3 (m), 28.8. HRMS (ESI) calculated for [C₁₃H₁₆DNNaO₄]⁺ [M + Na]⁺: 275.1113, found: 275.1114. Enantiomeric excess was established by HPLC analysis using a Chiralpak IG column, ee = 90% (HPLC: 210 nm, n-hexane/iPrOH = 90/10 + 0.1% TFA, flow rate 1.0 mL/min, 25 °C, tr (major) = 21.1 min, tr (minor) = 14.4 min). [α]_{D}²⁵ = +100.9° (c 1.0, MeOH).

### 9. Increasing the Enantiomeric Purity by Crystallization

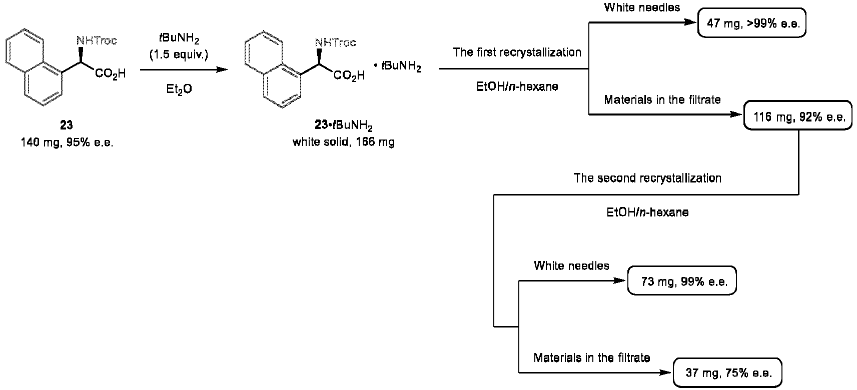

Enantiopure α-amino acid **23** could be obtained by recrystallization of the corresponding ammonium salt following a modified literature procedure.⁴

**Preparation of the ammonium salt:** to a solution of α-amino acid **23** (140 mg, 0.37 mmol, 95% e.e.) in diethyl ether (2 mL) at room temperature was added *t*BuNH₂ (59 µL, 0.56 mmol, 1.5 equiv.). The mixture was stirred for 10 minutes and the solvent was removed under reduced pressure to give the ammonium salt as a white solid (166 mg, quant. yield).

**Recrystallization of the ammonium salt:** the ammonium salt (166 mg) was mixed with 6 mL *n-*hexane, and the suspension was heated to gentle reflux. Ethanol was added in 1 mL portions until the solid completely dissolved (3 mL ethanol in total) and then another 6 mL *n*-hexane was added in one portion under heating. The solution was placed in a -20 °C refrigerator for 16 hours, during which white needles formed. The crystal was collected by filtration and was washed with *n*-hexane and dried under reduced pressure (47 mg with >99% e.e.).

**Procedures for measuring the e.e. of ammonium salts:** the sample (0.5 mg) was dissolved in 0.5 mL of the mobile phase used for HPLC analysis (*i*PrOH/*n*-hexane = 20/80 with 0.1% TFA) and was acidified by addition of excess amount of TFA (5 µL). The mixture was directly injected into the HPLC for determination of e.e. The HPLC chromatograms are shown in Fig. 5A to 5C.

After the first recrystallization, the filtrate was evaporated under reduced pressure to recover impure **23**•*t*BuNH₂ (116 mg, and the e.e. of which was 92%), which was subjected to the second recrystallization following the same procedure to give another portion of enantiopure **23**•*t*BuNH₂ as white needles (73 mg with 99% e.e.).

The free acid was recovered by acidification of the ammonium salt.⁴ Enantiopure **23**•*t*BuNH₂ (50 mg) was partitioned between hydrochloric acid (aqueous solution, 1 M, 5 mL) and diethyl ether (10 mL). The organic layer was separated, and the aqueous layer was extracted with Et₂O for two times. The combined organic layer was washed with brine and dried over anhydrous sodium sulfate. After filtration, the solvent was evaporated under reduced pressure to give α-amino acid **23** as a colorless oil (43 mg, quant.).

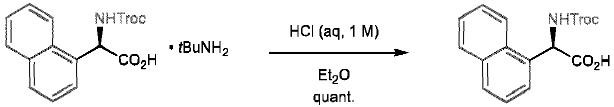

### 10. References of Experimental Section

1. Zheng, Y. et al. Octahedral ruthenium complex with exclusive metal-centered chirality for highly effective asymmetric catalysis. J. Am. Chem. Soc. 2017, 139, 4322-4325.
2. Mitra, M. et al. Highly enantioselective epoxidation of olefins by H2O2 catalyzed by a non-heme Fe(II) catalyst of a chiral tetradentate ligand. Dalton Trans. 2019, 48, 6123-6131.
3. Ye, L., Tian, Y., Meng, X., Gu, Q.-S. & Liu, X.-Y. Enantioselective copper(I)/chiral phosphoric acid catalyzed intramolecular amination of allylic and benzylic C-H bonds. Angew. Chem. Int. Ed. 2020, 59, 1129-1133.
4. Zuend, S. J., Coughlin, M. P., Lalonde, M. P. & Jacobsen, E. N. Scaleable catalytic asymmetric Strecker syntheses of unnatural α-amino acids. Nature 2009, 461, 968-970.

## Claims

1. Method for preparing α-amino acids and α-deuterated α-amino acids, comprising the following steps:
providing carboxylic acid azanyl esters, in which the N-atom of the azanyl ester group is protected by a protecting group, and
carrying out a 1,3-nitrogen migration in the presence of a catalyst to provide the α-amino acids and the α-deuterated α-amino acids.

2. The method according to claim 1, wherein the carboxylic acid azanyl ester is prepared by reacting the carboxylic acid with the compound OH-NH-PG, wherein PG is the protecting group.

3. The method according to any of the preceding claims, wherein the protecting group is a electron withdrawing protecting group.

4. The method of claim 3, wherein the protecting group is selected from the group consisting of the CO₂Me group, the CO₂CH₂CCl₃ group (Troc) and the butyloxy carbonyl group (Boc), the benzyloxycarbonyl group (Cbz), the allyloxycarbonyl group, among other amine protecting groups which form a carbamate.

5. The method of any of the preceding claims, wherein the carboxylic acid has at least one hydrogen at the α-C-atom and one or two groups bound to the α-C-atom.

6. The method of any of the preceding claims, wherein the catalyst is a chiral non-racemic catalyst providing chiral non-racemic α-amino acids and chiral non-racemic α-deuterated amino acids in a stereocontrolled 1,3-nitrogen migration or wherein the catalyst is a achiral or racemic catalyst providing racemic α-amino acids and racemic α-deuterated amino acids in a non-stereocontrolled 1,3-nitrogen migration.

7. The method of claim 6, wherein the chiral non-racemic catalyst is a chiral non-racemic ruthenium catalyst or a chiral non-racemic iron catalyst.

8. The method of claim 7, wherein the chiral non-racemic ruthenium catalyst is a chiral non-racemic ruthenium(II) catalyst or wherein the chiral non-racemic iron catalyst is a chiral non-racemic iron(II) catalyst.

9. The method of any of claims 6 to 8, wherein the catalyst contains two mondodentate ligands.

10. The method of claim 9, wherein the monodentate ligand is selected from the group consisting of acetonitrile, triflate or other sulfates, acetate or other carboxylates, chloride, bromide, iodide, pseudohalides and other potentially labile monodentate ligands.

11. The method of any of claims 6 to 10, wherein the catalyst contains either two bidentate ligands or one tetradentate ligand.

12. The method of claim 11, wherein the bidentate ligand contains on C-atom and one N-atom to bind to a central atom, and the tetradentate ligand contains four N-atoms to bind to a central atom.

13. The method of any of claims 6 to 12, wherein the catalyst is selected from the group consisting of RuDMP. RuTMS. RuCF₃, RuH, Δ-Fe1, (*R*,*R*)-Fe2, (*R*,*R*)-Fe3 and (*R*,*R*)-FeBIP

14. Use of a catalyst in the method as defined in any of claims 1 to 13.

## Patentansprüche

1. Verfahren zur Herstellung von α-Aminosäuren und α-deuterierten α-Aminosäuren, umfassend die folgenden Schritte:
Bereitstellen von Carbonsäure-Azanylestern, bei denen das N-Atom der Azanylestergruppe durch eine Schutzgruppe geschützt ist, und
Durchführen einer 1,3-Stickstoffmigration in Gegenwart eines Katalysators, um die α-Aminosäuren und die α-deuterierten α-Aminosäuren bereitzustellen.

2. Verfahren nach Anspruch 1, wobei der Carbonsäure-Azanylester durch Umsetzen der Carbonsäure mit der Verbindung OH-NH-PG hergestellt wird, wobei PG die Schutzgruppe ist.

3. Verfahren nach einem der vorhergehenden Ansprüche, wobei die Schutzgruppe eine elektronenziehende Schutzgruppe ist.

4. Verfahren nach Anspruch 3, wobei die Schutzgruppe ausgewählt ist aus der Gruppe bestehend aus der CO₂Me-Gruppe, der CO₂CH₂CCl₃-Gruppe (Troc) und der Butyloxycarbonylgruppe (Boc), der Benzyloxycarbonylgruppe (Cbz), der Allyloxycarbonylgruppe und anderen Aminschutzgruppen, die ein Carbamat bilden.

5. Verfahren nach einem der vorhergehenden Ansprüche, wobei die Carbonsäure mindestens ein Wasserstoffatom am α-C-Atom und eine oder zwei an das α-C-Atom gebundene Gruppen aufweist.

6. Verfahren nach einem der vorhergehenden Ansprüche, wobei der Katalysator ein chiraler nicht-racemischer Katalysator ist, der chirale nicht-racemische α-Aminosäuren und chirale nicht-racemische α-deuterierte Aminosäuren in einer stereokontrollierten 1,3-Stickstoffmigration bereitstellt, oder wobei der Katalysator ein achiraler oder racemischer Katalysator ist, der racemische α-Aminosäuren und racemische α-deuterierte Aminosäuren in einer nichtstereokontrollierten 1,3-Stickstoffmigration bereitstellt.

7. Verfahren nach Anspruch 6, wobei der chirale nicht-racemische Katalysator ein chiraler nicht-racemischer Rutheniumkatalysator oder ein chiraler nicht-racemischer Eisenkatalysator ist.

8. Verfahren nach Anspruch 7, wobei der chirale nicht-racemische Rutheniumkatalysator ein chiraler nicht-racemischer Ruthenium(II)-Katalysator ist oder wobei der chirale nicht-racemische Eisenkatalysator ein chiraler nicht-racemischer Eisen(II)-Katalysator ist.

9. Verfahren nach einem der Ansprüche 6 bis 8, wobei der Katalysator zwei monodentate Liganden enthält.

10. Verfahren nach Anspruch 9, wobei der monodentate Ligand ausgewählt ist aus der Gruppe bestehend aus Acetonitril, Triflat oder anderen Sulfaten, Acetat oder anderen Carboxylaten, Chlorid, Bromid, Iodid, Pseudohaliden und anderen potentiell labilen monodentaten Liganden.

11. Verfahren nach einem der Ansprüche 6 bis 10, wobei der Katalysator entweder zwei bidentate Liganden oder einen tetradentaten Liganden enthält.

12. Verfahren nach Anspruch 11, wobei der bidentate Ligand ein C-Atom und ein N-Atom enthält, um an ein Zentralatom zu binden, und der tetradentate Ligand vier N-Atome enthält, um an ein Zentralatom zu binden.

13. Verfahren nach einem der Ansprüche 6 bis 12, wobei der Katalysator ausgewählt ist aus der Gruppe bestehend aus RuDMP, RuTMS, RuCF₃, RuH, Δ-Fe1, (R,R)-Fe2, (R,R)-Fe3 und (R,R)-FeBIP.

14. Verwendung eines Katalysators in dem Verfahren nach einem der Ansprüche 1 bis 13.

## Revendications

1. Procédé de préparation d'acides α-aminés et d'acides α-aminés α-deutérés, comprenant les étapes suivantes consistant à :
fournir des esters azanyliques d'acide carboxylique, dans lesquels l'atome N du groupe ester azanylique est protégé par un groupe protecteur, et
réaliser une migration d'azote 1,3 en présence d'un catalyseur pour fournir les acides α-aminés et les acides α-aminés α-deutérés.

2. Procédé selon la revendication 1, dans lequel l'ester azanylique d'acide carboxylique est préparé en faisant réagir l'acide carboxylique avec le composé OH-NH-PG, dans lequel PG est le groupe protecteur.

3. Procédé selon l'une quelconque des revendications précédentes, dans lequel le groupe protecteur est un groupe protecteur attracteur d'électrons.

4. Procédé selon la revendication 3, dans lequel le groupe protecteur est choisi dans le groupe constitué par le groupe CO₂Me, le groupe CO₂CH₂CCl₃ (Troc) et le groupe butyloxycarbonyle (Boc), le groupe benzyloxycarbonyle (Cbz), le groupe allyloxycarbonyle, entre autres groupes protecteurs d'amine qui forment un carbamate.

5. Procédé selon l'une quelconque des revendications précédentes, dans lequel l'acide carboxylique possède au moins un hydrogène au niveau de l'atome α-C et un ou deux groupes liés à l'atome α-C.

6. Procédé selon l'une quelconque des revendications précédentes, dans lequel le catalyseur est un catalyseur chiral non racémique fournissant des acides α-aminés non racémiques chiraux et des acides aminés α-deutérés non racémiques chiraux dans une migration d'azote 1,3 stéréocontrôlée ou dans lequel le catalyseur est un catalyseur achiral ou racémique fournissant des acides α-aminés racémiques et des acides aminés α-deutérés racémiques dans une migration d'azote 1,3 non stéréocontrôlée.

7. Procédé selon la revendication 6, dans lequel le catalyseur non racémique chiral est un catalyseur au ruthénium non racémique chiral ou un catalyseur au fer non racémique chiral.

8. Procédé selon la revendication 7, dans lequel le catalyseur au ruthénium non racémique chiral est un catalyseur au ruthénium(II) non racémique chiral ou dans lequel le catalyseur au fer non racémique chiral est un catalyseur au fer(II) non racémique chiral.

9. Procédé selon l'une quelconque des revendications 6 à 8, dans lequel le catalyseur contient deux ligands mondodentés.

10. Procédé selon la revendication 9, dans lequel le ligand monodenté est choisi dans le groupe constitué par l'acétonitrile, le triflate ou d'autres sulfates, l'acétate ou d'autres carboxylates, le chlorure, le bromure, l'iodure, les pseudohalogénures et d'autres ligands monodentés potentiellement labiles.

11. Procédé selon l'une quelconque des revendications 6 à 10, dans lequel le catalyseur contient soit deux ligands bidentés, soit un ligand tétradenté.

12. Procédé selon la revendication 11, dans lequel le ligand bidenté contient un atome C et un atome N pour se lier à un atome central, et le ligand tétradenté contient quatre atomes N pour se lier à un atome central.

13. Procédé selon l'une quelconque des revendications 6 à 12, dans lequel le catalyseur est choisi dans le groupe constitué par RuDMP, RuTMS, RuCF₃, RuH, Δ-Fe1, (R,R)-Fe2, (R,R)-Fe3 et (R,R)-FeBIP

14. Utilisation d'un catalyseur dans le procédé tel que défini dans l'une quelconque des revendications 1 à 13.
